# EUROPEAN PATENT SPECIFICATION

(11) **EP 3 897 842 B1**
(45) Date of publication and mention of the grant of the patent: **29.01.2025**
(21) Application number: 19832821.3
(22) Date of filing: 13.12.2019
(51) Int. Cl.: A61P 33/00, C07D 215/42, C07D 311/58, C07D 405/12, C07D 471/04, A61K 31/4709, A61K 31/4706, A61K 31/5377, A61K 31/4375, A61K 31/519, A61K 31/353

(54) **BICYCLIC DERIVATIVES**
BIZYKLISCHE DERIVATE
DÉRIVÉS BICYCLIQUES

(30) Priority: 18.12.2018 US 201862781132 P
(43) Date of publication of application: 27.10.2021
(73) Proprietor: Elanco Tiergesundheit AG, 4058 Basel (CH)
(72) Inventor: DUCRAY, Pierre, Greenfield, Indiana 46140 (US); PAUTRAT, Francois, Greenfield, Indiana 46140 (US); RAGEOT, Denise, Greenfield, Indiana 46140 (US); TAHTAOUI, Chouaib, Greenfield, Indiana 46140 (US)
(74) Representative: Cohausz & Florack
(86) International application number: PCT/US2019/066302
(87) International publication number: WO 2020/131631

(56) References cited:
- WO-A1-2017/178416
- WO-A1-2018/087036

## Description

### FIELD

The present invention relates to medicinal chemistry, pharmacology, and veterinary and human medicine. More particularly, the present invention relates to compounds of formula (I) and said compounds for use in the control of endoparasites, for example heartworms, in warm-blooded animals.

### BACKGROUND

Heartworm (*Dirofilaria immitis)* is a parasitic roundworm that is spread from host to host through the bites of mosquitoes. The lifecycle starts when a female mosquito takes a blood meal from an infected host. The mosquito ingests immature heartworms which then molt to the infective larvae stage and travel to the mosquitoes' mouth parts. The mosquito then feeds on a susceptible host, such as a dog or cat, depositing the infective larvae. The larvae then molt to the next larval stage in the new host and then migrate through the body, eventually ending up in the blood vessels. As the larvae migrate through the tissues they molt into juvenile adults. The juvenile adults eventually move into the blood vessels of the lungs where they mature into sexually active adults. The adult heartworms then breed and release immature heartworms completing the cycle. Heartworm infection may result in serious disease for the host.

Adult heartworm infections may be treated with arsenic-based compounds; the treatment is time consuming, cumbersome, and often only partly successful. Accordingly, treatment is focused on the control of heartworm infection. Heartworm control is currently performed exclusively by year round periodical administration of drugs. Typical treatments include macrocyclic lactones such as ivermectin, moxidectin, and milbemycin oxime. Unfortunately, developing resistance of *Dirofilaria immitis* to macrocyclic lactones has been observed. Accordingly, there is a need for new compounds which effectively control heartworm infections either by way of prophylaxis or by directly killing heartworms. Certain treatments of endoparasites are described in WO 2017/178416, WO 2018/087036, WO 2018/197401, WO 2019/025341, and WO 2019/002132.

### SUMMARY

The present invention provides compounds of formula (I) which effectively treat and/or control endoparasites (e.g., heartworm) in warm-blooded animals.

The present invention provides compounds of formula (I): 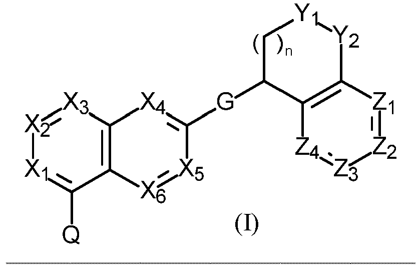 wherein
n is 1;
X₁ is selected from the group consisting of N and CR₁;
X₂ is selected from the group consisting of CR₂;
X₃ is selected from the group consisting of N and CR₃;
X₄ is selected from the group consisting of CR₄;
X₅ is selected from the group consisting of CR₅;
X₆ is selected from the group consisting of N and CR₆;
G is the group 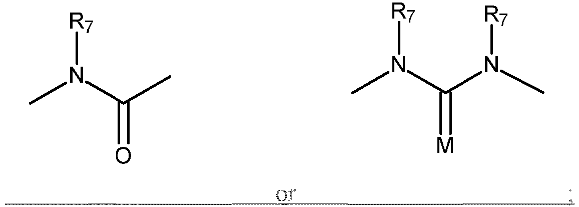
Y₁ is selected from the group consisting of CR₈R₉, and O;
Y₂ is selected from the group consisting of CR₈R₉, and O;
   wherein at least one of the groups Y₁ or Y₂ is CR₈R₉;
Z₁ is selected from the group consisting of N, O, S, and CR₁₁;
Z₂ is selected from the group consisting of nil, N₁ and CR₁₁;
Z₃ is selected from the group consisting of nil, N and CR₁₁;
Z₄ is selected from the group consisting of N, O, S, and CR₁₁;
   wherein no more than 2 of Z₁, Z₂, Z₃, and Z₄ are N and wherein only one of Z₁ and
   Z₄ is O or S, Z₂ is nil only when Z₁ is O or S, and Z₃ is nil only when Z₄ is O or S;
R₁ is selected from the group consisting of hydrogen, halogen, hydroxyl, -SH,-SC₁-C₄ alkyl, -S(O)(C₁-C₄ alkyl, -S(O)₂(C₁-C₄ alkyl, cyano, C₁-C₄ alkyl, C₁-C₄ halogenoalkyl, C₁-C₄-alkoxy, -B(OR₁₆)(OR₁₇) wherein R₁₆ is, each time taken, selected from the group consisting or hydrogen, C₁-C₄ alkyl, and C₃-C₆ cycloalkyl, R₁₇ is, each time taken, selected from the group consisting or hydrogen, C₁-C₄ alkyl, and C₃-C₆ cycloalkyl, or R₁₆ and R₁₇ together with the oxygen atoms to which they are attached form a 5- to 7-membered ring which is optionally substituted with 1 to 4 C₁-C₄ alkyl; -NH₂, -NH(C₁-C₄ alkyl), and -N(C₁-C₄ alkyl)₂;
R₂ is selected from the group consisting of hydrogen, halogen, hydroxyl, -SH, -SC₁-C₄ alkyl, -S(O)(C₁-C₄ alkyl, -S(O)₂(C₁-C₄ alkyl, cyano, C₁-C₄ alkyl, C₁-C₄ halogenoalkyl, C₁-C₄-alkoxy, -B(OR₁₆)(OR₁₇) wherein R₁₆ is, each time taken, selected from the group consisting or hydrogen, C₁-C₄ alkyl, and C₃-C₆ cycloalkyl, R₁₇ is, each time taken, selected from the group consisting or hydrogen, C₁-C₄ alkyl, and C₃-C₆ cycloalkyl, or R₁₆ and R₁₇ together with the oxygen atoms to which they are attached form a 5- to 7-membered ring which is optionally substituted with 1to 4 C₁-C₄ alkyl; -NH₂, -NH(C₁-C₄ alkyl), and -N(C₁-C₄ alkyl)₂;
R₃ is selected from the group consisting of hydrogen, halogen, hydroxyl, -SH, -SC₁-C₄ alkyl, -S(O)(C₁-C₄ alkyl, -S(O)₂(C₁-C₄ alkyl, cyano, C₁-C₄ alkyl, C₁-C₄ halogenoalkyl, C₁-C₄-alkoxy, -B(OR₁₆)(OR₁₇) wherein R₁₆ is, each time taken, selected from the group consisting or hydrogen, C₁₋C₄ alkyl, and C₃-C₆ cycloalkyl, R₁₇ is, each time taken, selected from the group consisting or hydrogen, C₁-C₄ alkyl, and C₃-C₆ cycloalkyl, or R₁₆ and R₁₇ together with the oxygen atoms to which they are attached form a 5- to 7-membered ring which is optionally substituted with 1 to 4 C₁-C₄ alkyl; -NH₂, -NH(C₁-C₄ alkyl), and -N(C₁-C₄ alkyl)₂;
R₄ is selected from the group consisting of C₁-C₄ alkyl, C₃-C₆ cycloalkyl, -N(C₁-C₄ alkyl)₂, -N(C₁-C₄ alkyl)(C₁-C₄ alkoxy) and 4- to 7-membered heterocycloalkyl;
R₆ is selected from the group consisting of hydrogen, halogen, hydroxyl, -SH, -SC₁-C₄ alkyl, -S(O)(C₁-C₄ alkyl, -S(O)₂(C₁-C₄ alkyl, cyano, C₁-C₄ alkyl, C₁-C₄ halogenoalkyl, C₁-C₄-alkoxy, -B(OR₁₆)(OR₁₇) wherein R₁₆ is, each time taken, selected from the group consisting or hydrogen, C₁-C₄ alkyl, and C₃-C₆ cycloalkyl, R₁₇ is, each time taken, selected from the group consisting or hydrogen, C₁-C₄ alkyl, and C₃-C₆ cycloalkyl, or R₁₆ and R₁₇ together with the oxygen atoms to which they are attached form a 5- to 7-membered ring which is optionally substituted with 1 to 4 C₁-C₄ alkyl; -NH₂, -NH(C₁-C₄ alkyl), and -N(C₁-C₄ alkyl)₂;
R₇ is selected from the group consisting of hydrogen, C₁-C₄ alkyl, and C₃-C₆ cycloalkyl optionally substituted with 1 to 5 halogen atoms, -C(H)O, C₂-C₄ alkenyl, C₂-C₄ alkynyl, C₁-C₄ halogenoalkyl, and C₁-C₄-alkoxy;
R₈ is, each time selected, independently selected from the group consisting of hydrogen, fluoro, and C₁-C₄ alkyl;
R₉ is, each time selected, independently selected from the group consisting of hydrogen, fluoro, and C₁-C₄ alkyl;
R₁₁ is, each time selected, independently selected from the group consisting of hydrogen, halogen, hydroxyl, cyano, C₁-C₄ alkyl, C₁-C₄ halogenoalkyl, C₁-C₄-alkoxy, C₃-C₆ cycloalkyl, -NH₂, -NH(C₁-C₄ alkyl), and -N(C₁-C₄ alkyl)₂; and
Q is selected from the group consisting of 6- or 10 membered aryl optionally substituted with 1, 2 or 3 substituents independently selected from the group consisting of halogen, cyano, nitro, hydroxyl, C₁-C₄ alkyl, C₁-C₄ halogenoalkyl, C₁-C₄ alkoxy, C₃-C₆ cycloalkyl, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl, wherein the 6- or 10 membered aryl is optionally fused with a 4- to 7-membered heterocycloalkyl having 1 or 2 heteroatoms selected from the group O, S, and N and wherein the carbons of the heterocycloalkyl are optionally substituted with 1, 2 or 3 substituents independently selected from the group halogen, cyano, nitro, hydroxyl, oxo, C₁-C₄ alkyl, C₃-C₆ cycloalkyl, C₁-C₄ halogenoalkyl, C₁-C₄ alkoxy, -NH₂, -NH(C₁-C₄ alkyl), and -N(C₁-C₄ alkyl)₂ and any N in the heterocycloalkyl is, valency permitting, substituted with a substituent selected from the group consisting of hydrogen, C₁-C₄ alkyl, and C₃-C₆ cycloalkyl;
M is selected from the group consisting of N-R₁₃, O, and S;
R₁₃ is selected from the group consisting of hydroxy, C₁-C₄ alkoxy, and -NH₂;
or a salt thereof.

In one embodiment, the present invention also provides compositions, comprising: a compound of formula (I) or a salt thereof and an acceptable excipient, the composition optionally further comprising at least one additional active compound.

Also described herein, but not encompassed by the claims, is a method for treating parasites, comprising: administering to a subject in need thereof an effective amount of a compound of formula (I) or a salt thereof, the method optionally further comprising an effective amount of at least one additional active compound.

Also described herein, but not encompassed by the claims, is a method for controlling parasites, comprising: administering to a subject in need thereof an effective amount of a compound of formula (I) or a salt thereof, the method optionally further comprising an effective amount of at least one additional active compound.

Also described herein, but not encompassed by the claims, is a method for treating or controlling parasites, comprising: contacting a subject's environment with an effective amount of a compound of formula (I) or a salt thereof, the method optionally further comprising an effective amount of at least one additional active compound.

Thus, the invention provides compounds of the invention for use as a medicament, including for the manufacture of a medicament. In one embodiment, the invention provides the manufacture of a medicament comprising a compound of formula (I) or a salt thereof for treating parasites. In one embodiment, the invention provides the manufacture of a medicament comprising a compound of formula (I) or a salt thereof for controlling parasites.

Also described herein, but not encompassed by the claims, are processes from making compounds of the invention and intermediates thereof.

### DETAILED DESCRIPTION

The term "C₁-C₄ alkyl" refers to a straight or branched alkyl chain having from one to four carbon atoms and includes methyl, ethyl, propyl, isopropyl, butyl, and the like.

The term "C₁-C₄ halogenoalkyl" refers to a straight or branched alkyl chain having from one to four carbon atoms and 1 to 5 halogen and includes fluoromethyl, difluoromethyl, trifluoromethyl, 2,2,2-trifluoroethyl, 1,2,2-trifluoroethyl, 3,3,3-trifluoropropyl, and the like.

The term "C₂-C₄ alkenyl" refers to a straight or branched alkenyl chain having from two to four carbon atoms and one carbon-carbon double bond, and includes ethylene, propylene, iso-propylene, butylene, iso-butylene, sec-butylene, and the like.

The term "C₂-C₄ alkynyl" refers to a straight or branched alkynyl chain having from two to four carbon atoms and one carbon-carbon triple bond, and includes acetylene, propargyl, and the like.

The term "C₁-C₄ alkoxy" refers to a C₁-C₄ alkyl attached through an oxygen atom and includes methoxy, ethoxy, propoxy, isopropoxy, butoxy, and the like.

The term "C₃-C₆ cycloalkyl" refers to an alkyl ring of three to six carbon atoms, and includes cyclopropyl, cyclobutyl, cyclopentyl, and cyclohexyl.

The terms "halogen" and "halogeno" refers to a chloro, fluoro, bromo or iodo atom.

The term "C₆- or C₁₀- membered aryl" refers to phenyl or naphthyl.

The term "C₆- or C₁₀- membered aryloxy" refers to phenyl or naphthyl attached through an oxygen atom and includes phenoxy and naphtyloxy.

The term "C₆- or C₁₀- membered arylthio-oxy" refers to phenyl or naphthyl attached through an sulfur atom and includes phenthio-oxy and naphtylthio-oxy. Further it is understood that the term "C₆- or C₁₀- membered arylthio-oxy" also encompasses in which the sulfur is the -SO₂- and -S(O)-.

The term "4- to 7-membered heterocycloalkyl" refers to a 4 to 7 membered monocyclic saturated or partially (but not fully) unsaturated ring having one or two heteroatoms selected from the group consisting of nitrogen, oxygen, and sulfur and the ring optionally includes a carbonyl to form a lactam or lactone. It is understood that where sulfur is included that the sulfur may be either -S-, -SO-, or -SO₂-. For example, but not limiting, the term includes azetidinyl, pyrrolidinyl, piperidinyl, piperazinyl, morpholinyl, thiomorpholinyl, oxetanyl, dioxolanyl, tetrahydropyranyl, tetrahydrothiopyranyl, tetrahydrofuryl, hexahydropyrimidinyl, tetrahydropyrimidinyl, dihydroimidazolyl, and the like.

The term "5-membered heteroaryl" refers to a five membered, monocyclic, fully unsaturated, ring with one to four carbon atoms and one to four heteroatoms selected from the group consisting of nitrogen, oxygen, and sulfur. For example, but not limiting, the term includes furyl, thienyl, pyrrolyl, imidazolyl, isothiazolyl, isoxazolyl, oxadiazolyl, oxazolyl, thiazolyl, thiadiazolyl, triazolyl, tetrazolyl, and the like. It is understood that a 5-membered heteroaryl can be attached as a substituent through a ring carbon or a ring nitrogen atom where such an attachment mode is available, for example for a pyrrolyl, imidazolyl, pyrazolyl, triazolyl, and the like.

The term "6-membered heteroaryl" refers to a six membered, monocyclic, fully unsaturated ring with one to five carbon atoms and one or more, typically one to four, heteroatoms selected from the group consisting of nitrogen, oxygen, and sulfur. For example, but not limiting, the term includes pyrazinyl, pyrazolyl, pyridazinyl, pyridyl, pyrimidyl, and the like. It is understood that a 6-membered heteroaryl can be attached as a substituent through a ring carbon or a ring nitrogen atom where such an attachment mode is available.

The term "5- to 10-membered heteroaryl having 1 or 2 heteroatoms selected from the group O, S, and N" refers to a five to ten membered, monocyclic or polycyclic fully unsaturated, ring or ring system with one to nine carbon atoms and one or two heteroatoms selected from the group consisting of nitrogen, oxygen, and sulfur. For example, but not limiting, the term includes furyl, thienyl, pyrrolyl, imidazolyl, isothiazolyl, isoxazolyl, oxadiazolyl, oxazolyl, thiazolyl, pyrazinyl, pyrazolyl, pyridazinyl, pyridyl, pyrimidyl, azepinyl, diazepinyl, benzofuryl, benzothienyl, indolyl, isoindolyl, benzimidazolyl, benzisothiazolyl, benzisoxazolyl, benzoxazolyl, benzopyrazinyl, benzopyrazolyl, quinazolyl, thienopyridyl, quinolyl, isoquinolyl benzothiazolyl, and the like. It is understood that a 5- to 10-membered heteroaryl having 1 or 2 heteroatoms selected from the group O, S, and N can be attached as a substituent through a ring carbon or a ring nitrogen atom where such an attachment mode is available.

The term "5- to 10-membered heteroaryloxy" refers to a 5- to 10-membered heteroaryl having 1 or 2 heteroatoms selected from the group O, S, and N attached through an oxygen atom and includes imidazolyloxy, pyrazolyloxy, pyridyloxy, pyrimidyloxy, quinolyloxy, and the like

The term "oxo" refers to an oxygen atom doubly bonded to the carbon to which it is attached to form the carbonyl of a ketone or aldehyde. For example, a pryidone radical is contemplated as an oxo substituted 6-membered heteroaryl.

The term "carboxyl" refers to the group below:

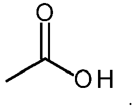

The term "carbamoyl" refers the group below:

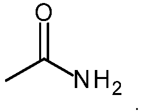

The term "C₁-C₄ alkoxy carbonyl" refers the group below: 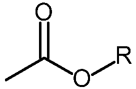 wherein R is a C₁-C₄ alkyl.

The term "nil" as used herein with reference to a group, substituent, moiety, or the like, indicates that that group, substituent, or moiety is not present. Wherein a group, substituent, or moiety is ordinarily bonded to two or more other groups, substituents, or moieties, the others are bonded together in lieu of the group, substituent, or moiety which is nil. For example, with a compound having the structure A-B-C; wherein B is nil, then A is directly bonded to C and the compound is A-C. As another example, with a compound having the structure A-B-C; wherein C is nil, then the compound is A-B.

The term "salt" refers to salts of veterinary or pharmaceutically acceptable organic acids and bases or inorganic acids and bases. Such salts are well known in the art and include those described in Journal of Pharmaceutical Science, 66, 2-19 (1977). An example is the hydrochloride salt.

The term "substituted," including when used in "optionally substituted" refers to one or more hydrogen radicals of a group being replaced with non-hydrogen radicals (substituent(s)). It is understood that the substituents may be either the same or different at every substituted position. Combinations of groups and substituents envisioned by this invention are those that are stable or chemically feasible.

The term "stable" refers to compounds that are not substantially altered when subjected to conditions to allow for their production. In a non-limiting example, a stable compound or chemically feasible compound is one that is not substantially altered when kept at a temperature of 40°C or less, in the absence of moisture or other chemically reactive conditions, for about a week.

It is understood that, where the terms defined herein mention a number of carbon atoms, that the mentioned number refers to the mentioned group and does not include any carbons that may be present in any optional substituent(s) thereon or any carbons that may be present as part of a fused ring, including a benzo-fused ring.

The skilled artisan will appreciate that certain of the compounds of the present invention exist as isomers. All stereoisomers of the compounds of the invention, including geometric isomers, enantiomers, and diastereomers, in any ratio, are contemplated to be within the scope of the present invention.

The skilled artisan will also appreciate that certain of the compounds of the present invention exist as tautomers. All tautomeric forms the compounds of the invention are contemplated to be within the scope of the present invention.

Compounds of the invention also include all isotopic variations, in which at least one atom of the predominant atom mass is replaced by an atom having the same atomic number, but an atomic mass different from the predominant atomic mass. Use of isotopic variations (*e.g.*, deuterium, ²H) may afford greater metabolic stability. Additionally, certain isotopic variations of the compounds of the invention may incorporate a radioactive isotope (*e.g.*, tritium, ³H, or ¹⁴C), which may be useful in drug and/or substrate tissue distribution studies. Substitution with positron emitting isotopes, such as ¹¹C, ¹⁸F, ¹⁵O and ¹³N, may be useful in Positron Emission Topography (PET) studies.

The terms "compounds of the invention" and "a compound of the invention" and "compounds of the present invention" and a like include the embodiment of formula (I) and the other more particular embodiments encompassed by formula (I) described herein and the exemplified compounds described herein and a salt of each of these embodiments.

The present invention pertains to (although not all is part, of the claimed invention which is defined in the claims) a compound of formula (I) with G as defined has the formulae: 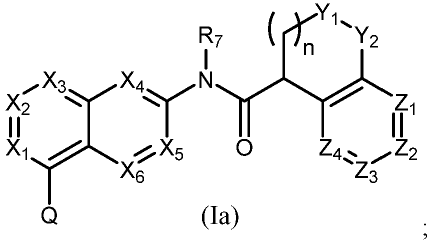 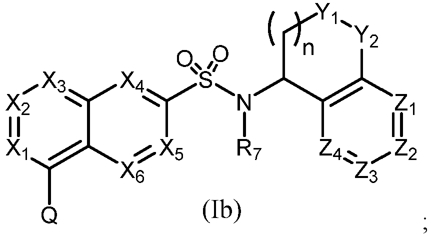 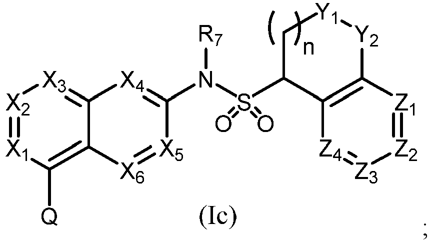 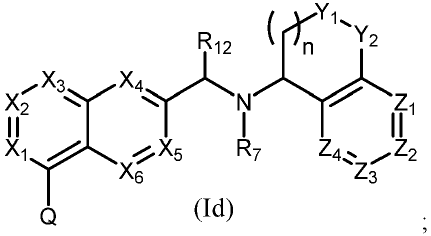 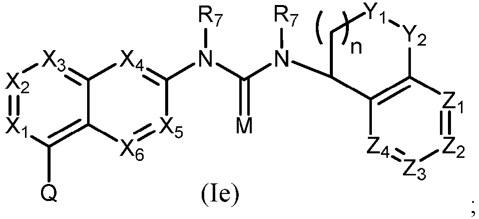 or

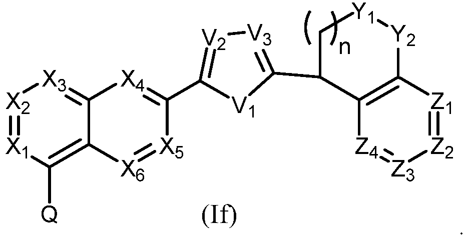

The present invention pertains to (although not all is part of the claimed invention which is defined in the claims):
(a) One embodiment relates to a compound of formula (I).
(b) One embodiment relates to a compound of formula (I) wherein G is as in a compound of formula (Ia); or a salt thereof.
(c) One embodiment relates to a compound of formula (I) wherein G is as in a compound of formula (Ib); or a salt thereof.
(d) One embodiment relates to a compound of formula (I) wherein G is as in a compound of formula (Ic); or a salt thereof.
(e) One embodiment relates to a compound of formula (I) wherein G is as in a compound of formula (Id); or a salt thereof.
(f) One embodiment relates to a compound of formula (I) wherein G is as in a compound of formula (Ie); or a salt thereof.
(g) One embodiment relates to a compound of formula (I) wherein G is as in a compound of formula (If); or a salt thereof.
(1) One embodiment relates to embodiments (a), (b), (c), (d), (e), (f), and (g) wherein at least one of X₁, X₂, X₃, and X₅ is N.
(h) One embodiment relates to embodiments (a), (b), (c), (d), (e), (f), (g), and (1) wherein X₁ is CR₁; X₂ is CR₂; X₃ is CR₃; X₄ is CR₄; X₅ is CR₅; and X₆ is CR₆; or a salt thereof.
(i) One embodiment relates to embodiments (a), (b), (c), (d), (e), (f), and (g) wherein X₁ is N; X₂ is CR₂; X₃ is CR₃; X₄ is CR₄; X₅ is CR₅; and X₆ is N; or a salt thereof.
(j) One embodiment relates to embodiments (a), (b), (c), (d), (e), (f), (g), and (1) wherein X₁ is CR₁; X₂ is CR₂; X₃ is CR₃; X₄ is CR₄; X₅ is N; and X₆ is N; or a salt thereof.
(k) One embodiment relates to embodiments (a), (b), (c), (d), (e), (f), (g), and (1) wherein X₁ is CR₁; X₂ is CR₂; X₃ is CR₃; X₄ is CR₄; X₅ is N; and X₆ is CR₆; or a salt thereof.
(l) One embodiment relates to embodiments (a), (b), (c), (d), (e), (f), (g), and (1) wherein X₁ is CR₁; X₂ is CR₂; X₃ is CR₃; X₄ is N; X₅ is N; and X₆ is N; or a salt thereof.
(m) One embodiment relates to embodiments (a), (b), (c), (d), (e), (f), (g), (1), (h), (i), (j), (k), and (l) wherein Q is a 6- or 10 membered aryl optionally substituted with 1, 2 or 3 substituents independently selected from the group consisting of halogen, cyano, nitro, hydroxy, C₁-C₄ alkyl, C₁-C₄ halogenoalkyl, C₁-C₄ alkoxy, C₃-C₆ cycloalkyl, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl; or a salt thereof.
(n) One embodiment relates to embodiments (a), (b), (c), (d), (e), (f), (g), (1), (h), (i), (j), (k), and (l) wherein Q is 6-membered aryl optionally substituted with 1, 2 or 3 substituents independently selected from the group consisting of halogen, cyano, nitro, hydroxy, C₁-C₄ alkyl, C₁-C₄ halogenoalkyl, C₁-C₄ alkoxy, C₃-C₆ cycloalkyl, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl, wherein the 6-membered aryl is fused with a 4- to 7-membered heterocycloalkyl having 1 or 2 heteroatoms selected from the group O, S, and N and wherein the carbons of the heterocycloalkyl are optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, -NH₂, -NH(C₁-C₄ alkyl), and -N(C₁-C₄ alkyl)₂ and any N in the heterocyclalkyl is substituted with a substituent selected from the group consisting of hydrogen, C₁-C₄ alkyl, and C₃-C₆ cycloalkyl; or a salt thereof.
(o) One embodiment relates to embodiments (a), (b), (c), (d), (e), (f), (g), (1), (h), (i), (j), (k), and (l) wherein Q is a 5- to 10-membered heteroaryl having 1 or 2 heteroatoms selected from the group O, S, and N and wherein the carbons of the heteroaryl are optionally substituted with 1, 2 or 3 substituents independently selected from the group consisting of halogen, cyano, nitro, -OH, C₁-C₄ alkyl, C₃-C₆ cycloalkyl, C₁-C₄ halogenoalkyl, C₁-C₄ alkoxy, -NH₂, -NH(C₁-C₄ alkyl), and -N(C₁-C₄ alkyl)₂ and any N in the heteroaryl is optionally substituted with a substituent selected from the group consisting of hydrogen, C₁-C₄ alkyl, and C₃-C₆ cycloalkyl; or a salt thereof.
(p) One embodiment relates to embodiments (a), (b), (c), (d), (e), (f), (g), (1), (h), (i), (j), (k), and (l) wherein Q is a 4- to 7-membered heterocycloalkyl having 1 or 2 heteroatoms selected from the group O, S, N, wherein the heterocycloalkyl is optionally benzo-fused, wherein the carbons of the heterocycloalkyl or optionally benzo-fused heterocycloalkyl are optionally substituted with 1, 2, 3, or 4 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, -NH₂, -NH(C₁-C₄ alkyl), and -N(C₁-C₄ alkyl)₂ and any N in the heterocyclalkyl is optionally substituted with a substituent selected from the group consisting of hydrogen, C₁-C₄ alkyl, and C₃-C₆ cycloalkyl;
or a salt thereof.
(q) One embodiment relates to embodiments (a), (b), (c), (d), (e), (f), (g), (1), (h), (i), (j), (k), and (l) wherein Q is a 6- or 10 membered aryloxy optionally substituted with 1, 2 or 3 substituents independently selected from the group consisting of halogen, cyano, nitro, hydroxyl, C₁-C₄ alkyl, C₃-C₆ cycloalkyl, C₁-C₄ halogenoalkyl, C₁-C₄ alkoxy, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl; or a salt thereof.
(r) One embodiment relates to embodiments (a), (b), (c), (d), (e), (f), (g), (1), (h), (i), (j), (k), and (l) wherein Q is a and 5- to 10-membered heteroaryloxy optionally substituted with 1, 2 or 3 substituents independently selected from the group consisting of halogen, cyano, nitro, hydroxyl, oxo, C₁-C₄ alkyl, C₃-C₆ cycloalkyl, C₁-C₄ halogenoalkyl, C₁-C₄ alkoxy, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl; or a salt thereof.
(s) One embodiment relates to embodiments (a), (b), (c), (d), (e), (f), (g), (1), (h), (i), (j), (k), (l), (m), (n), (o), (p), (q), and (r) wherein n is 1; or a salt thereof.
(t) One embodiment relates to embodiments (a), (b), (c), (d), (e), (f), (g), (1), (h), (i), (j), (k), (l), (m), (n), (o), (p), (q), (r), and (s) wherein Y₁ is CR₈R₉ and Y₂ is O; or a salt thereof.
(u) One embodiment relates to embodiments (a), (b), (c), (d), (e), (f), (g), (1), (h), (i), (j), (k), (l), (m), (n), (o), (p), (q), (r), (s), and (t) wherein Z₁ is CR₁₁, Z₂ is CR₁₁, Z₃ is CR₁₁, and Z₄ is CR₁₁; or a salt thereof.
(v) One embodiment relates to embodiments (a), (b), (c), (d), (e), (f), (g), (1), (h), (i), (j), (k), (l), (m), (n), (o), (p), (q), (r), (s), and (t) wherein Z₁ is CR₁₁, Z₂ is CR₁₁, Z₃ is nil, and Z₄ is S; or a salt thereof.
(w) One embodiment relates to embodiments (a), (b), (c), (d), (e), (f), (g), (1), (h), (i), (j), (k), (l), (m), (n), (o), (p), (q), (r), (s), (t), (u) and (v) wherein R₄ is selected from the group consisting of C₁-C₄ alkyl, C₃-C₆ cycloalkyl, -N(C₁-C₄ alkyl)₂, and 4- to 7-membered heterocycloalkyl; or a salt thereof.
(v) One embodiment relates to embodiments (a), (b), (c), (d), (e), (f), (g), (1), (h), (i), (j), (k), (l), (m), (n), (o), (p), (q), (r), (s), (t), (u) and (v) wherein R₄ is -N(C₁-C₄ alkyl)₂; or a salt thereof.
(w) Another embodiment relates to a salt of each of the exemplified compounds.

The compounds of the invention can be prepared by a variety of procedures, some of which are described below. All substituents, unless otherwise indicated, are as previously defined.

The products of each step can be recovered by conventional methods including extraction, evaporation, precipitation, chromatography, filtration, trituration, crystallization, and the like. The procedures may require protection of certain groups, for example hydroxy, thiol, amino, or carboxy groups to minimize unwanted reactions. The selection, use, and removal of protecting groups are well known and appreciated as standard practice, for example T.W. Greene and P. G. M. Wuts in Protective Groups in Organic Chemistry (John Wiley and Sons, 1991).

As used herein: AcOH refers to acetic acid; aq. refers to aqueous, br refers to broad, CH₃CN refers to acetonitrile, CH₂Cl₂ refers to methylene chloride, d refers to doublet, dd refers to doublet of doublet, DIPEA refers to N-diisopropylethylamine, DMA refers to N,N-dimethylacetamide, DMF refers to N,N-dimethylformamide, DMSO refers to dimethylsulfoxide, ee: refers to enantiomeric excess, eq. refers to equivalent, ES refers to electrospray ionization, EtOAc refers to ethyl acetate, EtOH refers to ethanol, HATU refers to 1-[bis(dimethylamino)methylene]-1*H*-1,2,3-triazolo[4,5-*b*]pyridinium 3-oxid hexafluorophosphate, HPLC refers to high performance liquid chromatography, iPrOH refers to isopropanol, J refers to coupling constant, KOAc refers to potassium acetate, K₂CO₃ refers to potassium carbonate, LCMS refers to liquid chromatography - mass spectrometry, m/z: refers to mass-to-charge ratio, M refers to molarity, m refers to multiplet, MeOH refers to methanol, min. refers to minutes, NaHCO₃ refers to sodium bicarbonate, Na₂CO₃ refers to sodium carbonate, NEt₃ refers to triethylamine, NMR refers to nuclear magnetic resonance, NMP refers to N-methylpyrrolidone, q refers to quartet, rt refers to room temperature, Rt refers to retention time, s refers to singlet, sat. refers to saturated, T refers to temperature, t refers to triplet, td refers to triplet of doublets, THF refers to tetrahydrofuran, wt refers to weight, and δ refers to chemical shift.

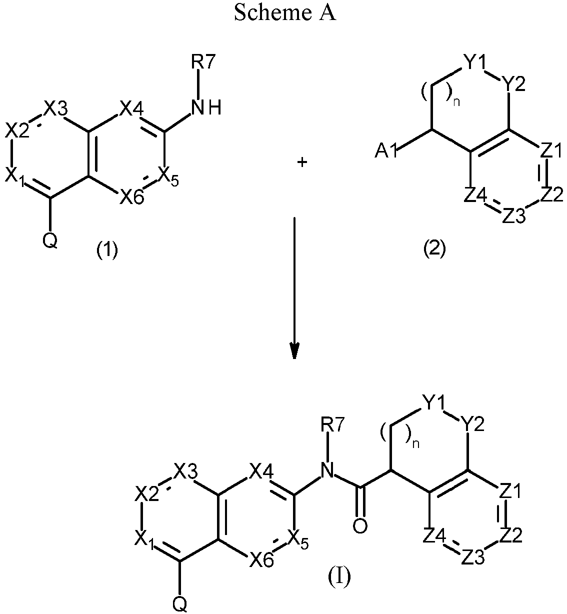

Scheme A depicts the reaction of a compound of formula (1) and a compound of formula (2) to give a compound of formula (I). The depicted compound of formula (1) Q, R₇, X₁, X₂, X₃, X₄, X₅, and X₆ are as desired in the final compound of formula (I) or a group that gives rise to Q, R₇, X₁, X₂, X₃, X₄, X₅, and X₆ as desired in the final compound of formula (I) . For example, a compound of formula (1) can be one in which the depicted group "Q" is a halogen which is further elaborated, in a subsequent step, not shown, to give a compound in which Q is as defined in formula (I). The preparation of such compounds of formula (1) is readily appreciated in the art. A compound of formula (2) is one in which is one in which the group A₁ is a carboxy group, or an activating groups as is discussed below, or a sulfonyl halide and n, Y₁, Y₂, Z₁, Z₂, Z₃, and Z₄ are as desired in the final product of formula (I) or a group that gives rise to Y₁, Y₂, Z₁, Z₂, Z₃, and Z₄ as desired in the final product of formula (I). The preparation of such compounds of formula (2) is readily appreciated in the art.

As mentioned above, Scheme A depicts the amidation of a compound of formula (1) in which using a compound of formula (2) to give a compound of formula (I). Typical groups A₁ are sulfonyl chloride, carboxy or an acid chloride or acid bromide, or imidazide, an activating moiety, a mixed anhydride of another carboxylic acid, such as formic acid, acetic acid, or represents the other part of a symmetrical anhydride formed from two compounds of formula (2) in which A₁ is carboxy. For example, standard carboxy amide forming conditions can be used, such as those using coupling agents, including those used in peptide couplings, such as 2-(1H-7-azabenzotriazol-1-yl)- 1,1,3,3-tetramethyl uronium hexafluorophosphate methanaminium (HATU), dicyclohexylcarbodiimide (DCC), and 1-(3-dimethylaminopropyl)-3-ethylcarbodiimide•HCl. If necessary or desired, an additive such as 4-(dimethylamino)pyridine, 1-hydroxybenzotriazole, and the like may be used to facilitate the reaction. Such reactions are generally carried out using a base, such as N-methylmorpholine or triethylamine, in a wide variety of suitable solvents such as CH₂Cl₂, DMF, N-methylpyrrolidone (NMP), DMA, THF, and the like. Such carboxy amide forming reactions are well understood and appreciated in the art. The formation of sulfonyl amides likewise are generally carried out using a base, such as N-methylmorpholine or triethylamine, in a wide variety of suitable solvents such as CH₂Cl₂, toluene, THF, and the like. Such sulfonyl amide forming reactions are well understood and appreciated in the art.

It will be recognized by one of ordinary skill in the art that a compound of formula (I) can be elaborated in a variety of ways to give other compounds of formula (I). Such reactions include hydrolysis, oxidation, reduction, alkylation, arylation (including heteroaryl groups) amidations, sulfonations, and the like.

Also, in an optional step, not shown, the compounds of formula (I) can be converted to salts by methods well known and appreciated in the art.

The skilled person will readily appreciate that compounds of formula Ib are readily prepared by the methodology of Scheme A using compounds for formula (1) in which -NHR₇ is replaced by a sulfonyl halide and compounds of formula (2) in which "A₁" is -NHR₇.

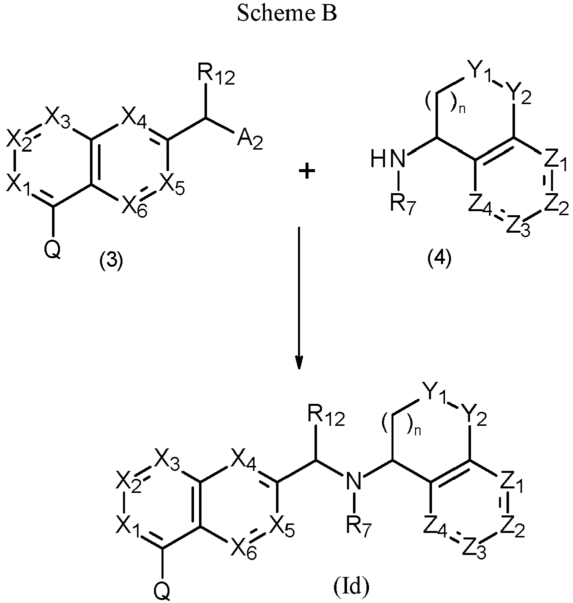

Scheme B depicts the Mitsunobu-type reaction of a compound of formula (3) and a compound of formula (4) to give a compound of formula (I). The depicted compound of formula (3) is one in which the group A₂ is an -OH group and Q, X₁, X₂, X₃, X₄, X₅, and X₆ are as desired in the final compound of formula (I) or a group that gives rise to Q, X₁, X₂, X₃, X₄, X₅, and X₆ as desired in the final compound of formula (I) . For example, a compound of formula (3) can be one in which the depicted group "Q" is a halogen which is further elaborated, in a subsequent step, not shown, to give a compound in which Q is as defined in formula (I). The preparation of such compounds of formula (1) is readily appreciated in the art. A compound of formula (4) is one in which is one in which n, R₇, Y₁, Y₂, Z₁, Z₂, Z₃, and Z₄ are as desired in the final product of formula (I) or a group that gives rise to R₇, Y₁, Y₂, Z₁, Z₂, Z₃, and Z₄ as desired in the final product of formula (I). The preparation of such compounds of formula (4) is readily appreciated in the art. Such Mitsunobu-type reactions of alcohols and acidic components are well-known and appreciated in the art.

Alternatively, Scheme B depicts a reductive amination of a compound of formula (3), as described above except that A₂ is an oxygen atom and a double bond between A2 and the carbon to which it is attached and a compound of formula (4), as described above to give a compound of formula (I). Such reductive amination of aldehydes or ketones with amines are well-known and appreciated in the art.

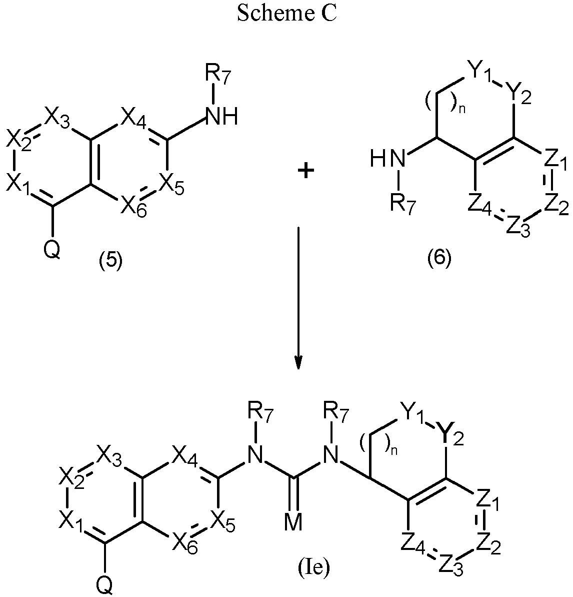

Scheme C depicts the reaction of a compound of formula (5) and a compound of formula (6) to give a compound of formula (I) in which M is O. The depicted compound of formula (5) is one in which the depicted R₇ and Q, X₁, X₂, X₃, X₄, X₅, and X₆ are as desired in the final compound of formula (I) or a group that gives rise to the depicted R₇, and Q, X₁, X₂, X₃, X₄, X₅, and X₆ as desired in the final compound of formula (I) . As above, a compound of formula (5) can be one in which the depicted group "Q" is a halogen which is further elaborated, in a subsequent step, not shown, to give a compound in which Q is as defined in formula (I). The preparation of such compounds of formula (5) is readily appreciated in the art. A compound of formula (6) is one in which is one in which the depicted R₇ and n, Y₁, Y₂, Z₁, Z₂, Z₃, and Z₄ are as desired in the final product of formula (I) or a group that gives rise to the depicted R₇, and Y₁, Y₂, Z₁, Z₂, Z₃, and Z₄ as desired in the final product of formula (I). The preparation of such compounds of formula (6) is readily appreciated in the art. The formation of unsymmetrical ureas is well known using phosgene, carbonyldiimidazole, and isopropenyl carbamates.

The following examples are intended to be illustrative and non-limiting, and represent specific embodiments of the present invention.

Analyses methods A and B were performed using an Agilent 1200 Infinity Series Liquid Chromatography (LC) system, consisting of a 1260 HiP degasser (G4225A), 1260 Binary Pump (G1312B), 1290 auto-sampler (G4226A), 1290 thermo-stated column compartment (G1316C) and a 1260 Diode Array Detector (G4212B) coupled to an Agilent 6150 single quadrupole mass spectrometry (MS) detector. The injection volume was set to 1 µL by default. The UV (DAD) acquisition was performed at 40 Hz, with a scan range of 190-400 nm (by 5nm step). A1:1 flow split was used before the MS detector. The MS was operated with an electro-spray ionization source (ESI) in both positive & negative ion mode. The nebulizer pressure was set to 50 psi, the drying gas temperature and flow to 350 °C and 12 L/min respectively. The capillary voltages used were 4000V in positive mode and 3500V in negative mode. The MS acquisition range was set to 100-800 m/z with a step size of 0.2 m/z in both polarity modes. Fragmentor voltage was set to 70 (ESI+) or 120 (ESI-), Gain to 0.40 (ESI+) or 1.00 (ESI-) and the ion count threshold to 4000 (ESI+) or 1000 (ESI-). The overall MS scan cycle time was 0.15s/cycle. Data acquisition was performed with Agilent Chemstation software.

Method A: Analyses were carried out on a Phenomenex Gemini-NX C18 column of 50 mm length, 2.1 mm internal diameter and 3 µm particle size. The mobile phase used was: A1=Water with 0.1% formic acid / B1= CH₃CN with 0.1% formic acid. The run was performed at a temperature of 50 °C and a flow rate of 1.2 mL/min, with a gradient elution from 5% to 95% (B1) over 1.5 min followed by a 0.5 min hold at 95% (B1).

Method B: Analyses were carried out on a Waters XBridge C18 column of 50 mm length, 2.1 mm internal diameter and 3.5 µm particle size. The mobile phase used was: A2=Water with 10mM ammonium bicarbonate, adjusted at pH 9 with ammonium hydroxide / B2= CH₃CN. The run was performed at a temperature of 50°C and a flow rate of 1.2 mL/min, with a gradient elution from 5% to 95% (B2) over 1.5 min followed by a 0.5 min hold at 95% (B2).

Analyses methods C and D were performed using a Waters Acquity UPLC Liquid Chromatography (LC) system, coupled to an Waters SQ Detector 2 single quadrupole mass spectrometry (MS) detector. The UV (DAD) acquisition was performed with a scan range of 200-400 nm (by 1.2nm resolution). The MS was operated with an electro-spray ionization source (ESI) in both positive & negative ion mode. Capillary Voltage 3.50(kV), Cone Voltage 35(V), and Desolvation Temperature of 550 °C. Desolvation gas flow 1000(L/Hr), Cone gas flow 50(L/Hr). The MS acquisition range was set to 100-1500 m/z . MS scan cycle time was 0.5s. Data acquisition was performed with Waters Masslynx software.

Method C: Analyses were carried out on an Acquity UPLC BEH C18 column of 50 mm length, 2.1 mm internal diameter and 1.7 µm particle size. The mobile phase used was: A1= Water with 0.1% formic acid / B1= CH₃CN with 0.1% formic acid. The injection volume was 1 µL. The run was performed at a temperature of 40 °C and a flow rate of 0.6 mL/min, with a gradient elution. Method info (Time (min) and B %): 0-5; 0.3-5; 2.5-95; 3.7-95; 4-5; 4.6-5.

Method D: Analyses were carried out on an Acquity UPLC BEH C18 column of 50 mm length, 2.1 mm internal diameter and 1.7 µm particle size. The mobile phase used was: A1= Water with 10 mM Ammonium acetate / B1= CH₃CN with 0.1% formic acid. The injection volume was 0.1µL. The run was performed at a temperature of 45 °C and a flow rate of 0.5 mL/min, with a gradient elution. Method info (Time (min) and A %): 0-98; 0.3-98; 3.2-2; 4.4-2; 4.7-98.

### Examples 1.1 and 1.2

### (4R) and (4S)-N-[8-(3,5-dichlorophenyl)-4-(dimethylamino)-3-quinolyl]chromane-4-carboxamide

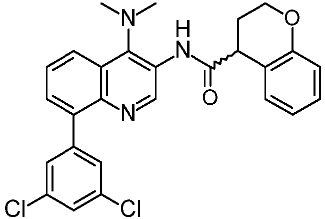

To a stirred solution of 8-bromoquinolin-4-ol (2 g, 8.82 mmol) in propionic acid (20 mL, 265 mmol) at 100°C was added nitric acid (1 mL, 16 mmol) slowly over 5 min. The reaction was heated to 125°C and left to stir for 45 min. The reaction was then allowed to cool to rt to give the precipitate. The solid was collected by filtration and was washed with water (3x10 mL), iPrOH (10 mL), isohexane (10 mL), and then dried in the vacuum oven for 1 hour to give 8-bromo-3-nitro-quinolin-4-ol. LCMS (method B): Rt= 0.54 min, m/z= 269 [M+H]⁺.

To a solution of 8-bromo-3-nitro-quinolin-4-ol (1.52 g, 5.37 mmol) was added POCl₃ (10 mL, 107 mmol). The suspension was heated to reflux and left to stir for 2 hours. The reaction mixture was allowed to cool to rt, and left to stand overnight. The reaction mixture was concentrated *in vacuo* (azeotroping with toluene) to give 8-bromo-4-chloro-3-nitro-quinoline which was used directly in the next step.

To a solution of 8-bromo-4-chloro-3-nitro-quinoline (2.32 g, 5.38 mmol,) in THF (30 mL) was slowly added dimethylamine (2 M in THF, 7 mL, 14 mmol, 2 M). The reaction was left to stir at rt for 1.5 hour. The reaction mixture was partitioned between EtOAc and sat. aq. NaHCO₃ (50 mL of each). Brine (50 mL) was added. The layers were separated, and the aq. layer was extracted with EtOAc (2x50 mL). The combined organic layers were concentrated *in vacuo* to give 8-bromo-N,N-dimethyl-3-nitro-quinolin-4-amine. LCMS (method B): Rₜ= 1.13 min, m/z= 296 [M+H]⁺.

To a solution of 8-bromo-N,N-dimethyl-3-nitro-quinolin-4-amine (505 mg, 1.62 mmol), was added (3,5-dichlorophenyl)boronic acid (314 mg, 1.61 mmol), tetrakis(triphenylphosphine)palladium(0) (92 mg, 0.079 mmol), and Na₂CO₃ (351 mg, 3.28 mmol). The vial was sealed, then evacuated and back-filled with N₂ three times. 1,4-Dioxane (9 mL) was added, followed by water (3 mL) and the reaction was heated to 100°C in the microwave for 1 hour. The reaction mixture was partitioned between EtOAc and sat. aq. NaHCO₃ (50 mL of each). The layers were separated, and the aq. layer was extracted with EtOAc (2x25 mL). The combined organic layers were concentrated *in vacuo,* then purified by column chromatography to give 8-(3,5-dichlorophenyl)-N,N-dimethyl-3-nitro-quinolin-4-amine. LCMS (method B): Rt= 1.57 min, m/z= 362 [M+H]⁺. To a stirred suspension of 8-(3,5-dichlorophenyl)-N,N-dimethyl-3-nitro-quinolin-4-amine (401 mg, 1.052 mmol) in THF (5 mL), EtOH (5 mL) and water (2.5 mL) was added iron (184 mg, 3.23 mmol) and NH₄Cl (168 mg, 3.13 mmol). The reaction was heated to 75°C and left to stir for 45 min. The reaction was allowed to cool to rt, then partitioned between sat. aq. NaHCO₃ and EtOAc (25 mL of each). The mixture was filtered through Celite^{®} (washing with EtOAc), and the layers of the filtrate were separated. The aq. layer was extracted with EtOAc (2x25 mL), and the combined organic layers were concentrated *in vacuo* before being purified by column chromatography to give 8-(3,5-dichlorophenyl)-N4,N4-dimethyl-quinoline-3,4-diamine. LCMS (method B): Rt= 1.48 min, m/z= 3632 [M+H]⁺.

To a stirred solution of (rac)-chromane-4-carboxylic acid (182 mg, 0.97 mmol) in CH₂Cl₂ (10 mL) under N₂-atmosphere was added oxalyl chloride (172 µL, 1.94 mmol), followed by DMF (4 µL, 0.0516 mmol). The reaction was left to stir at rt for 45 min. The reaction mixture was concentrated *in vacuo* to give (rac)-chromane-4-carboxylic acid chloride which used directly in the next step.

To a stirred suspension of 8-(3,5-dichlorophenyl)-N4,N4-dimethyl-quinoline-3,4-diamine (0.237 g, 0.62 mmol) and NEt₃ (262 µL, 1.86 mmol) in CH₂Cl₂ (6 mL) under N₂ was added (rac)-chromane-4-carbonyl chloride (242 mg, 0.97 mmol) in CH₂Cl₂ (6 mL) dropwise. The reaction was left to stir at rt for 15 min. The reaction mixture was partitioned between sat. aq. NaHCO₃ and CH₂Cl₂ (25 mL of each). The combined layers were filtered and the aq. layer was separated and washed with CH₂Cl₂ (2x25 mL). The combined organic layers were concentrated *in vacuo* and the resulting residue was purified by column chromatography to give the title compounds. LCMS (method B): Rt= 1.58 min, m/z= 492 [M+H]⁺. Chiral SFC separation was performed on Chiralpak^{®} IG with column dimensions of 250 mm × 30 mm (5 µm), a flow rate of 118 g/min, and a CO₂-based mobile phase with 40% iPrOH containing 0.2% isopropylamine as additive to give (4R)-N-[8-(3,5-dichlorophenyl)-4-(dimethylamino)-3-quinolyl]chromane-4-carboxamide and (4S)-N-[8-(3,5-dichlorophenyl)-4-(dimethylamino)-3-quinolyl]chromane-4-carboxamide. ¹H-NMR (400 MHz, CDCl₃) δ [ppm]: 9.98 (s, 1H), 8.87 (s, 1 h), 7.86 (dd, J= 1.6, 8 Hz, 1 H), 7.51 (m, 4 H), 7.38 (t, J= 2 Hz, 1 H), 7.33 (t, J= 8.4Hz, 1 H), 7.04 (t, J=7.6 Hz, 1 H), 6.98 (d, J= 8 Hz, 1 H), 4.37 (d, J= 11.6 Hz, 1 H), 4.09 (td, J= 2, 12 Hz, 1 H), 3.88 (d, J= 2.8 Hz, 1 H), 2.7 (s, 6 H), 2.65 (m, 1 H), 2.25 (m, 1 H).

The following compounds were prepared analogously by the methodology of Example 1:

| Ex. | Name | Structure |
|---|---|---|
| 1.3 | (1R)-N-[8-(3,5-dichlorophenyl)-4-(dimethylamino)-3-quinolyl]tetralin-1-carboxamide | 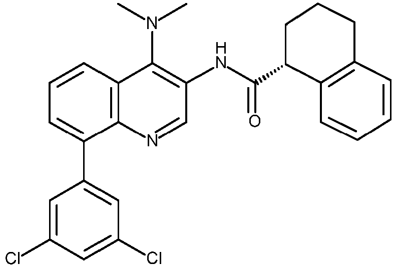 |
| 1.4 | (1S)-N-[8-(3,5-dichlorophenyl)-4-(dimethylamino)-3-quinolyl]tetralin-1-carboxamide | 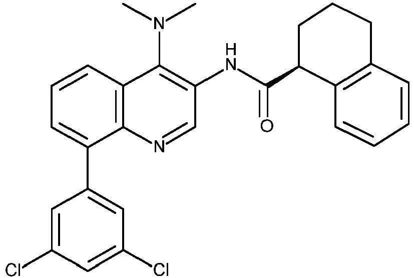 |
| 1.5 | (4R)-N-[4-cyclopropyl-8-(3,5 -dichlorophenyl)-3 - quinolyl]chromane-4-carboxamide | 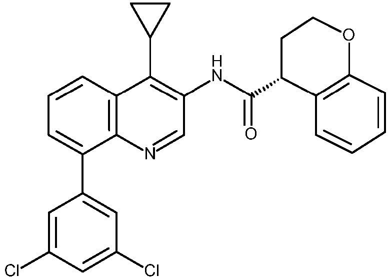 |
| 1.6 | (4S)-N-[4-cyclopropyl-8-(3,5 -dichlorophenyl)-3 - quinolyl]chromane-4-carboxamide | 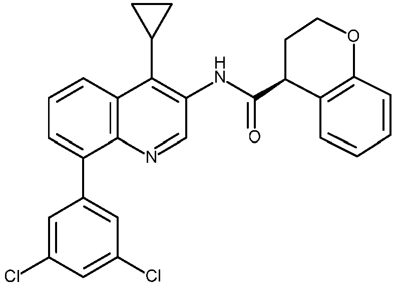 |
| 1.7 | (4R)-N-[8-(3,5-dichlorophenyl)-4-[methoxy(methyl)amino]-3-quinolyl]chromane-4-carboxamide | 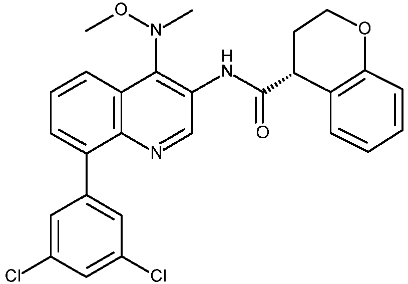 |
| 1.8 | (4S)-N-[8-(3,5-dichlorophenyl)-4-[methoxy(methyl)amino]-3-quinolyl]chromane-4-carboxamide | 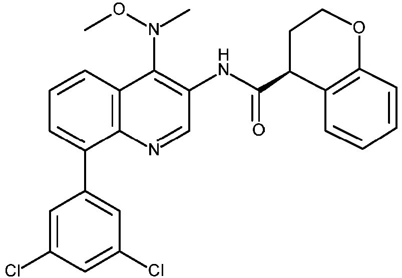 |
| 1.9 | (4S)-N-[8-(2,3-dichlorophenyl)-4-(dimethylamino)-3-quinolyl]chromane-4-carboxamide | 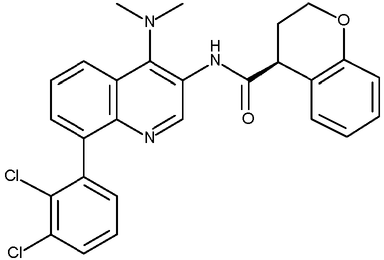 |
| 1.10 | (4R)-N-[8-[3-chloro-5-(trifluoromethyl)phenyl]-4-(dimethylamino)-3-quinolyl]chromane-4-carboxamide | 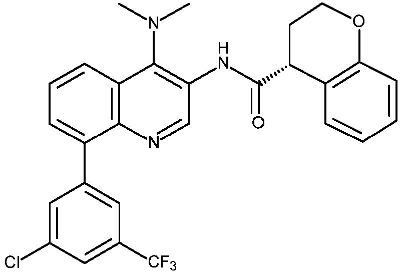 |
| 1.11 | (4S)-N-[8-[3-chloro-5-(trifluoromethyl)phenyl]-4-(dimethylamino)-3-quinolyl]chromane-4-carboxamide | 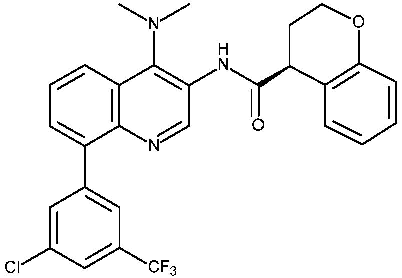 |
| 1.12 | (4S)-N-[8-(3,5-dichlorophenyl)-4-morpholino-3-quinolyl]chromane-4-carboxamide | 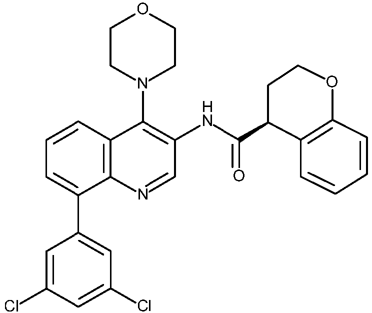 |
| 1.13 | (4R)-N-[8-(3,5-dichlorophenyl)-4-morpholino-3-quinolyl]chromane-4-carboxamide | 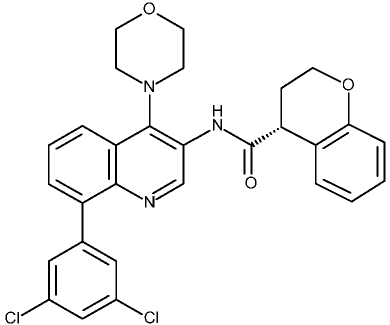 |
| 1.14 | (4S)-N-[4-morpholino-8-(2,3,5-trifluorophenyl)-3-quinolyl]chromane-4-carboxamide | 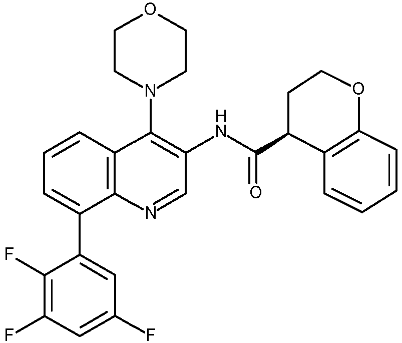 |
| 1.15 | (4R)-N-[4-morpholino-8-(2,3,5-trifluorophenyl)-3-quinolyl]chromane-4-carboxamide | 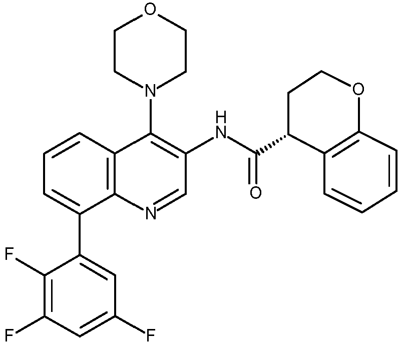 |

### Comparative Example 2.1

### 3-[1-[[(4R and S)-chroman-4-yl]amino]-2,2,2-trifluoro-ethyl]-8-(3,5-dichlorophenyl)-N,N-dimethyl-quinolin-4-amine

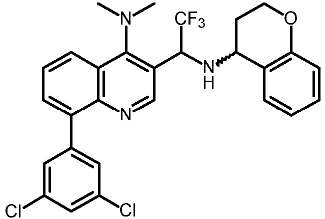

8-Bromoquinolin-4-ol (1 g, 4.46 mmol) was charged into a microwave vial and was suspended in POCl₃ (3 mL, 32.3 mmol). The mixture was submitted to microwave irradiation for 1 hour at 100°C. The reaction mixture was poured onto an ice/water mixture and was stirred for ca. 5 min. pH was adjusted to pH= 9 with solid K₂CO₃. The mixture was diluted with CH₂Cl₂ (100 mL). The layers were separated and the aq. layer was extracted with CH₂Cl₂ (2x25 mL). Combined organic layers were concentrated *in vacuo* to give 8-bromo-4-chloro-quinoline. LCMS (method B): Rt=1.13 min, m/z= 241.8 [M+H]⁺.

8-Bromo-4-chloro-quinoline (0.99 g, 4 mmol) was charged into a microwave vial and was dissolved in 1,4-dioxane (13 mL). To this solution dimethylamine•HCl (650 mg, 7.97 mmol) was added followed by addition of DIPEA (2.8 mL, 16 mmol). The mixture was submitted to microwave irradiation at 150°C for 2.5 hours. The reaction mixture was concentrated under reduced pressure and purified by column chromatography to give 8-bromo-N,N-dimethyl-quinolin-4-amine. LCMS (method B): Rt= 1.04 min, m/z= 251.0 [M+H]⁺.

4-Dimethlyaminopyridine (1.2 g, 9.8 mmol) was dissolved in o-xylene (16 mL). To this solution, trifluoroacetic anhydride (1.4 mL, 10 mmol) was added and the mixture was stirred for 30 min at rt. Then, a solution of 8-bromo-N,N-dimethyl-quinolin-4-amine (0.85 g, 3.3 mmol) in o-xylene (6 mL) was added to the flask and the mixture was stirred at reflux for 3 hours. The reaction mixture was cooled down to rt and was quenched by addition of sat. aq. NaHCO₃ solution. The layers were separated and the aq. layer was extracted with CH₂Cl₂ (2x25 mL). Combined organic layers were dried over anhydrous Na₂SO₄, filtered and reduced *in vacuo* to give crude material which was purified by column chromatography and afforded 1-[8-bromo-4-(dimethylamino)-3-quinolyl]-2,2,2-trifluoro-ethanone. LCMS (method A): Rₜ= 0.90 min, m/z= 346.8 [M+H]⁺. 1-[8-Bromo-4-(dimethylamino)-3-quinolyl]-2,2,2-trifluoro-ethanone (1.13 g, 2.93 mmol) was loaded into a microwave vial together with (3,5-dichlorophenyl)boronic acid (560 mg, 2.93 mmol), 1,1'-bis(diphenylphosphino) ferrocene-Pd(II)·CH₂Cl₂ complex (125 mg, 0.150 mmol) and Na₂CO₃ (620 mg, 5.85 mmol). Then, 1,4-dioxane (10 mL) and water (5 mL) was added to the vial and the mixture was submitted to microwave irradiation for 1 hour at 100°C. The crude reaction mixture was reduced to dryness, and then purified by column chromatography to give 1-[8-(3,5-dichlorophenyl)-4-(dimethylamino)-3-quinolyl]-2,2,2-trifluoro-ethanone. LCMS (method A): Rt= 1.34 min, m/z= 413.0 [M+H]⁺.

1-[8-(3,5-Dichlorophenyl)-4-(dimethylamino)-3-quinolyl]-2,2,2-trifluoro-ethanone (513 mg, 0.95 mmol) was dissolved in MeOH (3 mL). The solution was cooled down to 0°C and then NaBH₄ (72 mg, 1.90 mmol) was added to the flask. The mixture was stirred at 0°C for 15 min and then it was allowed to warm to rt. The reaction mixture was quenched by addition of water (50 mL). The layers were separated and the aq. layer was extracted with CH₂Cl₂ (2x25 mL). The combined organic layers were reduced to dryness *in vacuo* to give (1R)-1-[8-(3,5-dichlorophenyl)-4-(dimethylamino)-3-quinolyl]-2,2,2-trifluoro-ethanol. LCMS (method A): Rₜ= 1.34 min, m/z= 415.0 [M+H]⁺.

(1R)-1-[8-(3,5-Dichlorophenyl)-4-(dimethylamino)-3-quinolyl]-2,2,2-trifluoro-ethanol (360 mg, 0.67 mmol) and (4S)-chroman-4-amine•HCl (250 mg, 1.35 mmol) were dissolved in 1,4-dioxane (2.5 mL) and NEt₃ (0.1 mL, 0.72 mmol). Then, PPh₃ (880 mg, 3.32 mmol) and DIAD (0.660 mL, 3.33 mmol) were added to the flask at rt. The mixture was heated to 90°C overnight. The reaction mixture was cooled down to rt, reduced to dryness under vacuum and purified by chromatography (0-20% EtOAc in cyclohexane) to give the title compound. LCMS (method B): Rt=1.81 min, m/z= 546.0 [M+H]⁺. ¹H-NMR (400 MHz, DMSO-d₆) δ [ppm]: 9.14 (s, 1 H), 8.19 (dd, J= 0.8, 8.4 Hz, 1 H), 7.85 (t, J= 7.2 Hz, 1 H), 7.46-7.76 (m, 4 H), 7.41 (dd, J= 0.8, 7.6 Hz, 1 H), 7.1 (m, 1 H), 6.97 (dd, J= 0.8, 7.6 Hz, 1 H), 6.85 (t, J= 7.6 Hz, 1 H), 6.8 (t, J= 7.6 Hz, 1 H), 6.72 (t, J= 9.6 Hz, 1 H), 5.34 (m, 1 H), 4.29 (t, J= 10 Hz, 1 H), 3.97-4.19 (m, 2 H), 3.54-3.59 (m, 1 H), 3.06 (s, 3 H), 3.02 (s, 3 H), 1.64-2.04 (m, 2 H).

### Comparative Example 3.1

### N-[(4S)-chroman-4-yl]-8-(3,5-dichlorophenyl)-4-(dimethylamino)quinoline-3-sulfonamide

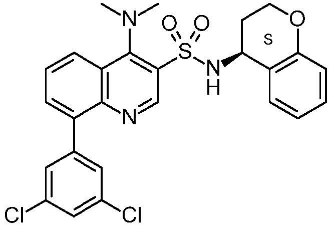

A solution of 8-bromoquinolin-4-ol (496.4 mg, 2.21 mmol) in chlorosulfonic acid (5 mL, 75.22 mmol) under N₂-atmosphere was heated to 100°C. The reaction was then cooled to rt and poured onto ice, causing solid to precipitate. This solid was filtered off, washed with water (50 mL) and air dried to afford a colorless solid. 8-Bromo-4-hydroxyquinoline-3-sulfonyl chloride was taken on to the next step without any further purification.

A suspension of 8-bromo-4-hydroxy-quinoline-3-sulfonyl chloride (571.9 mg, 1.59 mmol) in POCl₃ (5 mL, 53.8 mmol,) was heated to 130°C under N₂-atmosphere. The reaction was cooled to rt, then concentrated *in vacuo.* The residue was then azotroped with toluene (2x15 mL) to give 8-bromo-4-chloro-quinoline-3-sulfonyl chloride as a brown oil. LCMS (method B): Rₜ=1.27 min, m/z=340 [M+H]⁺.

To a chilled solution of 8-bromo-4-chloro-quinoline-3-sulfonyl chloride (985 mg, 2.54 mmol) in CH₂Cl₂ (10 mL) at 0°C under N₂-atmosphere was added (4S)-chroman-4-amine•HCl (365 mg, 1.96 mmol) followed by NEt₃ (0.54 mL, 3.9 mmol). The reaction was stirred at 0°C, and slowly allowed to warm to rt overnight. The reaction mixture was concentrated *in vacuo* and the solid was then purified by column chromatography to give 8-bromo-4-chloro-N-[(4S)-chroman-4-yl]quinoline-3-sulfonamide. LCMS (method B): Rₜ=1.25 min, m/z=453 [M+H]⁺.

A mixture of 8-bromo-4-chloro-N-[(4S)-chroman-4-yl]quinoline-3-sulfonamide (420 mg, 0.88 mmol), dimethylamine•HCl (144 mg, 1.77 mmol) and DIPEA (0.62 mL, 3.6 mmol) in 1,4-dioxane (4 mL) in a 5 mL microwave vial was irradiate to 100°C for 1 hour. The reaction was concentrated, and the resulting residue was purified by column chromatography to give 8-bromo-N-[(4S)-chroman-4-yl]-4-(dimethylamino)quinoline-3-sulfonamide. LCMS (method B): Rₜ= 1.26 min, m/z=462 [M+H]⁺.

To a mixture of (3,5-dichlorophenyl) boronic acid (158.3 mg, 0.83 mmol), Na₂CO₃ (192 mg, 1.81 mmol) and 1,1'-bis(diphenylphosphino) ferrocene-Pd(II)·CH₂Cl₂ complex (38.8 mg, 0.047 mmol) in a 5mL microwave vial was added 8-bromo-N-[(4S)-chroman-4-yl]-4-(dimethylamino)quinoline-3-sulfonamide (383 mg, 0.78 mmol) in 1,4-dioxane (3 mL) followed by water (1.5 mL). The microwave vial was then sealed and irradiated to 100°C for 1 hour. The reaction was diluted with EtOAc (20 mL) and passed through a pad of Celite^{®}. The Celite^{®} was washed through with EtOAc (100 mL). The combined filtrates were then concentrated *in vacuo* and the residue was purified by column chromatography to afford the title compound. LCMS (method B): Rt=1.58 min, m/z=528.1 [M+H]⁺. 1H-NMR (400 MHz, DMSO-d6) δ [ppm]: 9.15 (s, 1H), 8.50 (d, J= 8.4 Hz, 1 h), 8.29 (dd, J= 1.2, 8.4 Hz, 1 H), 7.93 (dd, J= 1.2, 7.2 Hz, 1 H), 7.75 (t, J= 7.2 Hz, 1 H), 7.67 (s, 3 H), 7.12 (m, 2 H), 6.75 (m, 2 H), 4.58 (q, J= 7.8 Hz, 1 H), 4.18 (m, 2 H), 3.18 (s, 6 H), 1.87 (q, J= 5.8 Hz, 2 H).

The following compounds were prepared analogously by the methodology of Comparative Example 3.1:

| Ex. | Name | Structure |
|---|---|---|
| 3.2 Comparative Example | N-[8-(3,5-dichlorophenyl)-4-(dimethylamino)-3-quinolyl]-(4R and S)-chromane-4-sulfonamide | 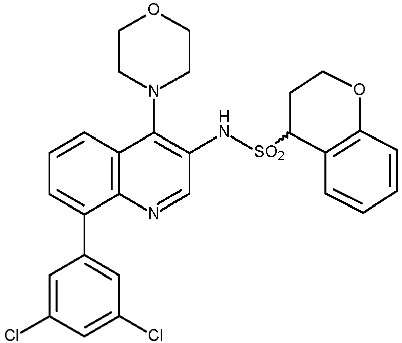 |

### Example 4.1

### (4S)-N-[8-(3,5-dichlorophenyl)-4-(dimethylamino)-1,7-naphthyridin-3-yl]chromane-4-carboxamide

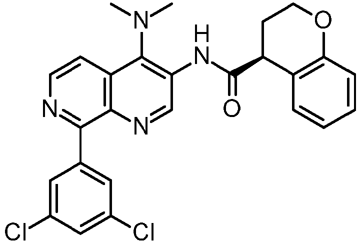

A solution of 2-chloro-3-fluoro-pyridine-4-carboxylic acid (15.1 g, 84.2 mmol) and thionyl chloride (60 mL, 821 mmol,) was heated to 80°C for 2 hours. The reaction was allowed to cool to rt, and concentrated *in vacuo* to give 2-chloro-3-fluoro-pyridine-4-carbonyl chloride as a residue which was used directly in the next step.

To a stirring solution of 2-chloro-3-fluoro-pyridine-4-carbonyl chloride (17.5 g, 83.9 mmol) in toluene (210 mL) was added NEt₃ (14 mL, 99.4 mmol) followed by ethyl 3-(dimethylamino)prop-2-enoate (14.7 g, 101 mmol). The reaction was heated to 80°C and left to stir for 45 min. The reaction was allowed to cool to rt, and filtered through Celite^{®} (washing with EtOAc). The filtrate was concentrated *in vacuo,* and the residue was partitioned between EtOAc and aq. 2M HCl (200 mL of each). The layers were separated, and the aq. layer was extracted with EtOAc (200 mL). The combined organics were dried over anhydrous MgSO₄, filtered and concentrated *in vacuo* to give ethyl 2-(2-chloro-3-fluoro-pyridine-4-carbonyl)-3-[(4-methoxyphenyl)methylamino]prop-2-enoate. LCMS (method B) Rₜ=0.86min, m/z= 301.0 [M+H]⁺.

To a stirring solution of ethyl 2-(2-chloro-3-fluoro-pyridine-4-carbonyl)-3-[(4-methoxyphenyl)methylamino]prop-2-enoate (19.37 g, 46.8 mmol) in DMF (90 mL) was added K₂CO₃ (19.3 g, 140 mmol). The reaction was heated to 50°C and left to stir for 2 hours. The reaction was allowed to cool to rt and was concentrated under vacuum. The mixture was then partitioned between EtOAc and water. The aq. layer was extracted with EtOAc (2x), and the combined organics were washed with, brine, dried over anhydrous MgSO₄, and filtered. The combined organic layers were concentrated *in vacuo* and the residue was triturated with Et₂O. The solvent was removed under reduced pressure to give ethyl 8-chloro-1-[(4-methoxyphenyl)methyl]-4-oxo-1,7-naphthyridine-3-carboxylate. LCMS (method B) Rₜ= 1.02, m/z= 373.0 [M+H]⁺.

To a stirring solution of ethyl 8-chloro-1-[(4-methoxyphenyl)methyl]-4-oxo-1,7-naphthyridine-3-carboxylate (9.00 g, 22.93 mmol) in CH₂Cl₂ (115 mL) was added anisole (12.5 mL, 114 mmol), followed by TFA (35 mL, 458.3 mmol). The reaction was left to stir at rt for 1 hour. The reaction was concentrated *in vacuo* and the crude product was partitioned between sat. aq. NaHCO₃ and EtOAc (300 mL of each). The suspension was stirred vigorously for 15 min. The precipitate was isolated by filtration (washing with water, then EtOAc) to give ethyl 8-chloro-4-hydroxy-1,7-naphthyridine-3-carboxylate. LCMS (method B) Rₜ= 0.47min, m/z= 253.0 [M+H]⁺.

To a stirring suspension of ethyl 8-chloro-4-hydroxy-1,7-naphthyridine-3-carboxylate (4.76 g, 18.85 mmol) in CH₂Cl₂ (190 mL) was added oxalyl chloride (2.5 mL, 28 mmol), followed by DMF (73 µL, 0.94 mmol) at 0°C. The reaction was left to stir at rt for 4 hours. The reaction was quenched by the addition into NaHCO₃ (200mL), and the reaction mixture was extracted twice with CH₂Cl₂. The combined organics were dried over anhydrous MgSO₄, filtered and concentrated *in vacuo* to give ethyl 4,8-dichloro-1,7-naphthyridine-3-carboxylate. LCMS (method B) Rt= 1.07 min, m/z= 271.0 [M+H]⁺.

To ethyl 4,8-dichloro-1,7-naphthyridine-3-carboxylate (5.23 g, 18.33 mmol) in THF (18 mL), dimethylamine in THF (18 mL, 36 mmol, 2 M) was added, immediately forming a yellow solution with precipitate. The reaction was left to stir at rt for 20 min. EtOAc (250 mL) was added followed by aq. NaHCO₃ (200 mL). The layers were separated, and the aq. layer was extracted with EtOAc (250mL). The combined organics were dried over anhydrous MgSO₄, filtered and concentrated *in vacuo* to give ethyl 8-chloro-4-(dimethylamino)-1,7-naphthyridine-3-carboxylate. LCMS (method B) Rt= 0.96 min, m/z= 280.0 [M+H]⁺.

To a mixture of ethyl 8-chloro-4-(dimethylamino)-1,7-naphthyridine-3-carboxylate (3.00 g, 10.2 mmol), (3,5-dichlorophenyl)boronic acid (1.89 g, 9.68 mmol), Na₂CO₃ (2.12 g, 20.4 mmol) was added 1,4-dioxane (50 mL), followed by water (17 mL). N₂-gas was bubbled through the mixture for 5 min. 1,1'-Bis(diphenylphosphino) ferrocene-Pd(II)·CH₂Cl₂ complex (380 mg, 0.51 mmol) was then added and the reaction was heated at 80°C for 90 min. The reaction was cooled to rt and diluted with CH₂Cl₂ /water. The layers were separated. The CH₂Cl₂ layer was collected and the solvent was removed under reduced pressure to give the crude product. The crude product was purified by column chromatography (0-40% EtOAc:cyclohexane) to give ethyl 8-(3,5-dichlorophenyl)-4-(dimethylamino)-1,7-naphthyridine-3-carboxylate. LCMS (method B) Rₜ= 1.52 min, m/z= 390.0 [M+H]⁺.

To a solution of ethyl 8-(3,5-dichlorophenyl)-4-(dimethylamino)-1,7-naphthyridine-3-carboxylate (4.46 g, 9.71 mmol) in THF (95 mL) and MeOH (32 mL) was added lithium hydroxide (712 mg, 29.1 mmol) in water (32 mL). The reaction was heated to 50°C and left to stir for 4 hours. The mixture was concentrated under reduced pressure, water was added and the aq. layer was washed with EtOAc. The aq. layer was adjusted to pH 4 by the addition of 2 M HCl, forming a yellow precipitate which was isolated by filtration to give 8-(3,5-dichlorophenyl)-4-(dimethylamino)-1,7-naphthyridine-3-carboxylic acid. LCMS analysis (method B) Rt= 0.78 min, m/z= 362.0 [M+H]⁺ .

A suspension of 8-(3,5-dichlorophenyl)-4-(dimethylamino)-1,7-naphthyridine-3-carboxylic acid (0.42 g, 0.93 mmol) in DMF (10 mL) was placed under N₂-atmosphere and treated with NEt₃ (2.9 mmol, 0.4 mL). The resulting solution was treated with diphenylphosphoryl azide (0.41 g, 1.5 mmol, 0.325 mL) before being allowed to stir for 45 min at rt. The reaction mixture was then treated with MeOH (4 mL) and was left to stir overnight. The reaction mixture was quenched by the addition of sat. aq. NaHCO₃ (25 mL) and extracted with CH₂Cl₂ (3x20 mL). The combined organic layers were filtered and concentrated *in vacuo.* The residue containing methyl N-[8-(3,5-dichlorophenyl)-4-(dimethylamino)-1,7-naphthyridin-3-yl]carbamate was carried into the next step without further purification.

A solution of methyl N-[8-(3,5-dichlorophenyl)-4-(dimethylamino)-1,7-naphthyridin-3-yl]carbamate (1.23 g, 0.932 mmol) in 1,4-dioxane (10 mL) was treated with sodium hydroxide (2 mol/L) in water (10 mL) and was heated at 100°C for 2 hours. The reaction mixture was allowed to cool to rt, diluted with water (20 mL) and extracted with CH₂Cl₂ (3x 20mL). The combined organic layers were filtered and concentrated *in vacuo.* The residues were purified by column chromatography (0-25% EtOAc in cyclohexane) to give 8-(3,5-dichlorophenyl)-N,N-dimethyl-1,7-naphthyridine-3,4-diamine. LCMS analysis (method B) Rₜ= 1.29 min, m/z= 333.0 [M+H]⁺.

A mixture of 8-(3,5-dichlorophenyl)-N,N-dimethyl-1,7-naphthyridine-3,4-diamine (105 mg, 0.3 mmol) and S-chromane-4-carboxylic acid (0.065 g, 0.35 mmol) was placed under N₂-atmosphere, treated with EtOAc (1.5 mL) and cooled over ice. The reaction mixture was then treated with pyridine (0.075 mL, 0.918 mmol) and T₃P (50 wt% in EtOAc; 0.608 mmol, 0.365 mL) before being allowed to stir overnight. The reaction mixture was diluted with water (20 mL) and extracted with CH₂Cl₂ (3x15 mL). The combined organic layers were filtered and concentrated *in vacuo.* The residues were dissolved in MeOH, filtered and purified by prep-HPLC ((Phenomenex Gemini 5 Micron 30x100mm C-18) (Acetonitrile and water adjusted to approx. pH 9 with conc. ammonium hydroxide solution [0.5 mL of conc. ammonium hydroxide per 2.5 L of water], 30% to 100% CH₃CN over 9 min at 60 mL/min) to give the title compound. LCMS analysis (method B) Rₜ= 1.47 min, m/z= 493.0 [M+H]⁺. ¹H-NMR (400 MHz, DMSO-d₆) δ [ppm]: 10.15 (s, 1 H), 8.8 (s, 1 H), 8.63 (d, J= 5.6 Hz, 1 H), 8.09 (d, J= 2 Hz, 2 H), 8.00 (d, J= 2.8 Hz, 1 H), 7.72 (t, J= 2 Hz, 1 H), 7.27 (d, J= 7.6 Hz, 1 H), 7.16 (m, 1 H), 6.91 (m, 1 H), 6.82 (d, J= 8 Hz, 1 H), 4.38 (m, 1 H), 4.2 (m, 1 H), 4.08 (t, J= 6 Hz, 1 H), 3.04 (s, 6 H), 2.25 (m, 2 H).

The following compounds were prepared analogously by the methodology of Example 4.1:

| Ex. | Name | Structure |
|---|---|---|
| 4.2 | (4R)-N-[8-(3,5-dichlorophenyl)-4-(dimethylamino)-1,7-naphthyridin-3-yl]chromane-4-carboxamide | 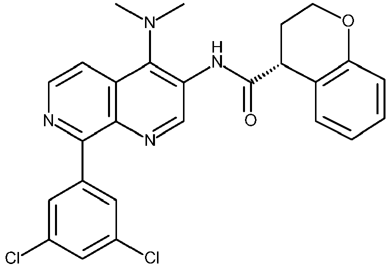 |
| 4.3 | (4S)-N-[4-morpholino-8-(2,3,5-trifluorophenyl)-1,7-naphthyridin-3-yl]chromane-4-carboxamide | 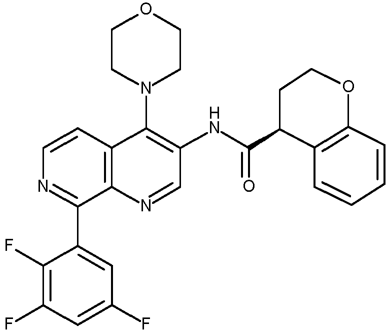 |
| 4.4 | (4R)-N-[4-morpholino-8-(2,3,5-trifluorophenyl)-1,7-naphthyridin-3-yl]chromane-4-carboxamide | 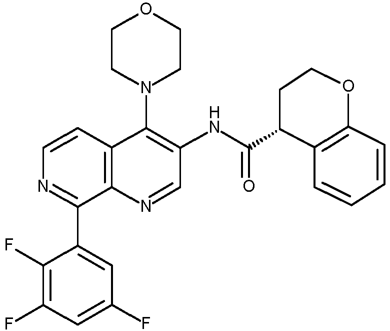 |

### Example 5.1

### (4S)-N-[4-(3,5-dichlorophenyl)-8-(dimethylamino)pyrido[3,2-d]pyrimidin-7-yl]chromane-4-carboxamide

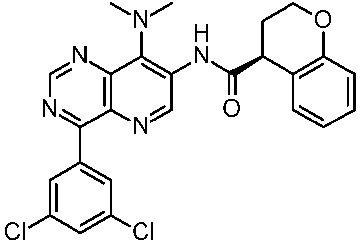

To a suspension of ethyl 6-hydroxypyrimidine-4-carboxylate (5.04 g, 28.7 mmol) in DMF (25 mL, 323 mmol) under N₂-atmosphere was added 1,3-dichloro-5,5-dimethlyhydantoin (3.48 g, 17.3 mmol). The mixture was stirred overnight at rt. The reaction was partitioned between water (200 mL) and EtOAc (100 mL). The aq. layer was separated and extracted with EtOAc (2x75 mL). The combined organic layers were dried over anhydrous Na₂SO₄, filtered and concentrated *in vacuo.* LCMS (method B) Rₜ= 0.54 min, m/z=203.0 [M+H]⁺.

To a suspension of ethyl 5-chloro-6-hydroxy-pyrimidine-4-carboxylate (8.74 g, 28.1 mmol) in CH₃CN (100 mL) at rt under N₂-atmosphere was added DIPEA (6.4 mL, 36 mmol) and then phosphorous oxybromide (9.44 g, 31.28 mmol). The resulting mixture was stirred at rt. The reaction was diluted with CH₂Cl₂ (100 mL) and slowly poured into water (100 mL). The mixture was then extracted with CH₂Cl₂ (3x100 mL). The combined organic layers were dried over anhydrous Na₂SO₄, filtered and concentrated *in vacuo.* The oil was purified by column chromatography (0-10% EtOAc in cyclohexane) to give ethyl 6-bromo-5-chloro-pyrimidine-4-carboxylate. LCMS (method B) Rₜ= 0.98 min, m/z= 265.0 [M+H]⁺.

To a stirred solution of ethyl 6-bromo-5-chloro-pyrimidine-4-carboxylate (4.31 g, 14.9 mmol) and (3,5-dichlorophenyl) boronic acid ( 2.71 g, 14.20 mmol) in 1,4-dioxane (55 mL) under N₂-atmosphere was added K₂CO₃ (8.69 g, 62.87 mmol) followed by tetrakis (triphenylphosphine) palladium (0) (732 mg, 0.63 mmol). The reaction was degassed and put under N₂-atmosphere, then heated at 90°C for 16 hours. The mixture was diluted with EtOAc (50 mL) and passed through Celite^{®}. The combined organic filtrates were concentrated *in vacuo.* The residue was purified by column chromatography (0-20% EtOAc in cyclohexane) to give ethyl 5-chloro-6-(3,5-dichlorophenyl)pyrimidine-4-carboxylate. LCMS (method A) Rₜ= 1.43 min, m/z= 331.0 [M+H]⁺.

To a mixture of ethyl 5-chloro-6-(3,5-dichlorophenyl)pyrimidine-4-carboxylate (2.90 g, 8.75 mmol) in THF (85 mL) and water (30 mL) at rt under N₂-atmosphere was added lithium hydroxide (624.4 mg, 25.55 mmol). The resulting mixture was heated to 50°C for 1 hour. The reaction was cooled to rt, then concentrated *in vacuo* to remove THF. The resulting solution was diluted with water (50 mL) then acidified with 2M HCl until the pH=1, causing a solid to precipitate. The precipitate was filtered off and washed with water (25 mL). The precipitate was then dried *in vacuo* at 50°C to give 5-chloro-6-(3,5-dichlorophenyl)pyrimidine-4-carboxylic acid. LCMS (method A) Rₜ= 0.72 min, m/z= 303.0 [M+H]⁺.

A suspension of 5-chloro-6-(3,5-dichlorophenyl)pyrimidine-4-carboxylic acid (2.49 g, 7.82 mmol) in thionyl chloride (30 mL, 411 mmol) was heated to 80°C under N₂-atmosphere. DMF (0.5 mL, 6 mmol) was added and the reaction fully dissolved. The reaction was then concentrated *in vacuo.* The residue was taken up in toluene (20 mL) and azotroped (3 x) to give 5-chloro-6-(3,5-dichlorophenyl)pyrimidine-4-carbonyl chloride which was used in the next step without further purification.

To a solution of 5-chloro-6-(3,5-dichlorophenyl)pyrimidine-4-carbonyl chloride (2.65 g, 7.82 mmol) in toluene (20 mL) at rt under N₂-atmosphere was added NEt₃ (2 mL, 14 mmol) followed by ethyl 3-(dimethylamino)prop-2-enoate (1.4 mL, 9.7 mmol). The reaction was stirred at rt under N₂-atmosphere. The reaction was diluted with EtOAc (125 mL), filtered through Celite^{®}. The Celite^{®} was washed through with EtOAc (125 mL). The combined organic filtrates were concentrated *in vacuo.* The residues were taken up in EtOAc (250 mL) and 2M HCl (aq, 100 mL). The aq. layer was separated and extracted with EtOAc (125 mL). The combined organic layers were dried over anhydrous Na₂SO₄, filtered and concentrated *in vacuo* to give ethyl 2-[5-chloro-6-(3,5-dichlorophenyl)pyrimidine-4-carbonyl]-3-(dimethylamino)prop-2-enoate. LCMS (method A) Rₜ= 1.24 min, m/z= 428.0 [M+H]⁺.

4-Methoxybenzylamine (1.20 mL, 9.09 mmol) was added to a solution of ethyl 2-[5-chloro-6-(3,5-dichlorophenyl)pyrimidine-4-carbonyl]-3-(dimethylamino)prop-2-enoate (3.59 g, 6.29 mmol) in diethyl ether (25 mL) and EtOH (6 mL) at rt under N₂-atmopshere for 1 hour. The reaction was diluted with water (150 mL) and extracted with CH₂Cl₂ (4x75 mL). The combined organic layers were washed with brine (100 mL), dried over anhydrous Na₂SO₄, filtered and concentrated *in vacuo* to give ethyl 2-[5-chloro-6-(3,5-dichlorophenyl)pyrimidine-4-carbonyl]-3-[(4-methoxyphenyl) methylamino] prop-2-enoate which was taken on without further purification. LCMS (method A) Rₜ= 1.49 min, m/z= 520.0 [M+H]⁺.

To a solution of ethyl 2-[5-chloro-6-(3,5-dichlorophenyl)pyrimidine-4-carbonyl]-3-[(4-methoxyphenyl) methylamino] prop-2-enoate (4.4 g, 5.66 mmol) in DMF (15 mL, 194 mmol) at rt under N₂-atmosphere was added K₂CO₃ (2.37 g, 17.1 mmol). The resulting mixture was heated to 90°C for 24 hours. The reaction was cooled to rt, then poured into water (300 mL) and extracted with CH₂Cl₂ (3x100 mL). The combined organic layers were washed with brine (200 mL), dried over anhydrous Na₂SO₄, filtered and concentrated *in vacuo.* The residue was purified by column chromatography (0-5% MeOH in CH₂Cl₂) to give ethyl 4-(3,5-dichlorophenyl)-5-[(4-methoxyphenyl)methyl]-8-oxo-pyrido[3,2-d] pyrimidine-7-carboxylate. LCMS (method A) Rₜ= 1.17 min, m/z= 484.0 [M+H]⁺.

To a solution of ethyl 4-(3,5-dichlorophenyl)-5-[(4-methoxyphenyl)methyl]-8-oxo-pyrido[3,2-d] pyrimidine-7-carboxylate (1.89 g, 3.70 mmol) in CH₂Cl₂ (75 mL) and DMF (0.5 mL, 6 mmol) at rt under N₂-atmosphere was added slowly oxalyl chloride (2 mL, 23.1 mmol). The reaction was heated to reflux at 60°C for 1 hour. The reaction was cooled down to rt, then quenched by the addition of sat. aq. NaHCO₃ (200 mL) and extracted with CH₂Cl₂ (3x100 mL). The combined organic layers were combined and concentrated *in vacuo* to give ethyl 8-chloro-4-(3,5-dichlorophenyl)pyrido[3,2-d]pyrimidine-7-carboxylate. LCMS (method A) Rₜ= 1.52 min, m/z= 382.0 [M+H]⁺.

To a suspension of ethyl 8-chloro-4-(3,5-dichlorophenyl)pyrido[3,2-d]pyrimidine-7-carboxylate (1.04 g, 1.93 mmol) in THF (10 mL) at rt under N₂-atmosphere was added dimethylamine in THF (2 mL, 4 mmol, 2 M). The reaction was stirred at rt for 1 hour. The mixture was poured into sat. aq. NaHCO₃ (50 mL) and extracted with CH₂Cl₂ (3x25 mL). The combined organic layers were dried over anhydrous Na₂SO₄, filtered and concentrated *in vacuo.* The residue was purified by column chromatography (0-15% EtOAc in cyclohexane) to give ethyl 4-(3,5-dichlorophenyl)-8-(dimethylamino) pyrido [3,2-d]pyrimidine-7-carboxylate. LCMS (method B) Rₜ=1.58 min, m/z= 391.0 [M+H]⁺. To a mixture of ethyl 4-(3,5-dichlorophenyl)-8-(dimethylamino) pyrido [3,2-d]pyrimidine-7-carboxylate (571 mg, 1.34 mmol) in 1,4-dioxane (14 mL) and water (4.5 mL) at rt under N₂-atmosphere was added lithium hydroxide (107 mg, 4.375 mmol). The resulting mixture was heated to 80°C for 4 hours. The reaction was concentrated *in vacuo* to remove dioxane. The residue was taken up in water (20 mL) and acidified with 2M HCl (aq, 2.2 mL). The resulting yellow precipitate was filtered off and washed with water (20 mL). The yellow solid was then dried *in vacuo* at 60°C to give 4-(3,5-dichlorophenyl)-8-(dimethylamino)pyrido[3,2-d]pyrimidine-7-carboxylic acid. LCMS (method B) Rₜ=0.78 min, m/z= 363 [M+H]⁺.

To a solution of 4-(3,5-dichlorophenyl)-8-(dimethylamino)pyrido[3,2-d]pyrimidine-7-carboxylic acid (209 mg, 0.52 mmol) in DMF (5.2 mL) at rt under N₂-atmosphere was added NEt₃ (0.22 mL, 1.6 mmol) followed by diphenylphosphoryl azide (0.17 mL, 0.79 mmol). After 2 hours at rt, anhydrous MeOH (2.1 mL, 52 mmol) was added and the reaction was left to stir at rt overnight under N₂-atmosphere.

The reaction was quenched by the addition of sat. aq. NaHCO₃ (25 mL) and then extracted with CH₂Cl₂ (3x50 mL). The combined organic layers were dried over anhydrous Na₂SO₄, filtered and concentrated *in vacuo* to give methyl N-[4-(3,5-dichlorophenyl)-8-(dimethylamino)pyrido[3,2-d]pyrimidin-7-yl]carbamate. LCMS (method B) Rₜ=1.47 min, m/z=392 [M+H]⁺.

A solution of methyl N-[4-(3,5-dichlorophenyl)-8-(dimethylamino)pyrido[3,2-d]pyrimidin-7-yl]carbamate (0.517 mmol, 0.517 mmol) in 1,4-dioxane (5.2 mL) at rt under N₂-atmopshere was treated with sodium hydroxide (aq., 2 M). The mixture was then heated to 100°C. The reaction was cooled to rt, diluted with water (25 mL) and extracted with CH₂Cl₂ (3x50 mL). The combined organic layers were dried over anhydrous Na₂SO₄, filtered and concentrated *in vacuo.* The residue was purified by column chromatography (0-25% EtOAc in cyclohexane) to give 4-(3,5-dichlorophenyl)-N,N-dimethyl-pyrido[3,2-d]pyrimidine-7,8-diamine. LCMS (method B) Rt=1.41 min, m/z=334.0 [M+H]⁺.

A suspension of 4-(3,5-dichlorophenyl)-N,N-dimethyl-pyrido[3,2-d]pyrimidine-7,8-diamine (114 mg, 0.32 mmol) and (4S)-chromane-4-carboxylic acid (69.4 mg, 0.39 mmol) in EtOAc (5 mL) was under N₂-atmosphere cooled to 0°C with an ice bath. Then pyridine (0.08 mL, 1 mmol) was added to the reaction at 0°C followed by T₃P (50 wt% in EtOAc; 0.39 mL, 0.65 mmol). The reaction was allowed to slowly warm to rt and was stirred overnight. The reaction mixture was diluted with water (25 mL) and extracted with CH₂Cl₂ (3x25 mL). The combined organic layers were dried over anhydrous Na₂SO₄, filtered and concentrated *in vacuo.* The oil was purified by column chromatography (0-15% EtOAc in cyclohexane) to give the title compound. LCMS (method B) Rt=1.61 min, m/z= 494.0 [M+H]⁺. ¹H NMR (400 MHz, DMSO) δ [ppm] 10.15 (s, 1 H), 9.32 (s, 1 H), 8.93 (s, 1 H), 8.29 (d, J= 1.6Hz, 2 H), 7.84 (t, J= 2 Hz, 1 H), 7.27 (d, J= 7.2 Hz, 1 H), 7.17 (t, J= 7.2 Hz, 1 H), 6.91 (t, J= 7.2 Hz, 1 H), 9.82 (d, J= 8 Hz, 1 H), 4.33-4.41 (m, 1 H), 4.16-4.24 (m, 1 H), 4.11 (t, J= 7.2 Hz, 1 H), 3.17 (s, 6 H), 2.17-2.27 (m , 2 H).

The following compounds were prepared analogously by the methodology of Example 5.1:

| Ex. | Name | Structure |
|---|---|---|
| 5.2 | (4R)-N-[4-(3,5-dichlorophenyl)-8-(dimethylamino)pyrido[3,2-d]pyrimidin-7-yl]chromane-4-carboxamide | 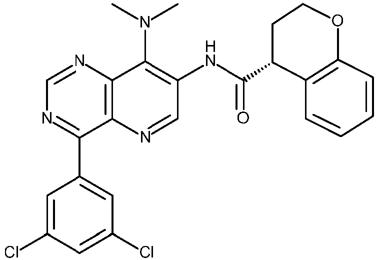 |

### Example 6.1

### (4R)-N-[8-(3,5-dichlorophenyl)-4-(dimethylamino)-1,5-naphthyridin-3-yl]chromane-4-carboxamide

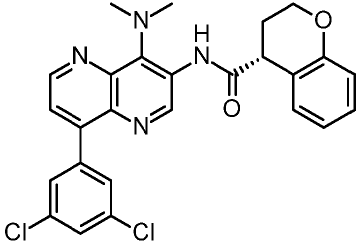

Thionyl chloride (15 mL, 205 mmol) was added to 3,4-dichloropyridine-2-carboxylic acid (3.96 g, 20.6 mmol) and the reaction was heated at 80°C for 1 hour. The reaction was allowed to cool to rt, and concentrated *in vacuo* to give 3,4-dichloropyridine-2-carbonyl chloride which was used without further purification in the next step.

To a solution of 3,4-dichloropyridine-2-carbonyl chloride (20.6 mmol, 4.76 g) in toluene (50 mL) was added NEt₃ (3.5 mL, 2.5 g) followed by ethyl 3-(dimethylamino)prop-2-enoate (3.6 mL, 25 mmol). The reaction was stirred at rt overnight. The reaction was filtered through Celite^{®} (washing with EtOAc). The filtrate was concentrated *in vacuo,* and the residue was partitioned between EtOAc and aq. 1 M HCl (100 mL of each). The layers were separated, and the aq. layer was extracted with EtOAc (50 mL). The combined organic layers were concentrated *in vacuo* to give ethyl 2-(3,4-dichloropyridine-2-carbonyl)-3-(dimethylamino)prop-2-enoate. LCMS (method B) Rₜ= 0.88 min, m/z= 317.0 [M+H]⁺.

To a stirring solution of ethyl 2-(3,4-dichloropyridine-2-carbonyl)-3-(dimethylamino) prop-2-enoate (5.58 g, 12.7 mmol) in diethyl ether (50 mL) and EtOH (12 mL) was added 4-methoxybenzylamine (1.9 mL, 14 mmol). The reaction was left to stir at rt for 2 hours. The reaction mixture was diluted with water (100 mL). Layers were separated and the aq. layer was extracted with CH₂Cl₂ (3x50 mL). The combined organic layers were dried over anhydrous Na₂SO₄, filtered and concentrated *in vacuo* to give ethyl 2-(3,4-dichloropyridine-2-carbonyl)-3-[(4-methoxyphenyl) methylamino] prop-2-enoate.

Ethyl 2-(3,4-dichloropyridine-2-carbonyl)-3-[(4-methoxyphenyl) methylamino] prop-2-enoate (5.92 g, 9.40 mmol) was dissolved in DMF (24 mL). K₂CO₃ (4.00 g, 28.9 mmol) was added and the mixture was stirred at 90°C for 6 hours. The reaction was cooled down to rt. The reaction mixture was quenched by addition of water (250 mL) and was diluted with CH₂Cl₂ (100 mL). The layers were separated and the aq. layer was extracted with CH₂Cl₂ (2x50 mL). The combined organic layers were filtered through Celite^{®} and then washed with brine (100 mL), dried over anhydrous Na₂SO₄, filtered and concentrated *in vacuo.* The crude material was purified by chromatography (0-6% MeOH in CH₂Cl₂) to give ethyl 8-hydroxy-1-[(4-methoxyphenyl) methyl]-4-oxo-1,5-naphthyridine-3-carboxylate.

Ethyl 8-hydroxy-1-[(4-methoxyphenyl) methyl]-4-oxo-1,5-naphthyridine-3-carboxylate (840 mg, 1.09 mmol) was dissolved in CH₂Cl₂ (11 mL) and DMF (0.05 mL). To this mixture, oxalyl chloride (0.48 mL, 5.5 mmol) was added and the mixture was heated to reflux for 3 hours. The reaction mixture was then cooled down and was quenched by addition of sat. aq. NaHCO₃ solution (50 mL). The layers were separated and the aq. layer was extracted with CH₂Cl₂ (2x25 mL). The combined organic layers were reduced *in vacuo* to give ethyl 4,8-dichloro-1,5-naphthyridine-3-carboxylate.

Ethyl 4,8-dichloro-1,5-naphthyridine-3-carboxylate (950 mg, 2.21 mmol) was dissolved in THF (5 mL). To this solution, dimethylamine in THF (1.1 mL, 2.2 mmol, 2 M) was added dropwise and the mixture was stirred at rt for 30 min. The reaction mixture was concentrated under reduced pressure and the resulting residue was purified by chromatography (20-50% EtOAc in cyclohexane) to give ethyl 8-chloro-4-(dimethylamino)-1,5-naphthyridine-3-carboxylate. LCMS (method B) Rₜ= 1.07 min, m/z= 280.0 [M+H]⁺.

Ethyl 8-chloro-4-(dimethylamino)-1,5-naphthyridine-3-carboxylate (315 mg, 0.93 mmol) was dissolved in 1,4-dioxane (3 mL) and water (1 mL). To this mixture, 1,1'-bis(diphenylphosphino) ferrocene] dichloropalladium(II) (40 mg, 0.048 mmol) was added, followed by addition of (3,5-dichlorophenyl)boronic acid (215 mg, 1.13 mmol) and Na₂CO₃ (300 mg, 2.83 mmol). The mixture was submitted to microwave irradiation for 1 hour at 100°C. The crude reaction mixture was reduced to dryness and the residue was purified by column chromatography (5-40% EtOAc in cyclohexane) to give ethyl 8-(3,5-dichlorophenyl)-4-(dimethylamino)-1,5-naphthyridine-3-carboxylate. LCMS (method B) Rₜ= 1.56 min, m/z= 390.0 [M+H]⁺.

To a stirring solution of ethyl 8-(3,5-dichlorophenyl)-4-(dimethylamino)-1,5-naphthyridine-3-carboxylate (272 mg, 0.65 mmol) in 1,4-dioxane (2 mL) was added lithium hydroxide (32 mg, 1.34 mmol) in water (2 mL). The reaction was heated to 100°C overnight. The reaction mixture was cooled down. The reaction mixture was quenched by addition of water (50 mL) and EtOAc (50 mL). pH was adjusted to pH= 4 with 2M HCl. The layers were separated and the aq. layer was extracted with EtOAc (2x50 mL). The combined organic layers were washed with brine, dried over anhydrous Na₂SO₄, filtered and reduced *in vacuo* to give 8-(3,5-dichlorophenyl)-4-(dimethylamino)-1,5-naphthyridine-3-carboxylic acid. LCMS (method B) Rₜ= 0.82 min, m/z= 362.0 [M+H]⁺. To a solution of 8-(3,5-dichlorophenyl)-4-(dimethylamino)-1,5-naphthyridine-3-carboxylic acid (282 mg, 0.71 mmol) in DMF (7 mL) at rt under N₂-atmosphere was added NEt₃ (3 equiv., 2.12 mmol) followed by diphenylphosphoryl azide (230 µL, 1.06 mmol). The mixture was stirred for 1 hour at rt. Anhydrous MeOH (2.90 mL) added and the reaction was left to stir at rt overnight under N₂-atmopshere. The reaction was quenched by the addition of sat. aq. NaHCO₃ (25 mL). The organic layer was separated and extracted with CH₂Cl₂ (3x50 mL). The combined organic layers were dried over anhydrous Na₂SO₄, filtered and concentrated *in vacuo* to give methyl N-[8-(3,5-dichlorophenyl)-4-(dimethylamino)-1,5-naphthyridin-3-yl] carbamate. LCMS (method B) Rₜ= 1.43 min, m/z=391 [M+H]+.

A solution of methyl N-[8-(3,5-dichlorophenyl)-4-(dimethylamino)-1,5-naphthyridin-3-yl] carbamate (0.71 mmol) in 1,4-dioxane (12 mL) at rt under N₂-atmosphere was treated with sodium hydroxide in water (10.6 mL, 21.2 mmol, 2 M). The mixture was heated to 100°C for 90 min. The reaction was diluted with water (25 mL) and extracted with CH₂Cl₂ (3x50 mL). The combined organic layers were dried over anhydrous Na₂SO₄, filtered and concentrated *in vacuo.* The residue was purified by column chromatography (5-10% EtOAc in cyclohexane) to give 8-(3,5-dichlorophenyl)-N4,N4-dimethyl-1,5-naphthyridine-3,4-diamine. LCMS (method B) Rt=1.41 min, m/z=333 [M+H]⁺.

A solution of 8-(3,5-dichlorophenyl)-N4,N4-dimethyl-1,5-naphthyridine-3,4-diamine (93.7 mg, 0.28 mmol) and (S)-chromane-4-carboxylic acid (65.7 mg, 0.35 mmol) in EtOAc (4 mL) under N₂-atmosphere was cooled to 0°C with an ice bath. Pyridine (0.07 mL, 0.9 mmol) was added to the reaction at 0°C followed by T₃P (50 wt% in EtOAc; 0.34 mL, 0.57 mmol). The reaction was allowed to slowly warm to rt and was stirred overnight. The reaction mixture was diluted with water (20 mL) and extracted with CH₂Cl₂ (3x15 mL). The combined organic layers were dried over anhydrous Na₂SO₄, filtered and concentrated *in vacuo.* The residue was purified by column chromatography (5-15% EtOAc in cyclohexane) to give the title compound. LCMS (method B) Rt=1.61 min, m/z= 493 [M+H]⁺. ¹H-NMR (400 MHz, DMSO-d₆) δ [ppm]: 9.97 (s, 1 H), 8.92 (d, J= 4 Hz, 1 H), 8.88 (s, 1 H), 7.74 (m, 4 H), 7.28 (d, J= 7.6 Hz. 1 H), 7.17 (td, J= 1.6, 8.4 Hz), 1 H),6.91 (td, J= 1.2, 7.2 Hz, 1 H), 6.82 (dd, J= 1.2, 8 Hz, 1 H), 4.35 (m, 1 H), 4.20 (m, 1 H), 4.10 (t, J= 5.6 Hz, 1 H), 3.12 (s, 6 H), 2.26 (m, 2 H).

The following compounds were prepared analogously by the methodology of Example 6.1:

| Ex. | Name | Structure |
|---|---|---|
| 6.2 | (4S)-N-[8-(3,5-dichlorophenyl)-4-(dimethylamino)-1,5-naphthyridin-3-yl]chromane-4-carboxamide | 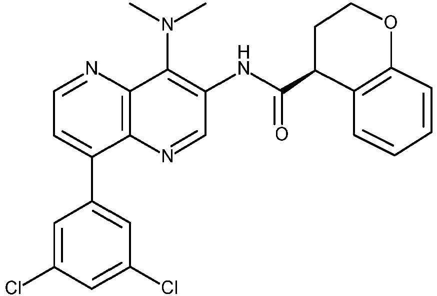 |
| 6.3 | (4S)-N-[8-(3,5-dichlorophenyl)-4-morpholino-1,5-naphthyridin-3-yl]chromane-4-carboxamide | 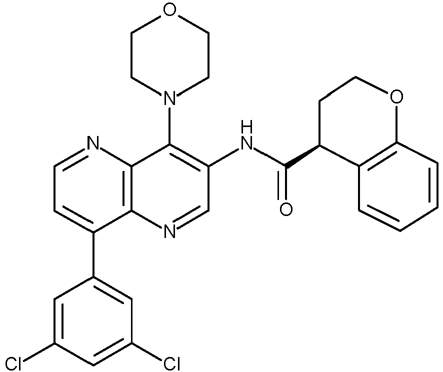 |

### Example 7.1

### 1-[(4S)-Chroman-4-yl]-3-[8-(3,5-dichlorophenyl)-4-(dimethylamino)-3-quinolyl]urea

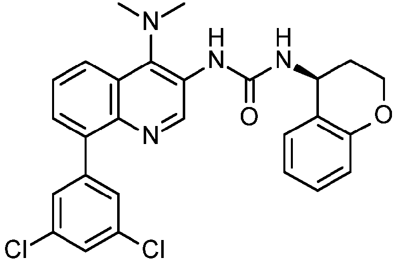

To a stirred solution of 8-bromoquinolin-4-ol (2.0 g, 8.82 mmol) in propionic acid (20 mL, 265 mmol) at 100°C was added nitric acid (1 mL, 16 mmol) slowly over 5 min.

The reaction was heated to 125°C and left to stir for 45 min. The reaction was then allowed to cool to rt, causing the product to precipitate. The solid was collected by filtration and washed with water (3x10 mL), iPrOH (10 mL), isohexane (10 mL), and then dried in the vacuum oven for 1 hour to give 8-bromo-3-nitro-quinolin-4-ol. LCMS (method B): Rₜ= 0.54 min, m/z= 269 [M+H]⁺.

To a solution of 8-bromo-3-nitro-quinolin-4-ol (1.52 g, 5.37 mmol) was added POCl₃ (10 mL, 107 mmol). The suspension was heated to reflux for 2 hours. The reaction mixture was allowed to cool to rt, and left to stand overnight. The reaction mixture was concentrated *in vacuo* (azeotroping with toluene) to give 8-bromo-4-chloro-3-nitro-quinoline which was used directly in the next step without further purification.

To a solution of 8-bromo-4-chloro-3-nitro-quinoline (2.32 g, 5.38 mmol,) in THF (30 mL) was slowly added dimethylamine (2 M in THF, 7 mL, 14 mmol). The reaction was left to stir at rt for 1.5 hour. The reaction mixture was partitioned between EtOAc and sat. aq. NaHCO₃ (50 mL of each). Brine (50 mL) was added. The layers were separated, and the aq. layer was extracted with EtOAc (2x50 mL). The combined organic layers were concentrated *in vacuo* to give 8-bromo-N,N-dimethyl-3-nitro-quinolin-4-amine. LCMS (method B): Rₜ= 1.13 min, m/z= 296 [M+H]⁺.

To a solution of 8-bromo-N,N-dimethyl-3-nitro-quinolin-4-amine (505 mg, 1.62 mmol), was added (3,5-dichlorophenyl) boronic acid (314 mg, 1.61 mmol), tetrakis (triphenyl phosphine) palladium(0) (92 mg, 0.08 mmol,), and Na₂CO₃ (351 mg, 3.28 mmol). The vial was sealed, then evacuated and back-filled with N₂-atmosphere three times. 1,4-Dioxane (9 mL) was added, followed by water (3 mL) and the reaction was heated to 100°C in the microwave for 1 hour. The reaction mixture was partitioned between EtOAc and sat. aq. NaHCO₃ (50 mL of each). The layers were separated, and the aq. layer was extracted with EtOAc (2x25 mL). The combined organic layers were concentrated *in vacuo,* then purified by column chromatography to give 8-(3,5-dichlorophenyl)-N,N-dimethyl-3-nitro-quinolin-4-amine. LCMS (method B): Rₜ= 1.57 min, m/z= 362 [M+H]⁺. To a stirred suspension of 8-(3,5-dichlorophenyl)-N,N-dimethyl-3-nitro-quinolin-4-amine (401 mg, 1.05 mmol) in THF (5 mL), EtOH (5 mL) and water (2.5 mL) was added iron (184 mg, 3.23 mmol) and NH₄Cl (168 mg, 3.13 mmol). The reaction was heated to 75°C and left to stir for 45 min. The reaction was allowed to cool to rt, then partitioned between sat. aq. NaHCO₃ and EtOAc (25 mL of each). The mixture was filtered through Celite^{®} (washing with EtOAc), and the layers of the filtrate were separated. The aq. layer was extracted with EtOAc (2x25 mL), and the combined organic layers were concentrated *in vacuo* before being purified by column chromatography to give 8-(3,5-dichlorophenyl)-N4,N4-dimethyl-quinoline-3,4-diamine. LCMS (method B): Rₜ= 1.48 min, m/z= 363.2 [M+H]⁺.

To a solution of 4-nitrophenyl chloroformate (94 mg, 0.45 mmol) in THF (2 mL) at 0°C under N₂-atmosphere was added 8-(3,5-dichlorophenyl)-N4,N4-dimethyl-quinoline-3,4-diamine (151 mg, 0.43 mmol) in THF (2.5 mL) dropwise over 2 min. The reaction was left to stir at 0°C for 1 hour. To the reaction mixture was added (4S)-chroman-4-amine·HCl (90 mg, 0.47 mmol) and NEt₃ (179 µL, 1.27 mmol). The reaction was allowed to warm to rt, and stirred for 30 min. The reaction mixture was partitioned between sat. aq. NaHCO₃ and CH₂Cl₂ (20 mL of each). The layers were separated, and the aq. layer was extracted with CH₂Cl₂ (2x20 mL). The combined organic layers were filtered and concentrated *in vacuo.* The residue was purified by column chromatography. LCMS (method B) Rₜ= 1.49 min, m/z= 507 [M + H]⁺. ¹H NMR (400 MHz, CDCl₃) δ [ppm]: 9.51 (s, 1 H), 7.98 (m, 1 H), 7.56 (m, 4 H), 7.38 (t, J= 2 Hz, 1 H), 7.31 (m, 2 H), 7.18 (t, J= 8.4 Hz, 1H), 6.91 (t, J= 7.6 Hz, 1 H), 6.83 (d, J= 8 Hz, 1 H), 5.12 (q, J= 6.8 Hz, 1 H), 4.94 (d, J= 7.2 Hz, 1 H), 4.2 (m, 2 H), 3.06 (s, 6 H), 2.23 (m, 2 H).

### Examples 8.1 and 8.2

### (4S and 4R)-N-[5-(3,5-Dichlorophenyl)-1-(dimethylamino)-2-naphthyl]chromane-4-carboxamide

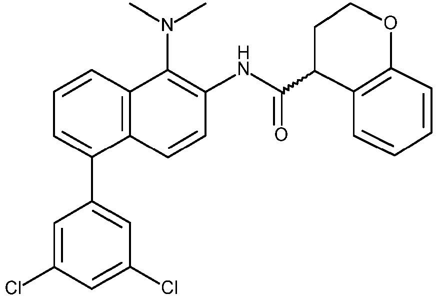

To a stirred solution of 1-bromo-5-nitronaphthalene (6.0 g, 23.90 mmol) and 3,5-dichlorophenyl boronic acid (4 g, 21.05 mmol) in 1,4-dioxane (80 mL)-water (26 mL) was added Na₂CO₃ (5.06 g, 47.73 mmol) and the reaction mixture was degassed using N₂ for 10 min. After adding Pd(dppf)Cl₂ (0.87 g, 1.19 mmol), the resulting reaction mixture was stirred for 45 min at 80°C. The reaction mixture was quenched by adding water (50 mL) and extracted with EtOAc (2x50 mL). The combined organic layers were washed with brine (50 mL), dried over anhydrous Na₂SO₄ and concentrated *in vacuo.* The crude compound was purified by column chromatography eluting with 50% EtOAc in ether to obtain 1-(3,5-dichlorophenyl)-5-nitronaphthalene.

To a stirred solution of 1-(3,5-dichlorophenyl)-5-nitronaphthalene (5 g, 15.77 mmol) and NH₄Cl (2.6 g, 48.88 mmol) in THF (70 mL), EtOH (70 mL) and water (35 mL) was added iron powder (2.6 g, 47.31 mmol) and the resulting reaction mixture was stirred for 16 hours at 75°C. The reaction mixture was filtered through Celite^{®} and washed with EtOH (30 mL). The filtrate was concentrated, then quenched with sat. aq. NaHCO₃ solution (30 mL) and extracted with EtOAc (3x40 mL). The combined organic layers were washed with brine (50 mL), dried over anhydrous Na₂SO₄ and concentrated *in vacuo.* The crude compound was triturated with Et₂O and pentane (1:1) to obtain 5-(3,5-dichlorophenyl)naphthalen-1 -amine.

To a stirred solution of 5-(3,5-dichlorophenyl)naphthalen-1-amine (3.3 g, 11.49 mmol) in DMF (10 mL) was added NBS (2.04 g, 11.49 mmol) at -5°C and the reaction mixture was stirred for 30 min. The reaction mixture was quenched by adding sat. aq. NaHCO₃ solution (20 mL) and extracted with CH₂Cl₂ (2x30 mL). The combined organic layers were washed with brine, dried over anhydrous Na₂SO₄ and concentrated under reduced pressure. The crude compound was purified by flash column eluting with 30% EtOAc in ether to obtain 2-bromo-5-(3,5-dichlorophenyl)naphthalen-1-amine. LCMS (method D) Rₜ= 2.69 min, m/z= 368.05 [M+H]⁺.

A stirred solution of 2-bromo-5-(3,5-dichlorophenyl)naphthalen-1-amine (1.6 g, 4.38 mmol) in formaldehyde solution (37 % in water, 15 mL) and formic acid (15 mL) was stirred for 1 hour at 100°C. The reaction was quenched by adding sat. aq. NaHCO₃ solution (2x20 mL) at 0°C and extracted with CH₂Cl₂ (3x20 mL). The combined organic layers were washed with brine, dried over anhydrous Na₂SO₄ and concentrated under reduced pressure. The crude compound was purified by column chromatography eluting with 30% EtOAc in Et₂O to obtain 2-bromo-5-(3,5-dichlorophenyl)-N,N-dimethylnaphthalen-1-amine. LCMS (method D) Rₜ= 3.76 min, m/z= 394.09 [M+H]⁺. To a stirred solution of 2-bromo-5-(3,5-dichlorophenyl)-N,N-dimethylnaphthalen-1-amine (1.2 g, 3.06 mmol) in toluene (25 mL) was added tert-butylcarbamate (537 mg, 4.59 mmol), cesium carbonate (5.23 g, 9.18 mmol) and degassed using N₂ for 10 min. Pd₂(dba)₃ (0.248 g, 0.15 mmol) and Xantphos (0.162 g, 0.15 mmol) were added sequentially and the resulting reaction mixture was stirred for 16 hours at 110°C. The reaction mixture was quenched by adding water (10 mL) and extracted with EtOAc (3x20 mL). The combined organic layers were washed with brine, dried over anhydrous Na₂SO₄ and concentrated under reduced pressure. The crude compound was purified by column chromatography eluting with 50% EtOAc in ether to obtain tert-butyl (5-(3,5-dichlorophenyl)-1-(dimethylamino)naphthalen-2-yl)carbamate. LCMS (method D) Rₜ= 3.06 min, m/z= 431 [M+H]⁺.

To a stirred solution of tert-butyl (5-(3,5-dichlorophenyl)-1-(dimethylamino)naphthalen-2-yl)-carbamate (0.56 g, 1.30 mmol) in EtOH (5 mL), 5M HCl in EtOH (5 mL),was added at rt and stirred for 16 hours at 50°C. The reaction mixture was cooled to rt and then concentrated under reduced pressure. The crude compound was purified by triturating with diethyl ether and pentane (1:1). LCMS (method D) Rₜ= 2.7 min; m/z= 331.21 [M+H]⁺.

To a stirred solution of 5-(3,5-dichlorophenyl)-N1,N1-dimethylnaphthalene-1,2-diamine·HCl (0.44 g, 1.34 mmol), (S)-chromane-4-carboxylic acid (0.2 g, 1.12 mmol) in CH₂Cl₂ (2 mL) was added and DIPEA (0.6 mL, 3.37 mmol) and T₃P (50 wt% in EtOAc, 0.5 mL, 1.68 mmol) at 0°C. The resulting reaction mixture was stirred for 24 hours at 50°C. The reaction mixture was quenched by adding water (5 mL) and extracted with CH₂Cl₂ (3x10 mL). The combined organic layers were washed with brine, dried over anhydrous Na₂SO₄ and concentrated under reduced pressure. The crude compound was purified by SFC: Chiralpak IC (30x250mm), 5µ % CO₂: 65% Co solvent: 35% (MeOH) Total Flow:100 g/min Back Pressure : 100 bar Temperature : 35°C UV : 260nm) to give the title compounds. LCMS (method D) Rₜ= 3.83; m/z= 491.0 [M+H]⁺. ¹H NMR (400 MHz, DMSO) δ [ppm]: 9.63 (s, 1 H), 8.26 (d, J= 9.2 Hz, 1 H), 8.02 (d, J= 8.4 Hz, 1 H), 7.72 (t, J= 2 Hz, 1 H), 7.49-7.59 (m, 4 H), 7.33-7.38 (m, 2 H), 7.26 (td, J= 1.2, 8 Hz, 1 H), 6.99 (t, J= 7.6 Hz, 1 H), 6.88 (d, J= 8.4 Hz, 1 H), 4.05-4.25 (m, 3 H), 2.79 (s, 6 H), 2.33-2.37 (m, 1 H), 2.07-2.18 (m, 1 H).

### Experimental details for compounds in the tables:

| Ex. | HPLC | NMR |
|---|---|---|
| 1.3 | Rₜ= 1.69; m/z (ES+)= 490.0 [M+H]⁺ / Method B | ¹H NMR (400 MHz, CDCl₃) δ [ppm]: 9.98 (s, 1 H), 8.44 (s, 1 H),7.83 (dd, J= 1.6, 7.6 Hz, 1 H), 7.51 (m, 5H), 7.37 (t, J= 1.6 Hz, 1 H), 7.27 (m, 2 H), 3.93 (m, 1 H), 2.89 (m, 2 H), 2.65 (s, 6H), 2.56 (m, 1 H), 1.91 (m , 3 H) |
| 1.4 | Rₜ= 1.69; m/z (ES+)= 490.0 [M+H]⁺ / Method B | ¹H NMR (400 MHz, CDCl₃) δ [ppm]: 9.98 (s, 1 H), 8.44 (s, 1 H),7.83 (dd, J= 1.6, 7.6Hz, 1 H), 7.51 (m, 5H), 7.37 (t, J= 1.6 Hz, 1 H), 7.27 (m, 2 H), 3.93 (m, 1 H), 2.89 (m, 2 H), 2.65 (s, 6H), 2.56 (m, 1 H), 1.91 (m , 3 H) |
| 1.5 | Rₜ= 1.59; m/z (ES+)= 489.0 [M+H]⁺ / Method B | ¹H NMR (400 MHz, CDCl₃) δ [ppm]: 9.78 (s, 1H), 8.36 (dd, J= 2.8, 7.6Hz, 1 H), 8.22 (s, 1 H), 7.56 (m, 2 H), 7.53 (d, J= 2 Hz, 2 H), 7.37 (d, J= Hz, 1 H), 7.31 (m, 2 H), 7.01 (m, 2 H), 4.37 (m, 1 H), 4.12 (m, 1 H), 3.93 (m, 1 H), 2.67 (m, 1 H), 2.27 (m, 1 H), 1.63 (m, 1 H), 0.91 (m, 1 H), 0.72 (m, 1 H), 0.46 (m, 1 H), 0.39 (m, 1 H) |
| 1.6 | Rₜ= 1.59; m/z (ES+)= 489.0 [M+H]⁺ / Method B | ¹H NMR (400 MHz, CDCl₃) δ [ppm]: 9.78 (s, 1H), 8.36 (dd, J= 2.8, 7.6Hz, 1 H), 8.22 (s, 1 H), 7.56 (m, 2 H), 7.53 (d, J= 2 Hz, 2 H), 7.37 (d, J= Hz, 1 H), 7.31 (m, 2 H), 7.01 (m, 2 H), 4.37 (m, 1 H), 4.12 (m, 1 H), 3.93 (m, 1 H), 2.67 (m, 1 H), 2.27 (m, 1 H), 1.63 (m, 1 H), 0.91 (m, 1 H), 0.72 (m, 1 H), 0.46 (m, 1 H), 0.39 (m, 1 H) |
| 1.7 | Rₜ= 1.67; m/z (ES+)= 508.2 [M+H]⁺ / Method B | ¹H NMR (400 MHz, CDCl₃) δ [ppm]: 9.88 (s, 1H), 9.25 (s, 1 H), 8.25 (dd, J= 1.6, 8.4 Hz, 1 H), 7.51-7.59 (m, 4 H), 7.38 (t, J= 2 Hz, 1 H), 7.23-7.31 (m, 32 H), 6.96-7.02 (m, 2 H), 4.35-4.39 (m, 1 H), 4.11 (td, J= 2, 12 Hz, 1 H), 3.88 (s, 1 H), 3.28 (s, 3 H), 2.93 (s, 3 H), 2.6-2.65 (m, 1 H), 2.22-2.31 (m, 1 H) |
| 1.8 | Rₜ= 1.67; m/z (ES+)= 508.2 [M+H]⁺ / Method B | ¹H NMR (400 MHz, CDCl₃) δ [ppm]: 9.88 (s, 1H), 9.25 (s, 1 H), 8.25 (dd, J= 1.6, Hz, 1 H), 7.51-7.59 (m, 4 H), 7.38 (t, J= 2 Hz, 1 H), 7.23-7.31 (m, 2 H), 6.96-7.02 (m, 2 H), 4.35-4.39 (m, 1 H), 4.11 (td, J= 2, 12 Hz, 1 H), 3.88 (s, 1 H), 3.28 (s, 3 H), 2.93 (s, 3 H), 2.6-2.65 (m, 1 H), 2.22-2.31 (m, 1 H) |
| 1.9 | Rₜ= 1.45; m/z (ES+)= 492.2 [M+H]⁺ / Method B | ¹H NMR (400 MHz, CDCl₃) δ [ppm]: 9.95 (s, 1H), 8.86 (s, 1 H), 7.89 (dd, J= 1.2, 8 Hz, 1 H), 7.46-7.54 (m, 5 H), 7.01-7.05 (t, J= 8 Hz, 1 H), 6.97 (d, J= 8 Hz, 1 H), 4.35 (d, J= 11 Hz, 1 H), 4.08 (q, J= 12 Hz, 1 H), 3.86 (d, J= 2.8 Hz, 1 H), 3.49 (d, J= 5.2 Hz, 1 H), 2.71 (s, 3 H), 2.70 (s, 3 H), 2.65 (d, J= 14 Hz, 2 H), 2.24 (m, 1 H) |
| 1.10 | Rₜ= 1.45; m/z (ES+)= 526.2 [M+H]⁺ / Method B | ¹H NMR (400 MHz, CDCl₃) δ [ppm]: 9.98 (s, 1H), 8.89 (s, 1 H), 7.88 (dd, J= 1.6, 8 Hz, 1 H), 7.84 (s, 1 H), 7.78 (s, 2 H), 7.63 (s, 1 H), 7.51-7.59 (m, 2 H), 7.34 (td, J= 8.4, 1.6 Hz, 1 H), 7.26 (m, 1 H), 7.04 (td, J= 7.2, 1.2 Hz, 1 H), 6.98 (d, J= 8.4 Hz, 1 H), 4.38 (m, 1 H), 4.09 (td, J= 12.4, 2 Hz, 1 H) , 3.89 (d, J= 3.2 Hz, 1 H), 2.7 (s, 6 H), 2.62 (m, 1 H) |
| 1.11 | Rₜ= 1.45; m/z (ES+)= 526.2 [M+H]⁺ / Method B | ¹H NMR (400 MHz, CDCl₃) δ [ppm]: 9.98 (s, 1H), 8.89 (s, 1 H), 7.88 (dd, J= 1.6, 8 Hz, 1 H), 7.84 (s, 1 H), 7.78 (s, 2 H), 7.63 (s, 1 H), 7.51-7.59 (m, 2 H), 7.34 (td, J= 8.4, 1.6 Hz, 1 H), 7.26 (m, 1 H), 7.04 (td, J= 7.2, 1.2 Hz, 1 H), 6.98 (d, J= 8.4 Hz, 1 H), 4.38 (m, 1 H), 4.09 (td, J= 12.4, 2 Hz, 1 H) , 3.89 (d, J= 3.2 Hz, 1 H), 2.7 (s, 6 H), 2.62 (m, 1 H) |
| 1.12 | Rₜ= 2.49; m/z= 534-35[M+H]⁺ / Method C | ¹H NMR (400 MHz, DMSO-d₆) δ [ppm]: 10.7 (s, 1 H), 8.64 (s, 1 H), 8.23 (dd, J= 1.6, 8.8 Hz, 1 H), 7.78 (dd, J= 1.2, 7.2 Hz, 1 H), 7.68 (dd, J= 1.2, 7.2 Hz, 1 H), 7.63-7.66 (m, 3 H), 7.30 (d, J= 7.2 Hz, 1 H). 7.17 (td, J= 1.6, 8.4 Hz, 1 H), 6.92 (td, J= 1.2, 7.6 Hz, 1 H), 6.82 (dd, J= 1.2, 8.4 Hz, 1 H), 4.36-4.42 (m, 1 H), 4.19-4.23 (m, 1 H), 4.06 (t, J= 5.6 Hz, 1 H), 3.82 (m, 4 H), 3.27 -3.29 (m, 4 H), 2.21-2.28 (m, 2 H) |
| 1.13 | Rₜ= 2.49; m/z= 534-35[M+H]⁺ / Method C | ¹H NMR (400 MHz, DMSO-d₆) δ [ppm]: 10.7 (s, 1 H), 8.64 (s, 1 H), 8.23 (dd, J= 1.6, 8.8 Hz, 1 H), 7.78 (dd, J= 1.2, 7.2 Hz, 1 H), 7.68 (dd, J= 1.2, 7.2 Hz, 1 H), 7.63-7.66 (m, 3 H), 7.30 (d, J= 7.2 Hz, 1 H). 7.17 (td, J= 1.6, 8.4 Hz, 1 H), 6.92 (td, J= 1.2, 7.6 Hz, 1 H), 6.82 (dd, J= 1.2, 8.4 Hz, 1 H), 4.36-4.42 (m, 1 H), 4.19-4.23 (m, 1 H), 4.06 (t, J= 5.6 Hz, 1 H), 3.82 (m, 4 H), 3.27 -3.29 (m, 4 H), 2.21-2.28 (m, 2 H) |
| 1.14 | Rₜ= 3.13 ; m/z= 520.1 [M+H]+ / Method D | ¹H NMR (400 MHz, DMSO-d₆) δ [ppm]: 8.58 (s, 1 H), 8.27 (dd, J= 1.6, 8.4 Hz, 1 H), 7.68-7.76 (m, 2 H), 7.56-7.61 (m, 1 H), 7.29 (d, J= 7.2 Hz, 1 H), 7.18-7.24 (m, 2 H), 6.91 (td, J= 1.2, 7.6 Hz, 1 H), 6.82 (dd, J= 1.2, 8.4 Hz, 1 H), 4.35-4.45 (m, 1 H), 4.19-4.22 (m, 1 H), 4.05 (t, J= 5.6 Hz, 1 H), 3.82 (t, J= 4.4 Hz, 4 H), 3.20-3.32 (m, 4H), 2.24 (m, 2 H) |
| 1.15 | Rₜ= 3.13 ; m/z= 520.1 [M+H]+ / Method D | ¹H NMR (400 MHz, DMSO-d₆) δ [ppm]: 8.58 (s, 1 H), 8.27 (dd, J= 1.6, 8.4 Hz, 1 H), 7.68-7.76 (m, 2 H), 7.56-7.61 (m, 1 H), 7.29 (d, J= 7.2 Hz, 1 H), 7.18-7.24 (m, 2 H), 6.91 (td, J= 1.2, 7.6 Hz, 1 H), 6.82 (dd, J= 1.2, 8.4 Hz, 1 H), 4.35-4.45 (m, 1 H), 4.19-4.22 (m, 1 H), 4.05 (t, J= 5.6 Hz, 1 H), 3.82 (t, J= 4.4 Hz, 4 H), 3.20-3.32 (m, 4H), 2.24 (m, 2 H) |
| 3.2 | Rₜ=1.39 min, m/z= 526.0 [M-H]⁻ / Method B | ¹H NMR (400 MHz, CDCl₃) δ [ppm]: 9.14 (s, 1 H), 7.98-8.01 (m, 2 H), 7.57-7.62 (m, 2 H), 7.52 (d, J= 1.6 Hz, 2 H), 7.40 (t, J= 1.6 Hz, 1 H), 7.25-7.27 (m, 2 H), 6.68-6.88 (m, 2 H), 4.62 (td, J= 2.4, 12 Hz, 1 H), 4.53 (d, J= 4.4 Hz, 1 H), 4.27-4.34 (m, 1 H), 3.05 (s, 6 H), 2.67-2.75 (m, 1 H), 2.35-2.45 (m 1 H), |
| 4.2 | Rₜ= 1.46; m/z (ES+)= 491.0 [M+H]⁺ / Method B | ¹H NMR (400 MHz, DMSO-d₆) δ [ppm]: 10.15 (s, 1 H), 8.8 (s, 1 H), 8.63 (d, J= 5.6 Hz, 1 H), 8.09 (d, J= 2 Hz, 2 H), 8.00 (d, J= 2.8 Hz, 1 H), 7.72 (t, J= 2 Hz, 1 H), 7.27 (d, J= 7.6 Hz, 1 H), 7.16 (m, 1 H), 6.91 (m, 1 H), 6.82 (d, J= 8 Hz, 1 H), 4.38 (m, 1 H), 4.2 (m, 1 H), 4.08 (t, J= 6 Hz, 1 H), 3.04 (s, 6 H), 2.25 (m, 2 H) |
| 4.3 | Rₜ= 2.15 ; m/z (ES+)= 521.33 [M+H]+ / Method C | ¹H NMR (400 MHz, DMSO-d₆) δ [ppm]: 10.19 (s, 1 H), 8.72 (s, 1 H), 8.68 (d, J= 5.6 Hz, 1 H), 8.07 (d, J= 6 Hz, 1 H), 7.65-7.72 (m, 1 H), 7.28-7.33 (m, 2 H), 7.17 (t, J= 7.2 Hz, 1 H), 6.91 (t, J= 7.6 Hz, 1 H), 6.82 (d, J= 8 Hz, 1 H), 4.35 (m, 1 H), 4.18-4.22 (m, 1 H), 4.06 (t, J= 5.6 Hz, 1 H), 3.82 (s, 4 H), 3.31 (s, 4 H), 2.25 (m, 2 H) |
| 4.4 | Rₜ= 2.15 ; m/z (ES+)= 521.33 [M+H]+ / Method C | ¹H NMR (400 MHz, DMSO-d₆) δ [ppm]: 10.19 (s, 1 H), 8.72 (s, 1 H), 8.68 (d, J= 5.6 Hz, 1 H), 8.07 (d, J= 6 Hz, 1 H), 7.65-7.72 (m, 1 H), 7.28-7.33 (m, 2 H), 7.17 (t, J= 7.2 Hz, 1 H), 6.91 (t, J= 7.6 Hz, 1 H), 6.82 (d, J= 8 Hz, 1 H), 4.35 (m, 1 H), 4.18-4.22 (m, 1 H), 4.06 (t, J= 5.6 Hz, 1 H), 3.82 (s, 4 H), 3.31 (s, 4 H), 2.25 (m, 2 H) |
| 5.2 | Rₜ= 1.61; m/z (ES+)= 494.0 [M+H]⁺ / Method B | ¹H NMR (400 MHz, DMSO-d₆) δ [ppm]: 10.15 (s, 1 H), 9.32 (s, 1 H), 8.93 (s, 1 H), 8.29 (d, J= 1.6Hz, 2 H), 7.84 (t, J= 2 Hz, 1 H), 7.27 (d, J= 7.2 Hz, 1 H), 7.17 (t, J= 7.2 Hz, 1 H), 6.91 (t, J= 7.2 Hz, 1 H), 9.82 (d, J= 8 Hz, 1 H), 4.33-4.41 (m, 1 H), 4.16-4.24 (m, 1H), 4.11 (t, J= 7.2 Hz, 1 H), 3.17 (s, 6 H), 2.17-2.27 (m, 2 H) |
| 6.2 | Rₜ= 1.61; m/z (ES+)= 493.0 [M+H]⁺ / Method B | ¹H NMR (400 MHz, DMSO-d₆) δ [ppm]: 9.97 (s, 1 H), 8.92 (d, J= 4 Hz, 1 H), 8.88 (s, 1 H), 7.74 (m, 4 H), 7.28 (d, J= 7.6 Hz. 1 H), 7.17 (td, J= 1.6, 8.4 Hz), 6.91 (td, J= 1.2, 7.2 Hz, 1 H), 6.82 (dd, J= 1.2, 8 Hz, 1 H), 4.35 (m, 1 H), 4.20 (m, 1 H), 4.10 (t, J= 5.6 Hz, 1 H), 3.12 (s, 6 H), 2.26 (m, 2 H) |
| 6.3 | Rₜ= 1.57; m/z (ES+)= 535.2 [M+H]⁺ / Method B | ¹H NMR (400 MHz, DMSO-d₆) δ [ppm]: 9.81 (s, 1 H), 9.20 (s, 1 H), 9.87 (d, J= 4.4 Hz, 1 H), 7.73-7.77 (m, 4 H), 7.32 (d, J= 7.6 Hz, 1 H), 7.22 (td, J= 1.6, 8.4 Hz, 1 H), 6.95 (td, J= 0.8, 7.2 Hz, 1 H), 6.87 (dd, J= 0.8, 8 Hz, 1 H), 4.22-4.31 (m, 2 H), 4.09 (t, J= 5.2 Hz, 1 H), 3.63-3.72 (m, 4 H), 3.38-3.48 (m, 4 H), 2.15-2.33 (m, 2 H) |

The compounds of formula (I) of the present invention are useful for the treatment and/or control, in particular helminths, in which the endoparasitic nematodes and trematodes may be the cause of serious diseases of mammals and poultry. Typical nematodes of this indication are: *Filariidae, Setariidae, Haemonchus, Trichostrongylus, Ostertagia, Nematodirus, Cooperia, Ascaris, Bunostonum, Oesophagostonum, Charbertia, Trichuris, Strongylus, Trichonema, Dictyocaulus, Capillaria, Heterakis, Toxocara, Ascaridia, Oxyuris,Ancylostoma, Uncinaria, Toxascaris* and *Parascaris.* The trematodes include, in particular, the family of *Fasciolideae,* especially *Fasciola hepatica.*

Certain parasites of the species *Nematodirus, Cooperia* and *Oesophagostonum* infest the intestinal tract of the host animal, while others of the species *Haemonchus* and *Ostertagia* are parasitic in the stomach and those of the species *Dictyocaulus* are parasitic in the lung tissue. Parasites of the families and may be found in the internal cell tissue and in the organs, *e.g.* the heart, the blood vessels, the lymph vessels and the subcutaneous tissue. A particularly notable parasite is the heartworm of the dog, *Dirofilaria iminitis.*

The parasites which may be treated and/or controlled by the compounds of formula (I) also include those from the class of *Cestoda* (tapeworms), *e.g.* the families *Mesocestoidae,* especially of the genus *Mesocestoides,* in particular *M. lineatus; Dipylidiidae,* especially *Dipylidium caninum, Joyeuxiella* spp., in particular *Joyeuxiella pasquali,* and *Diplopylidium* spp., and *Taeniidae,* especially *Taenia pisformis, Taenia cervi, Taenia ovis, Taeneia hydatigena, Taenia multiceps, Taenia taeniaeformis, Taenia serialis,* and *Echinococcus* spp., most particularly *Taneia hydatigena, Taenia ovis, Taenia multiceps, Taenia serialis; Echinococcus granulosus* and *Echinococcus multilocularis.*

Furthermore, the compounds of formula (I) are suitable for the treatment and/or control of human pathogenic parasites. Of these, typical representatives that appear in the digestive tract are those of the genus *Ancylostoma, Necator, Ascaris, Strongyloides, Trichinella, Capillaria, Trichuris* and *Enterobius.* The compounds of the present invention are also against parasites of the genus *Wuchereria, Brugia, Onchocerca* and *Loa* from the family of *Dracunculus* and parasites of the genus *Strongyloides* and *Trichinella,* which infect the gastrointestinal tract in particular.

A particular parasite to be treated and/or and controlled by the compounds of the invention is the heartworm (*Dirofilaria immitis*). Particular subjects for such treatment are dogs and cats.

The compounds of the invention can be administered alone or in the form of a composition. In practice, the compounds of the invention are usually administered in the form of compositions, that is, in admixture with at least one acceptable excipient. The proportion and nature of any acceptable excipient(s) are determined by the properties of the selected compound of the invention, the chosen route of administration, and standard practice as in the veterinary and pharmaceutical fields.

In one embodiment, the present invention provides compositions comprising: a compound of invention and at least one acceptable excipient.

In effecting such treatment and/or control, a compound of the invention can be administered in any form and route which makes the compound bioavailable. The compounds of the invention can be administered by a variety of routes, including orally, in particularly by tablets and capsules. The compounds of the invention can be administered parenteral routes, more particularly by inhalation, subcutaneously, intramuscularly, intravenously, intraarterially, transdermally, intranasally, rectally, vaginally, occularly, topically, sublingually, and buccally, intraperitoneally, intraadiposally, intrathecally and via local delivery for example by catheter or stent. One skilled in the art can readily select the proper form and route of administration depending upon the particular characteristics of the compound selected, the disorder or condition to be treated, the stage of the disorder or condition, and other relevant circumstances. The pharmaceutical compositions of the invention may be administered to the subject, for example, in the form of tablets, capsules, cachets, papers, lozenges, wafers, elixirs, ointments, transdermal patches, aerosols, inhalants, suppositories, drenches, solutions, and suspensions.

The term "acceptable excipient" refers to refers to those typically used in preparing veterinary and pharmaceutical compositions and should be pure and non-toxic in the amounts used. They generally are a solid, semi-solid, or liquid material which in the aggregate can serve as a vehicle or medium for the active ingredient. Some examples of acceptable excipients are found in Remington's Pharmaceutical Sciences and the Handbook of Pharmaceutical Excipients and include diluents, vehicles, carriers, ointment bases, binders, disintegrates, lubricants, glidants, sweetening agents, flavoring agents, gel bases, sustained release matrices, stabilizing agents, preservatives, solvents, suspending agents, buffers, emulsifiers, dyes, propellants, coating agents, and others.

In one embodiment, the composition is adapted for oral administration, such as a tablet or a capsule or a liquid formulation, for example, a solution or suspension, adapted for oral administration. In one embodiment, the composition is adapted for oral administration, such as chewable formulation, adapted for oral administration. In still another embodiment, the composition is a liquid or semi-solid formulation, for example, a solution or suspension or a paste, adapted for parenteral administration.

Particular compositions for usage on subjects in the treatment and/or control of nematodes/ helminths comprise solutions; emulsions including classical emulsions, microemulsions and self-emulsifying compositions, that are waterless organic, preferably oily, compositions which form emulsions, together with body fluids, upon addition to the subject's body; suspensions (drenches); pour-on formulations; food additives; powders; tablets including effervescent tablets; boli; capsules including micro-capsules; and chewable treats. Particularly composition forms are tablets, capsules, food additives or chewable treats.

The compositions of the present invention are prepared in a manner well known in the veterinary and pharmaceutical art and include at least one of the compounds of the invention as the active ingredient. The amount of a compound of the present invention may be varied depending upon its particular form and may conveniently be between 1% to about 50% of the weight of the unit dose form. The present pharmaceutical compositions are preferably formulated in a unit dose form, each dose typically containing from about 0.5 mg to about 100 mg of a compounds of the invention. One or more unit dose form(s) may be taken to affect the treatment dosage. Also described herein, but not encompassed by the claims, is a method for treating parasites, comprising: administering to a subject in need thereof an effective amount of a compound of formula (I) or a salt thereof, the method optionally further comprising an effective amount of at least one additional active compound.

Also described herein, but not encompassed by the claims, is a method for controlling parasites, comprising: administering to a subject in need thereof an effective amount of a compound of formula (I) or a salt thereof, the method optionally further comprising an effective amount of at least one additional active compound.

Also described herein, but not encompassed by the claims, is a method for treating or controlling parasites, comprising: contacting a subject's environment with an effective amount of a compound of formula (I) or a salt thereof, the method optionally further comprising an effective amount of at least one additional active compound.

Thus, the invention provides compounds of the invention for use as a medicament, including for the manufacture of a medicament. In one embodiment, the invention provides the manufacture of a medicament comprising a compound of formula (I) or a salt thereof for treating parasites. In one embodiment, the invention provides the manufacture of a medicament comprising a compound of the invention or a salt thereof for controlling parasites.

The terms "treating", "to treat", "treated", or "treatment", include without limitation restraining, slowing, stopping, reducing, ameliorating, reversing the progression or severity of an existing symptom, or preventing a disorder, condition, or disease. For example, an adult heartworm infection would be treated by administering a compound of the invention. A treatment may be applied or administered therapeutically.

The terms "control", "controlling" or "controlled" refers to include without limitation decreasing, reducing, or ameliorating the risk of a symptom, disorder, condition, or disease, and protecting an animal from a symptom, disorder, condition, or disease. Controlling may refer to therapeutic, prophylactic, or preventative administration. It is well understood that a larvae or immature heartworm infection may be asymptomatic and infection by mature parasites is symptomatic and/or debilitating, Therefore, for example, a heartworm infection would be controlled by acting on the larvae or immature parasite preventing the infection from progressing to an infection by mature parasites.

Thus, the invention concerns compounds of the invention for use in the treatment and/or control of parasites, in particular helminths, in which the endoparasitic nematodes and trematodes refers to compounds of the invention for the use to act on the various forms of the parasites throughout its life cycle, independent of whether a subject is manifesting a symptom, including morbidity or mortality, and independently of the phase(s) of the parasitic challenge.

As used herein, "administering to a subject" includes but is not limited to cutaneous, subcutaneous, intramuscular, mucosal, submucosal, transdermal, oral or intranasal administration. Administration could include injection or topical administration.

The terms "subject" and "patient" refers includes humans and non-human mammalian animals, such as dogs, cats, mice, rats, guinea pigs, rabbits, ferrets, cows, horses, sheep, goats, and pigs. It is understood that a more particular subject is a human. Also, a more particular subject are mammalian pets or companion animals, such as dogs and cats and also mice, guinea pigs, ferrets, and rabbits.

The term "effective amount" refers to an amount which gives the desired benefit to the subject and includes administration for both treatment and control. The amount will vary from one individual subject to another and will depend upon a number of factors, including the overall physical condition of the subject and the severity of the underlying cause of the condition to be treated, concomitant treatments, and the amount of compound of the invention used to maintain desired response at a beneficial level.

An effective amount can be readily determined by the attending diagnostician, as one skilled in the art, by the use of known techniques and by observing results obtained under analogous circumstances. In determining the effective amount, the dose, a number of factors are considered by the attending diagnostician, including, but not limited to: the species of patient; its size, age, and general health; the specific condition, disorder, infection, or disease involved; the degree of or involvement or the severity of the condition, disorder, or disease, the response of the individual patient; the particular compound administered; the mode of administration; the bioavailability characteristics of the preparation administered; the dose regimen selected; the use of concomitant medication; and other relevant circumstances. An effective amount of the present invention, the treatment dosage, is expected to range from 0.5 mg to 100 mg. Specific amounts can be determined by the skilled person. Although these dosages are based on a subject having a mass of about 1 kg to about 20 kg, the diagnostician will be able to determine the appropriate dose for a subject whose mass falls outside of this weight range. An effective amount of the present invention, the treatment dosage, is expected to range from 0.1 mg to 10 mg/kg of the subject. The dosing regimen is expected to be daily, weekly, or monthly administration.

The compounds of the invention may be combined with one or more other active compounds or therapies for the treatment of one or more disorders, diseases or conditions, including the treatment of parasites, for which it is indicated. The compounds of the invention may be administered simultaneously, sequentially or separately in combination with one or more compounds or therapies for treating parasites and other disorders.

For example, when used to treat parasites, including heartworm, a compound of the invention may be combined with a macrocyclic lactone such as ivermectin, moxidectin, or milbemycin oxime, or with imidacloprid. Particular combinations for treating parasites include a compound of the invention and ivermectin. Another particular combination for treating parasites include a compound of the invention and milbemycin oxime.

Thus, it is understood that the compositions of the present invention optionally include comprising an effective amount of at least one additional active compound.

The activity of compounds as parasiticides may be determined by a variety of methods, including in vitro and in vivo methods.

### Example A

### Dog heart worm microfilariae

*D. immitis* microfilariae are isolated by filtration from beagle blood of an infected donor and allowed to incubate in appropriate media. Test compounds are diluted in DMSO and added to a 96-well plate containing parasites. Plates are incubated for the desired time and motility is assessed using an LCD camera imaging system. Effect of serum is tested by addition of up to 20% fetal bovine serum in the assay. Percent motility inhibition values are generated relative to the average of the DMSO-only wells.

In this test for example, the following compounds from the preparation examples showed EC₅₀ <0.1 µg/mL: 1.1, 1.3, 1.4, 1.6, 1.7, 1.9, 1.11, 1.12, 6.2, and 6.3.

### Example B

### Ruminant gastrointestinal (H. contortus (H.c.)):

*H.c.* eggs isolated from lamb fecal matter are allowed to hatch overnight. Test compounds are diluted in DMSO and added to a 96-well plate containing appropriate media. *H.c.* larvae are added to each well and plates are incubated for the desired time(s). Motility is assessed using an LCD camera imaging system. Percent motility inhibition values are generated relative to the average of the DMSO-only wells.

In this test for example, the following compounds from the preparation examples showed EC₅₀ <1 µg/mL: 1.1, 1.4, 1.6, 1.9, 1.11, 1.12, 4.2, 6.1, 6.2, 6.3, and 7.1.

### Example C

### Gastro intestinal nematodes

Jirds (*Meriones unguiculatus*), are artificially infected by gavage with third instar larvae each of *T. colubriformis* and *H. contortus.* Then treated orally with the test compound formulated in eg DMSO/PEG 2/1, on Day 6 after infection at a dose in a range between 1x3 mg/kg up to 1×32mg/kg. Three days after treatment, gerbils are euthanized and dissected to recover *H. contortus* from stomach and *T. colubriformis* from the small intestine. Efficacy is expressed as a % reduction in worm numbers in comparison with a placebo treated group, using the Abbot's formula.

The compound of example 1.1 was > 80% effective against Hc and Tc. The compound of examples 1.4 and 1.11 were > 80% effective against Hc.

### Example D

### Filarial nematodes

Av model: Gerbils, injected subcutaneously with infective *A. viteae* larvae, were subsequently treated with the test article formulated in eg DMSO/PEG 2/1, by oral gavage at a dose in a range between 1x3 mg/kg up to 5x32mg/kg (one dose per day for 5 consecutive days). At necropsy 12 weeks after infection, efficacy is expressed as a % reduction in worm numbers in comparison with the placebo treated group, using the Abbot's formula.

The compound of examples 1.1, 1.4, and 1.11 were >80% effective against Av.

### Example E

### L.s. model

Mice, injected subcutaneously with infective *L. sigmodontis* larvae, were subsequently treated with the test article formulated in eg DMSO/PEG 2/1, by oral gavage at a dose in a range between 1x3 mg/kg up to 5x32mg/kg (one dose per day for 5 consecutive days). At necropsy 5 weeks after infection, efficacy is calculated by counting developed larvae vs. untreated animals using Abbot's formula.

The compound of example 1.4 was >80% effective against Ls.

## Claims

1. A compound of formula (I): 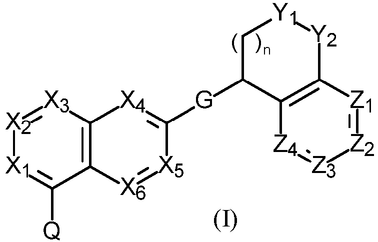 wherein
n is 1;
X₁ is selected from the group consisting of N and CR₁;
X₂ is selected from the group consisting of CR₂;
X₃ is selected from the group consisting of N and CR₃;
X₄ is selected from the group consisting of CR₄;
X₅ is selected from the group consisting of CR₅;
X₆ is selected from the group consisting of N and CR₆;
G is the group 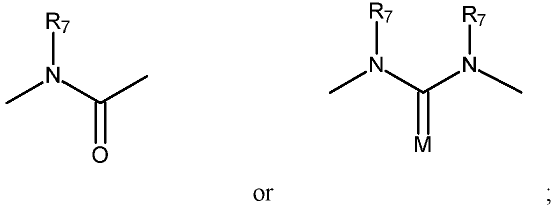
Y₁ is selected from the group consisting of CR₈R₉, and O;
Y₂ is selected from the group consisting of CR₈R₉, and O;
wherein at least one of the groups Y₁ or Y₂ is CR₈R₉;
Z₁ is selected from the group consisting of N, O, S, and CR₁₁;
Z₂ is selected from the group consisting of nil, N, and CR₁₁;
Z₃ is selected from the group consisting of nil, N and CR₁₁;
Z₄ is selected from the group consisting of N, O, S, and CR₁₁;
wherein no more than 2 of Z₁, Z₂, Z₃, and Z₄ are N and wherein only one of Z₁ and Z₄ is O or S, Z₂ is nil only when Z₁ is O or S, and Z₃ is nil only when Z₄ is O or S;
R₁ is selected from the group consisting of hydrogen, halogen, hydroxyl, -SH, -SC₁-C₄ alkyl, -S(O)(C₁-C₄ alkyl, -S(O)₂(C₁-C₄ alkyl, cyano, C₁-C₄ alkyl, C₁-C₄ halogenoalkyl, C₁-C₄-alkoxy, -B(OR₁₆)(OR₁₇) wherein R₁₆ is, each time taken, selected from the group consisting or hydrogen, C₁-C₄ alkyl, and C₃-C₆ cycloalkyl, R₁₇ is, each time taken, selected from the group consisting or hydrogen, C₁-C₄ alkyl, and C₃-C₆ cycloalkyl, or R₁₆ and R₁₇ together with the oxygen atoms to which they are attached form a 5- to 7-membered ring which is optionally substituted with 1 to 4 C₁-C₄ alkyl; -NH₂, -NH(C₁-C₄ alkyl), and -N(C₁-C₄ alkyl)₂;
R₂ is selected from the group consisting of hydrogen, halogen, hydroxyl, -SH, -SC₁-C₄ alkyl, -S(O)(C₁-C₄ alkyl, -S(O)₂(C₁-C₄ alkyl, cyano, C₁-C₄ alkyl, C₁-C₄ halogenoalkyl, C₁-C₄-alkoxy, -B(OR₁₆)(OR₁₇) wherein R₁₆ is, each time taken, selected from the group consisting or hydrogen, C₁-C₄ alkyl, and C₃-C₆ cycloalkyl, R₁₇ is, each time taken, selected from the group consisting or hydrogen, C₁-C₄ alkyl, and C₃-C₆ cycloalkyl, or R₁₆ and R₁₇ together with the oxygen atoms to which they are attached form a 5- to 7-membered ring which is optionally substituted with 1 to 4 C₁-C₄ alkyl; -NH₂, -NH(C₁-C₄ alkyl), and -N(C₁-C₄ alkyl)₂;
R₃ is selected from the group consisting of hydrogen, halogen, hydroxyl, -SH, -SC₁-C₄ alkyl, -S(O)(C₁-C₄ alkyl, -S(O)₂(C₁-C₄ alkyl, cyano, C₁-C₄ alkyl, C₁-C₄ halogenoalkyl, C₁-C₄-alkoxy, -B(OR₁₆)(OR₁₇) wherein R₁₆ is, each time taken, selected from the group consisting or hydrogen, C₁-C₄ alkyl, and C₃-C₆ cycloalkyl, R₁₇ is, each time taken, selected from the group consisting or hydrogen, C₁-C₄ alkyl, and C₃-C₆ cycloalkyl, or R₁₆ and R₁₇ together with the oxygen atoms to which they are attached form a 5- to 7-membered ring which is optionally substituted with 1 to 4 C₁-C₄ alkyl; -NH₂, -NH(C₁-C₄ alkyl), and -N(C₁-C₄ alkyl)₂;
R₄ is selected from the group consisting of C₁-C₄ alkyl, C₃-C₆ cycloalkyl, -N(C₁-C₄ alkyl)₂, -N(C₁-C₄ alkyl)(C₁-C₄ alkoxy) and 4- to 7-membered heterocycloalkyl;
R₆ is selected from the group consisting of hydrogen, halogen, hydroxyl, -SH, -SC₁-C₄ alkyl, -S(O)(C₁-C₄ alkyl, -S(O)₂(C₁-C₄ alkyl, cyano, C₁-C₄ alkyl, C₁-C₄ halogenoalkyl, C₁-C₄-alkoxy, -B(OR₁₆)(OR₁₇) wherein R₁₆ is, each time taken, selected from the group consisting or hydrogen, C₁-C₄ alkyl, and C₃-C₆ cycloalkyl, R₁₇ is, each time taken, selected from the group consisting or hydrogen, C₁-C₄ alkyl, and C₃-C₆ cycloalkyl, or R₁₆ and R₁₇ together with the oxygen atoms to which they are attached form a 5- to 7-membered ring which is optionally substituted with 1 to 4 C₁-C₄ alkyl; -NH₂, -NH(C₁-C₄ alkyl), and -N(C₁-C₄ alkyl)₂;
R₇ is selected from the group consisting of hydrogen, C₁-C₄ alkyl, and C₃-C₆ cycloalkyl optionally substituted with 1 to 5 halogen atoms, -C(H)O, C₂-C₄ alkenyl, C₂-C₄ alkynyl, C₁-C₄ halogenoalkyl, and C₁-C₄-alkoxy;
R₈ is, each time selected, independently selected from the group consisting of hydrogen, fluoro, and C₁-C₄ alkyl;
R₉ is, each time selected, independently selected from the group consisting of hydrogen, fluoro, and C₁-C₄ alkyl;
Rn is, each time selected, independently selected from the group consisting of hydrogen, halogen, hydroxyl, cyano, C₁-C₄ alkyl, C₁-C₄ halogenoalkyl, C₁-C₄-alkoxy, C₃-C₆ cycloalkyl, -NH₂, -NH(C₁-C₄ alkyl), and -N(C₁-C₄ alkyl)₂; and
Q is selected from the group consisting of 6- or 10 membered aryl optionally substituted with 1, 2 or 3 substituents independently selected from the group consisting of halogen, cyano, nitro, hydroxyl, C₁-C₄ alkyl, C₁-C₄ halogenoalkyl, C₁-C₄ alkoxy, C₃-C₆ cycloalkyl, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, - SO₂C₁-C₄ alkyl, -S(O)C₁-C₄-halogenoalkyl and -SO₂C₁-C₄ halogenoalkyl, wherein the 6- or 10 membered aryl is optionally fused with a 4- to 7-membered heterocycloalkyl having 1 or 2 heteroatoms selected from the group O, S, and N and wherein the carbons of the heterocycloalkyl are optionally substituted with 1, 2 or 3 substituents independently selected from the group halogen, cyano, nitro, hydroxyl, oxo, C₁-C₄ alkyl, C₃-C₆ cycloalkyl, C₁-C₄ halogenoalkyl, C₁-C₄ alkoxy, -NH₂, -NH(C₁-C₄ alkyl), and - N(C₁-C₄ alkyl)₂ and any N in the heterocycloalkyl is, valency permitting, substituted with a substituent selected from the group consisting of hydrogen, C₁-C₄ alkyl, and C₃-C₆ cycloalkyl;
M is selected from the group consisting of N-R₁₃, O, and S;
R₁₃ is selected from the group consisting of hydroxy, C₁-C₄ alkoxy, and -NH₂;
or a salt thereof.

2. A compound according to claim 1, wherein X₁ is CR₁; X₂ is CR₂; X₃ is CR₃; X₄ is CR₄; X₅ is CR₅; and X₆ is CR₆; or a salt thereof.

3. A compound according to claim 1 or 2, wherein X₁ is CR₁; X₂ is CR₂; X₃ is CR₃; X₄ is CR₄; X₅ is CR₅; and X₆ is N; or a salt thereof.

4. A compound according to any one of claims 1 to 3, wherein X₁ is N; X₂ is CR₂; X₃ is CR₃; X₄ is CR₄; X₅ is CR₅; and X₆ is N; or a salt thereof.

5. A compound according to any one of claims 1 to 4 wherein X₁ is CR₁; X₂ is CR₂; X₃ is CR₃; X₄ is CR₄; X₅ is CR₅; and X₆ is N; or a salt thereof.

6. A compound according to any one of claims 1 to 5, wherein
Q is a 6- or 10 membered aryl optionally substituted with 1, 2 or 3 substituents independently selected from the group consisting of halogen, cyano, nitro, hydroxy, C₁-C₄ alkyl, C₁-C₄ halogenoalkyl, C₁-C₄ alkoxy, C₃-C₆ cycloalkyl, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), - NHSO₂(C₁-C₄ alkyl), -SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl;
or a salt thereof.

7. A compound according to any one of claims 1 to 6 wherein
Q is 6-membered aryl optionally substituted with 1, 2 or 3 substituents independently selected from the group consisting of halogen, cyano, nitro, hydroxy, C₁-C₄ alkyl, C₁-C₄ halogenoalkyl, C₁-C₄ alkoxy, C₃-C₆ cycloalkyl, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl, wherein the 6-membered aryl is fused with a 4- to 7-membered heterocycloalkyl having 1 or 2 heteroatoms selected from the group O, S, and N and wherein the carbons of the heterocycloalkyl are optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, -NH₂,
-NH(C₁-C₄ alkyl), and -N(C₁-C₄ alkyl)₂ and any N in the heterocyclalkyl is substituted with a substituent selected from the group consisting of hydrogen, C₁-C₄ alkyl, and C₃-C₆ cycloalkyl;
or a salt thereof.

8. The compound according to any one of claims 1 to 7, wherein
Y₁ is CR₈R₉ and Y₂ is O; or a salt thereof;

9. A compound of claim 1, wherein the compound is selected from the group consisting of
(4R)-N-[8-(3,5-dichlorophenyl)-4-(dimethylamino)-3-quinolyl]chromane-4-carboxamide;
(4S)-N-[8-(3,5-dichlorophenyl)-4-(dimethylamino)-3-quinolyl]chromane-4-carboxamide;
(1R)-N-[8-(3,5-dichlorophenyl)-4-(dimethylamino)-3-quinolyl]tetralin-1-carboxamide
(1S)-N-[8-(3,5-dichlorophenyl)-4-(dimethylamino)-3-quinolyl]tetralin-1-carboxamide;
(4R)-N-[4-cyclopropyl-8-(3,5-dichlorophenyl)-3-quinolyl]chromane-4-carboxamide;
(4S)-N-[4-cyclopropyl-8-(3,5-dichlorophenyl)-3-quinolyl]chromane-4-carboxamide;
(4R)-N-[8-(3,5-dichlorophenyl)-4-[methoxy(methyl)amino]-3-quinolyl]chromane-4-carboxamide;
(4S)-N-[8-(3,5-dichlorophenyl)-4-[methoxy(methyl)amino]-3-quinolyl]chromane-4-carboxamide;
(4S)-N-[8-(2,3-dichlorophenyl)-4-(dimethylamino)-3-quinolyl]chromane-4-carboxamide;
(4R)-N-[8-[3-chloro-5-(trifluoromethyl)phenyl]-4-(dimethylamino)-3-quinolyl]chromane-4-carboxamide;
(4S)-N-[8-[3-chloro-5-(trifluoromethyl)phenyl]-4-(dimethylamino)-3-quinolyl]chromane-4-carboxamide;
(4S)-N-[8-(3,5-dichlorophenyl)-4-morpholino-3-quinolyl]chromane-4-carboxamide;
(4R)-N-[8-(3,5-dichlorophenyl)-4-morpholino-3-quinolyl]chromane-4-carboxamide;
(4S)-N-[4-morpholino-8-(2,3,5-trifluorophenyl)-3-quinolyl]chromane-4-carboxamide;
(4R)-N-[4-morpholino-8-(2,3,5-trifluorophenyl)-3-quinolyl]chromane-4-carboxamide;
(4S)-N-[8-(3,5-dichlorophenyl)-4-(dimethylamino)-1,7-naphthyridin-3-yl]chromane-4-carboxamide;
(4R)-N-[8-(3,5-dichlorophenyl)-4-(dimethylamino)-1,7-naphthyridin-3-yl]chromane-4-carboxamide;
(4 S)-N-[4-morpholino-8-(2,3, 5-trifluorophenyl)-1,7-naphthyridin-3-yl] chromane-4-carboxamide;
(4R)-N-[4-morpholino-8-(2,3,5-trifluorophenyl)-1,7-naphthyridin-3-yl]chromane-4-carboxamide;
(4S)-N-[4-(3,5-dichlorophenyl)-8-(dimethylamino)pyrido[3,2-d]pyrimidin-7-yl]chromane-4-carboxamide;
(4R)-N-[4-(3,5-dichlorophenyl)-8-(dimethylamino)pyrido[3,2-d]pyrimidin-7-yl]chromane-4-carboxamide;
(4R)-N-[8-(3,5-dichlorophenyl)-4-(dimethylamino)-1,5-naphthyridin-3-yl]chromane-4-carboxamide;
(4S)-N-[8-(3,S-dichlorophenyl)-4-(dimethylamino)-1,S-naphthyridin-3-yl]chromane-4-carboxamide;
(4S)-N-[8-(3,5-dichlorophenyl)-4-morpholino-1,5-naphthyridin-3-yl]chromane-4-carboxamide;
1-[(4S)-Chroman-4-yl]-3-[8-(3,5-dichlorophenyl)-4-(dimethylamino)-3-quinolyl]urea;
(4R)-N-[5-(3, 5-dichlorophenyl)-1-(dimethylamino)-2-naphthyl] chromane-4-carboxamide; and
(4S)-N-[5-(3,5-dichlorophenyl)-1-(dimethylamino)-2-naphthyl]chromane-4-carboxamide; or a salt of each of the above-mentioned compounds.

10. A composition comprising a compound of any one of claims 1 to 9, or a salt thereof, and at least one acceptable carrier.

11. A compound of any one of claims 1 to 9, or a salt thereof, for use as a medicament.

12. A compound of any one of claims 1 to 9, or a salt thereof, for use in treating and/or controlling endoparasites.

13. The compound or the salt thereof for use according to claim 12, wherein the endoparasites are heartworm.

## Patentansprüche

1. Eine Verbindung mit der Formel (I): 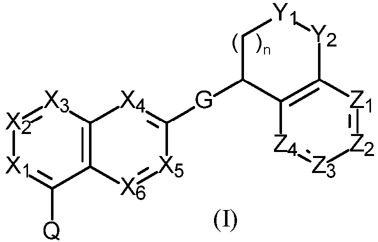 wobei:
n 1 ist;
X₁ aus der Gruppe ausgewählt ist, die aus N and CR₁ besteht;
X₂ aus der Gruppe ausgewählt ist, die aus CR₂ besteht;
X₃ aus der Gruppe ausgewählt ist, die aus N und CR₃ besteht;
X₄ aus der Gruppe ausgewählt ist, die aus CR₄ besteht;
X₅ aus der Gruppe ausgewählt ist, die aus CR₅ besteht;
X₆ aus der Gruppe ausgewählt ist, die aus N und CR₆ besteht;
G die Gruppe: 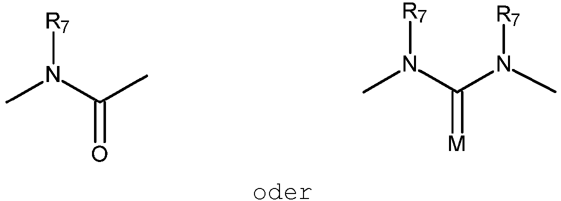 bedeutet;
Y₁ aus der Gruppe ausgewählt ist, die aus CR₈R₉ und O besteht;
Y₂ aus der Gruppe ausgewählt ist, die aus CR₈R₉ und O besteht;
wobei mindestens eine der Gruppen Y₁ oder Y₂ CR₈R₉ bedeutet;
Z₁ aus der Gruppe ausgewählt ist, die aus N, O, S und CR₁₁ besteht;
Z₂ aus der Gruppe ausgewählt ist, die aus Null, N und CR₁₁ besteht;
Z₃ aus der Gruppe ausgewählt ist, die aus Null, N und CR₁₁ besteht;
Z₄ aus der Gruppe ausgewählt ist, die aus N, O, S und CR₁₁ besteht;
wobei nicht mehr als 2 von Z₁, Z₂, Z₃ und Z₄ N bedeuten und wobei nur einer von Z₁ und Z₄ O oder S bedeutet, Z₂ nur Null bedeutet, wenn Z₁ O oder S bedeutet, und Z₃ nur Null bedeutet, wenn Z₄ O oder S bedeutet;
R₁ aus der Gruppe ausgewählt ist, die aus Wasserstoff, Halogen, Hydroxyl, -SH, -SC₁-C₄-Alkyl, -S(O)(C₁-C₄-Alkyl), -S(O)₂(C₁-C₄-Alkyl), Cyano, C₁-C₄-Alkyl, C₁-C₄ Halogenalkyl, C₁-C₄-Alkoxy und -B(OR₁₆)(OR₁₇) besteht, worin R₁₆ jedes Mal aus der Gruppe ausgewählt ist, die aus Wasserstoff, C₁-C₄-Alkyl und C₃-C₆-Cycloalkyl besteht, R₁₇ jedes Mal aus der Gruppe ausgewählt ist, die aus Wasserstoff, C₁-C₄-Alkyl und C₃-C₆-Cycloalkyl besteht, oder R₁₆ und R₁₇, zusammen mit den Sauerstoffatomen, an welche sie gebunden sind, einen 5- bis 7-gliedrigen Ring bilden, der wahlweise durch 1 bis 4 C₁-C₄-Alkyl, -NH₂, -NH(C₁-C₄ Alkyl) und - N(C₁-C₄ Alkyl)₂ substituiert ist;
R₂ aus der Gruppe ausgewählt ist, die aus Wasserstoff, Halogen, Hydroxyl, -SH, -SC₁-C₄-Alkyl, -S(O)(C₁-C₄-Alkyl), -S(O)₂(C₁-C₄ Alkyl), Cyano, C₁-C₄-Alkyl, C₁-C₄-Halogenalkyl, C₁-C₄-Alkoxy und -B(OR₁₆)(OR₁₇) besteht, worin R₁₆ jedes Mal, wenn er genommen wird, aus der Gruppe ausgewählt ist, die aus Wasserstoff, C₁-C₄-Alkyl und C₃-C₆-Cycloalkyl besteht, R₁₇ jedes Mal, wenn er genommen wird, aus der Gruppe ausgewählt ist, die aus Wasserstoff, C₁-C₄-Alkyl und C₃-C₆-Cycloalkyl besteht, oder R₁₆ und R₁₇, zusammen mit den Sauerstoffatomen, an welche sie gebunden sind, einen 5- bis 7-gliedrigen Ring bilden, der wahlweise durch 1 bis 4 C₁-C₄-Alkyl, -NH₂, -NH(C₁-C₄-Alkyl) und -N(C₁-C₄-Alkyl)₂ substituiert ist;
R₃ aus der Gruppe ausgewählt ist, die aus Wasserstoff, Halogen, Hydroxyl, -SH, -SC₁-C₄-Alkyl, -S(O)(C₁-C₄-Alkyl), -S(O)₂(C₁-C₄-Alkyl), Cyano, C₁-C₄-Alkyl, C₁-C₄-Halogenalkyl, C₁-C₄-Alkoxy und -B(OR₁₆)(OR₁₇) besteht, worin R₁₆ jedes Mal, wenn er genommen wird, aus der Gruppe ausgewählt ist, die aus Wasserstoff, C₁-C₄-Alkyl und C₃-C₆-Cycloalkyl besteht, R₁₇ jedes Mal, wenn er genommen wird, aus der Gruppe ausgewählt ist, die aus Wasserstoff, C₁-C₄-Alkyl und C₃-C₆-Cycloalkyl besteht, oder R₁₆ und R₁₇ zusammen mit den Sauerstoffatomen, an welche sie gebunden sind, einen 5- bis 7-gliedrigen Ring bilden, der wahlweise durch 1 bis 4 C₁-C₄-Alkyl, -NH₂, NH(C₁-C₄-Alkyl) und -N(C₁-C₄-Alkyl)₂ substituiert ist;
R₄ aus der Gruppe ausgewählt ist, die aus C₁-C₄-Alkyl, C₃-C₆-Cycloalkyl, -N(C₁-C₄-Alkyl)₂, -N(C₁-C₄-Alkyl)(C₁-C₄-Alkoxy) und 4- bis 7-gliedrigem Heterocycloalkyl besteht;
R₆ aus der Gruppe ausgewählt ist, die aus Wasserstoff, Halogen, Hydroxyl, -SH, -SC₁-C₄-Alkyl, -S(O)(C₁-C₄-Alkyl), -S(O)₂(C₁-C₄-Alkyl), Cyano, C₁-C₄-Alkyl, C₁-C₄-Halogenalkyl, C₁-C₄-Alkoxy und -B(OR₁₆)(OR₁₇) besteht, worin R₁₆ jedes Mal, wenn er genommen wird, aus der Gruppe ausgewählt ist, die aus Wasserstoff, C₁-C₄-Alkyl und C₃-C₆-Cycloalkyl besteht, R₁₇ jedes Mal, wenn er genommen wird, aus der Gruppe ausgewählt ist, die aus Wasserstoff, C₁-C₄-Alkyl und C₃-C₆-Cycloalkyl besteht, oder R₁₆ und R₁₇, zusammen mit den Sauerstoffatomen, an welche sie gebunden sind, einen 5- bis 7-gliedrigen Ring bilden, der wahlweise durch 1 bis 4 C₁-C₄-Alkyl, -NH₂, -NH (C₁-C₄-Alkyl) und -N(C₁-C₄-Alkyl)₂ substituiert ist;
R₇ aus der Gruppe ausgewählt ist, die aus Wasserstoff, C₁-C₄-Alkyl und C₃-C₆-Cycloalkyl wahlweise substituiert mit 1 bis 5 Halogenatomen, -C(H)O, C₂-C₄-Alkenyl, C₂-C₄-Alkinyl, C₁-C₄-Halogenalkyl und C₁-C₄-Alkoxy besteht;
R₈ jedes Mal, wenn er ausgewählt ist, unabhängig voneinander aus der Gruppe ausgewählt ist, die aus Wasserstoff, Fluor und C₁-C₄-Alkyl besteht;
R₉ jedes Mal, wenn er ausgewählt ist, unabhängig voneinander aus der Gruppe ausgewählt ist, die aus Wasserstoff, Fluor und C₁-C₄-Alkyl besteht;
R₁₁ jedes Mal, wenn er ausgewählt ist, unabhängig voneinander aus der Gruppe ausgewählt ist, die aus Wasserstoff, Halogen, Hydroxyl, Cyano, C₁-C₄-Alkyl, C₁-C₄-Halogenalkyl, C₁-C₄-Alkoxy, C₃-C₆-Cycloalkyl, -NH₂, -NH (C₁-C₄-Alkyl) und -N(C₁-C₄-Alkyl)₂ besteht; und
Q aus der Gruppe ausgewählt ist, die aus 6- oder 10-gliedrigem Aryl besteht, wahlweise substituiert mit 1, 2 oder 3 Substituenten, die unabhängig voneinander aus der Gruppe ausgewählt sind, die aus Halogen, Cyano, Nitro, Hydroxyl, C₁-C₄-Alkyl, C₁-C₄-Halogenalkyl, C₁-C₄-Alkoxy, C₃-C₆-Cycloalkyl, -NH₂, -NH (C₁-C₄-Alkyl), -N(C₁-C₄-Alkyl)₂, -NH(C₃-C₆-Cycloalkyl), -N(C₁-C₄-Alkyl)(C₃-C₆-Cycloalkyl), -NHSO₂(C₁-C₄-Alkyl), -SC₁-C₄-Alkyl, -S(O)C₁-C₄-Alkyl, -SO₂C₁-C₄-Alkyl, -S(O)C₁-C₄-Halogenalkyl und -SO₂C₁-C₄-Halogenalkyl besteht, worin das 6- oder 10-gliedrige Aryl wahlweise mit einem 4-bis 7-gliedrigen Heterocycloalkyl mit 1 oder 2 Heteroatomen, die aus der Gruppe ausgewählt sind, die aus O, S und N besteht, anelliert ist und worin die Kohlenstoffatome des Heterocycloalkyls wahlweise substituiert sind mit 1, 2 oder 3 Substituenten, die unabhängig voneinander aus der Gruppe ausgewählt sind, die aus Halogen, Cyano, Nitro, Hydroxyl, Oxo, C₁-C₄-Alkyl, C₃-C₆-Cycloalkyl, C₁-C₄-Halogenalkyl, C₁-C₄-Alkoxy, -NH₂, -NH(C₁-C₄-Alkyl) und -N(C₁-C₄-Alkyl)₂ besteht, und jedes N in dem Heterocycloalkyl, sofern es seine Wertigkeit erlaubt, durch einen Substituenten substituiert ist, der aus der Gruppe ausgewählt ist, die aus Wasserstoff, C₁-C₄-Alkyl und C₃-C₆-Cycloalkyl besteht;
M aus der Gruppe ausgewählt ist, die aus N-R₁₃, O und S besteht; und
R₁₃ aus der Gruppe ausgewählt ist, die aus Hydroxy, C₁-C₄-Alkoxy und -NH₂ besteht;
oder ein Salz davon.

2. Eine Verbindung nach Anspuch 1, wobei X₁ CR₁, X₂ CR₂, X₃ CR₃, X₄ CR₄, X₅ CR₅ und X₆ CR₆ bedeutet, oder ein Salz davon.

3. Eine Verbindung nach Anspuch 1 oder 2, wobei X₁ CR₁, X₂ CR₂, X₃ CR₃, X₄ CR₄, X₅ CR₅ und X₆ N bedeutet, oder ein Salz davon.

4. Eine Verbindung nach einem der Ansprüche 1 bis 3, wobei X₁ N, X₂ CR₂, X₃ CR₃, X₄ CR₄, X₅ CR₅ und X₆ N bedeutet, oder ein Salz davon.

5. Eine Verbindung nach einem der Ansprüche 1 bis 4, wobei X₁ CR₁, X₂ CR₂, X₃ CR₃, X₄ CR₄, X₅ CR₅ und X₆ N bedeutet, oder ein Salz davon.

6. Eine Verbindung nach einem der Ansprüche 1 bis 5, wobei:
Q ein 6- oder 10-gliedriges Aryl bedeutet, wahlweise substituiert mit 1, 2 oder 3 Substituenten, die unabhängig voneinander aus der Gruppe ausgewählt sind, die aus Halogen, Cyano, Nitro, Hydroxy, C₁-C₄-Alkyl, C₁-C₄-Halogenalkyl, C₁-C₄-Alkoxy, C₃-C₆-Cycloalkyl, -NH₂, -NH (C₁-C₄-Alkyl), -N(C₁-C₄-Alkyl)₂, -NH(C₃-C₆-Cycloalkyl), -N(C₁-C₄-Alkyl)(C₃-C₆-Cycloalkyl), -NHSO₂(C₁-C₄-Alkyl), -SC₁-C₄-Alkyl, -S(O)C₁-C₄-Alkyl, -SO₂C₁-C₄ Alkyl, -S(O)C₁-C₄-Halogenalkyl und -SO₂C₁-C₄-Halogenalkyl besteht;
oder ein Salz davon.

7. Eine Verbindung nach einem der Ansprüche 1 bis 6, wobei:
Q ein 6-gliedriges Aryl bedeutet, das wahlweise substituiert mit 1, 2 oder 3 Substituenten, die unabhängig voneinander aus der Gruppe ausgewählt sind, die aus Halogen, Cyano, Nitro, Hydroxy, C₁-C₄-Alkyl, C₁-C₄-Halogenalkyl, C₁-C₄-Alkoxy, C₃-C₆-Cycloalkyl, -NH₂, -NH(C₁-C₄-Alkyl), -N(C₁-C₄-Alkyl)₂, -NH(C₃-C₆-Cycloalkyl), -N(C₁-C₄-Alkyl)(C₃-C₆-Cycloalkyl), -NHSO₂(C₁-C₄-Alkyl), -SC₁-C₄-Alkyl, -S(O)C₁-C₄-Alkyl, -SO₂C₁-C₄-Alkyl, -S(O)C₁-C₄-Halogenalkyl und -SO₂C₁-C₄-Halogenalkyl besteht, wobei das 6-gliedrige Aryl mit einem 4- bis 7-gliedrigen Heterocycloalkyl mit 1 oder 2 Heteroatomen anelliert ist, die aus der Gruppe ausgewählt sind, die aus O, S und N besteht, und wobei die Kohlenstoffatome des Heterocycloalkyls wahlweise substituiert mit 1, 2 oder 3 Substituenten, die unabhängig voneinander aus der Gruppe ausgewählt sind, die aus Halogen, Cyano, Nitro, Hydroxy, Oxo, C₁-C₄-Alkyl, C₃-C₆-Cycloalkyl, C₁-C₄-Halogenalkyl, C₁-C₄-Alkoxy, -NH₂, -NH(C₁-C₄-Alkyl) und -N(C₁-C₄-Alkyl)₂ besteht, und jedes N in dem Heterocyclalkyl mit einen Substituenten substituiert ist, der aus der Gruppe ausgewählt ist, die aus Wasserstoff, C₁-C₄-Alkyl und C₃-C₆-Cycloalkyl besteht;
oder ein Salz davon.

8. Die Verbindung nach einem der Ansprüche 1 bis 7, wobei:
Y₁ CR₈R₉ und Y₂ O bedeutet;
oder ein Salz davon.

9. Eine Verbindung nach Anspruch 1, wobei die Verbindung aus der Gruppe ausgewählt ist, die aus:
(4R)-N-[8-(3,5-Dichlorphenyl)-4-(dimethylamino)-3-chinolyl]chroman-4-carboxamid;
(4S)-N-[8-(3,5-Dichlorphenyl)-4-(dimethylamino)-3-chinolyl]chroman-4-carboxamid;
(1R)-N-[8-(3,5-Dichlorphenyl)-4-(dimethylamino)-3-chinolyl]tetralin-1-carboxamid;
(1S)-N-[8-(3,5-Dichlorphenyl)-4-(dimethylamino)-3-chinolyl]tetralin-1-carboxamid;
(4R)-N-[4-Cyclopropyl-8-(3,5-dichlorphenyl)-3-chinolyl]chroman-4-carboxamid;
(4S)-N-[4-Cyclopropyl-8-(3,5-dichlorphenyl)-3-chinolyl]chroman-4-carboxamid;
(4R)-N-[8-(3,5-Dichlorphenyl)-4-[methoxy(methyl)amino]-3-chinolyl]chroman-4-carboxamid;
(4S)-N-[8-(3,5-Dichlorphenyl)-4-[methoxy(methyl)amino]-3-chinolyl]chroman-4-carboxamid;
(4S)-N-[8-(2,3-Dichlorphenyl)-4-(dimethylamino)-3-chinolyl]chroman-4-carboxamid;
(4R)-N-[8-[3-Chlor-5-(trifluormethyl)phenyl]-4-(dimethylamino)-3-chinolyl]chroman-4-carboxamid;
(4S)-N-[8-[3-Chlor-5-(trifluormethyl)phenyl]-4-(dimethylamino)-3-chinolyl]chroman-4-carboxamid;
(4S)-N-[8-(3,5-Dichlorphenyl)-4-morpholino-3-chinolyl]chroman-4-carboxamid;
(4R)-N-[8-(3,5-Dichlorphenyl)-4-morpholino-3-chinolyl]chroman-4-carboxamid;
(4S)-N-[4-Morpholino-8-(2,3,5-trifluorphenyl)-3-chinolyl]chroman-4-carboxamid;
(4R)-N-[4-Morpholino-8-(2,3,5-trifluorphenyl)-3-chinolyl]chroman-4-carboxamid;
(4S)-N-[8-(3,5-Dichlorphenyl)-4-(dimethylamino)-1,7-naphthyridin-3-yl]chroman-4-carboxamid;
(4R)-N-[8-(3,5-Dichlorphenyl)-4-(dimethylamino)-1,7-naphthyridin-3-yl]chroman-4-carboxamid;
(4S)-N-[4-Morpholino-8-(2,3,5-trifluorphenyl)-1,7-naphthyridin-3-yl]chroman-4-carboxamid;
(4R)-N-[4-Morpholino-8-(2,3,5-trifluorphenyl)-1,7-naphthyridin-3-yl]chroman-4-carboxamid;
(4S)-N-[4-(3,5-Dichlorphenyl)-8-(dimethylamino)pyrido[3,2-d]pyrimidin-7-yl]chroman-4-carboxamid;
(4R)-N-[4-(3,5-Dichlorphenyl)-8-(dimethylamino)pyrido[3,2-d]pyrimidin-7-yl]chroman-4-carboxamid;
(4R)-N-[8-(3,5-Dichlorphenyl)-4-(dimethylamino)-1,5-naphthyridin-3-yl]chroman-4-carboxamid;
(4S)-N-[8-(3,5-Dichlorphenyl)-4-(dimethylamino)-1,5-naphthyridin-3-yl]chroman-4-carboxamid;
(4S)-N-[8-(3,5-Dichlorphenyl)-4-morpholino-1,5-naphthyridin-3-yl]chroman-4-carboxamid;
1-[(4S)-Chroman-4-yl]-3-[8-(3,5-dichlorphenyl)-4-(dimethylamino)-3-chinolyl]urea;
(4R)-N-[5-(3,5-Dichlorphenyl)-1-(dimethylamino)-2-naphthyl]chroman-4-carboxamid; und
(4S)-N-[5-(3,5-Dichlorphenyl)-1-(dimethylamino)-2-naphthyl]chroman-4-carboxamid besteht;
oder ein Salz von jeder der zuvor genannten Verbindungen.

10. Eine Zubereitung, die eine Verbindung nach einem der Ansprüche 1 bis 9 oder ein Salz davon und mindestens einen verträglichen Träger umfasst.

11. Eine Verbindung nach einem der Ansprüche 1 bis 9 oder ein Salz davon für eine Verwendung als ein Arzneimittel.

12. Eine Verbindung nach einem der Ansprüche 1 bis 9 oder ein Salz davon für eine Verwendung zur Behandlung und/oder Bekämpfung von Endoparasiten.

13. Die Verbindung oder ein Salz davon für eine Verwendung nach Anspuch 12, wobei der Endoparasit der Herzwurm ist.

## Revendications

1. Composé de formule (I) : 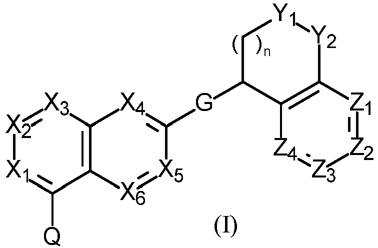 dans lequel
n vaut 1 ;
X₁ est choisi dans le groupe constitué par N et CR₁ ;
X₂ est choisi dans le groupe constitué par CR₂ ;
X₃ est choisi dans le groupe constitué par N et CR₃ ;
X₄ est choisi dans le groupe constitué par CR₄ ;
X₅ est choisi dans le groupe constitué par CR₅ ;
X₆ est choisi dans le groupe constitué par N et CR₆ ;
G est le groupe 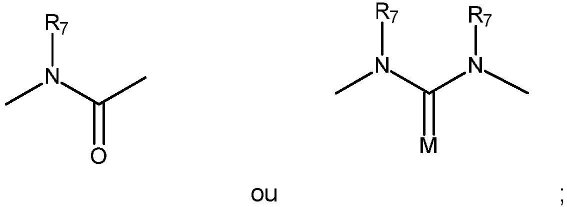
Y₁ est choisi dans le groupe constitué par CR₈R₉, et O ;
Y₂ est choisi dans le groupe constitué par CR₈R₉, et O ;
dans lequel au moins l'un des groupes Y₁ ou Y₂ est CR₈R₉ ;
Z₁ est choisi dans le groupe constitué par N, O, S et CR₁₁ ;
Z₂ est choisi dans le groupe constitué par nul, N et CR₁₁ ;
Z₃ est choisi dans le groupe constitué par nul, N et CR₁₁ ;
Z₄ est choisi dans le groupe constitué par N, O, S et CR₁₁ ;
dans lequel pas plus de 2 parmi Z₁, Z₂, Z₃ et Z₄ sont N et dans lequel un seul parmi Z₁ et Z₄ est O ou S, Z₂ est nul uniquement lorsque Z₁ est O ou S, et Z₃ est nul uniquement lorsque Z₄ est O ou S ;
R₁ est choisi dans le groupe constitué par hydrogène, halogène, hydroxyle, - SH, -S-alkyle en C₁ à C₄, -S(O)(alkyle en C₁ à C₄), -S(O)₂(alkyle en C₁ à C₄), cyano, alkyle en C₁ à C₄, halogénoalkyle en C₁ à C₄, alcoxy en C₁ à C₄, -B(OR₁₆)(OR₁₇) dans lequel R₁₆ est, à chaque reprise, choisi dans le groupe constitué par hydrogène, alkyle en C₁ à C₄, et cycloalkyle en C₃ à Ce, R₁₇ est, à chaque reprise, choisi dans le groupe constitué par hydrogène, alkyle en C₁ à C₄, et cycloalkyle en C₃ à C₆, ou R₁₆ et R₁₇ conjointement avec les atomes d'oxygène auxquels ils sont fixés forment un cycle de 5 à 7 chaînons qui est facultativement substitué par 1 à 4 alkyles en C₁ à C₄ ; -NH₂, -NH(alkyle en C₁ à C₄), et -N(alkyle en C₁ à C₄)₂ ;
R₂ est choisi dans le groupe constitué par hydrogène, halogène, hydroxyle, - SH, -S-alkyle en C₁ à C₄, -S(O)(alkyle en C₁ à C₄), -S(O)₂(alkyle en C₁ à C₄), cyano, alkyle en C₁ à C₄, halogénoalkyle en C₁ à C₄, alcoxy en C₁ à C₄, -B(OR₁₆)(OR₁₇) dans lequel R₁₆ est, à chaque reprise, choisi dans le groupe constitué par hydrogène, alkyle en C₁ à C₄, et cycloalkyle en C₃ à Ce, R₁₇ est, à chaque reprise, choisi dans le groupe constitué par hydrogène, alkyle en C₁ à C₄, et cycloalkyle en C₃ à C₆, ou R₁₆ et R₁₇ conjointement avec les atomes d'oxygène auxquels ils sont fixés forment un cycle de 5 à 7 chaînons qui est facultativement substitué par 1 à 4 alkyles en C₁ à C₄ ; -NH₂, -NH(alkyle en C₁ à C₄), et -N(alkyle en C₁ à C₄)₂ ;
R₃ est choisi dans le groupe constitué par hydrogène, halogène, hydroxyle, - SH, -S-alkyle en C₁ à C₄, -S(O)(alkyle en C₁ à C₄), -S(O)₂(alkyle en C₁ à C₄), cyano, alkyle en C₁ à C₄, halogénoalkyle en C₁ à C₄, alcoxy en C₁ à C₄, -B(OR₁₆)(OR₁₇) dans lequel R₁₆ est, à chaque reprise, choisi dans le groupe constitué par hydrogène, alkyle en C₁ à C₄, et cycloalkyle en C₃ à C₆, R₁₇ est, à chaque reprise, choisi dans le groupe constitué par hydrogène, alkyle en C₁ à C₄, et cycloalkyle en C₃ à C₆, ou R₁₆ et R₁₇ conjointement avec les atomes d'oxygène auxquels ils sont fixés forment un cycle de 5 à 7 chaînons qui est facultativement substitué par 1 à 4 alkyles en C₁ à C₄ ; -NH₂, -NH(alkyle en C₁ à C₄), et -N(alkyle en C₁ à C₄)₂ ;
R₄ est choisi dans le groupe constitué par alkyle en C₁ à C₄, cycloalkyle en C₃ à C₆, -N(alkyle en C₁ à C₄)₂, -N(alkyle en C₁ à C₄)(alcoxy en C₁ à C₄) et hétérocycloalkyle de 4 à 7 chaînons ;
R₆ est choisi dans le groupe constitué par hydrogène, halogène, hydroxyle, - SH, -S-alkyle en C₁ à C₄, -S(O)(alkyle en C₁ à C₄), -S(O)₂(alkyle en C₁ à C₄), cyano, alkyle en C₁ à C₄, halogénoalkyle en C₁ à C₄, alcoxy en C₁ à C₄, -B(OR₁₆)(OR₁₇) dans lequel R₁₆ est, à chaque reprise, choisi dans le groupe constitué par hydrogène, alkyle en C₁ à C₄, et cycloalkyle en C₃ à C₆, R₁₇ est, à chaque reprise, choisi dans le groupe constitué par hydrogène, alkyle en C₁ à C₄, et cycloalkyle en C₃ à C₆, ou R₁₆ et R₁₇ conjointement avec les atomes d'oxygène auxquels ils sont fixés forment un cycle de 5 à 7 chaînons qui est facultativement substitué par 1 à 4 alkyles en C₁ à C₄ ; -NH₂, -NH(alkyle en C₁ à C₄), et -N(alkyle en C₁ à C₄)₂ ;
R₇ est choisi dans le groupe constitué par hydrogène, alkyle en C₁ à C₄, et cycloalkyle en C₃ à C₆ facultativement substitué par 1 à 5 atomes d'halogène, - C(H)O, alcényle en C₂ à C₄, alcynyle en C₂ à C₄, halogénoalkyle en C₁ à C₄, et alcoxy en C₁ à C₄ ;
R₈ est, chaque fois qu'il est choisi, indépendamment choisi dans le groupe constitué par hydrogène, fluoro, et alkyle en C₁ à C₄ ;
R₉ est, chaque fois qu'il est choisi, indépendamment choisi dans le groupe constitué par hydrogène, fluoro, et alkyle en C₁ à C₄ ;
R₁₁ est, chaque fois qu'il est choisi, indépendamment choisi dans le groupe constitué par hydrogène, halogène, hydroxyle, cyano, alkyle en C₁ à C₄, halogénoalkyle en C₁ à C₄, alcoxy en C₁ à C₄, cycloalkyle en C₃ à C₆, -NH₂, - NH(alkyle en C₁ à C₄), et -N(alkyle en C₁ à C₄)₂ ; et
Q est choisi dans le groupe constitué par aryle de 6 ou 10 chaînons facultativement substitué par 1, 2 ou 3 substituants indépendamment choisis dans le groupe constitué par halogène, cyano, nitro, hydroxyle, alkyle en C₁ à C₄, halogénoalkyle en C₁ à C₄, alcoxy en C₁ à C₄, cycloalkyle en C₃ à C₆, -NH₂, - NH(alkyle en C₁ à C₄), -N(alkyle en C₁ à C₄)₂, -NH(cycloalkyle en C₃ à C₆), - N(alkyle en C₁ à C₄)(cycloalkyle en C₃ à C₆), -NHSO₂(alkyle en C₁ à C₄), -S-alkyle en C₁ à C₄, -S(O)-alkyle en C₁ à C₄, -SO₂-alkyle en C₁ à C₄, -S(O)-halogénoalkyle en C₁ à C₄ et -SO₂-halogénoalkyle en C₁ à C₄, dans lequel l'aryle de 6 ou 10 chaînons est facultativement condensé à un hétérocycloalkyle de 4 à 7 chaînons ayant 1 ou 2 hétéroatomes choisis dans le groupe O, S et N et dans lequel les carbones de l'hétérocycloalkyle sont facultativement substitués par 1, 2 ou 3 substituants indépendamment choisis dans le groupe halogène, cyano, nitro, hydroxyle, oxo, alkyle en C₁ à C₄, cycloalkyle en C₃ à C₆, halogénoalkyle en C₁ à C₄, alcoxy en C₁ à C₄, -NH₂, -NH(alkyle en C₁ à C₄), et -N(alkyle en C₁ à C₄)₂ et l'un quelconque N dans l'hétérocycloalkyle est, si la valence le permet, substitué par un substituant choisi dans le groupe constitué par hydrogène, alkyle en C₁ à C₄, et cycloalkyle en C₃ à C₆ ;
M est choisi dans le groupe constitué par N-R₁₃, O et S ;
R₁₃ est choisi dans le groupe constitué par hydroxy, alcoxy en C₁ à C₄ et -NH₂ ; ou un sel de celui-ci.

2. Composé selon la revendication 1, dans lequel X₁ est CR₁ ; X₂ est CR₂ ; X₃ est CR₃ ; X₄ est CR₄ ; X₅ est CR₅ ; et X₆ est CR₆ ; ou un sel de celui-ci.

3. Composé selon la revendication 1 ou 2, dans lequel X₁ est CR₁ ; X₂ est CR₂ ; X₃ est CR₃ ; X₄ est CR₄ ; X₅ est CR₅ ; et X₆ est N ; ou un sel de celui-ci.

4. Composé selon l'une quelconque des revendications 1 à 3, dans lequel X₁ est N ; X₂ est CR₂ ; X₃ est CR₃ ; X₄ est CR₄ ; X₅ est CR₅ ; et X₆ est N ; ou un sel de celui-ci.

5. Composé selon l'une quelconque des revendications 1 à 4 dans lequel X₁ est CR₁ ; X₂ est CR₂ ; X₃ est CR₃ ; X₄ est CR₄ ; X₅ est CR₅ ; et X₆ est N ; ou un sel de celui-ci.

6. Composé selon l'une quelconque des revendications 1 à 5, dans lequel
Q est un aryle de 6 ou 10 chaînons facultativement substitué par 1, 2 ou 3 substituants indépendamment choisis dans le groupe constitué par halogène, cyano, nitro, hydroxy, alkyle en C₁ à C₄, halogénoalkyle en C₁ à C₄, alcoxy en C₁ à C₄, cycloalkyle en C₃ à C₆, -NH₂, -NH(alkyle en C₁ à C₄), -N(alkyle en C₁ à C₄)₂, - NH(cycloalkyle en C₃ à C₆), -N(cycloalkyle en C₁ à C₄)(cycloalkyle en C₃ à C₆), - NHSO₂(alkyle en C₁ à C₄), -S-alkyle en C₁ à C₄, -S(O)-alkyle en C₁ à C₄, -SO₂-alkyle en C₁ à C₄, -S(O)-halogénoalkyle en C₁ à C₄ et -SO₂halogénoalkyle en C₁ à C₄ ;
ou un sel de celui-ci.

7. Composé selon l'une quelconque des revendications 1 à 6, dans lequel
Q est un aryle de 6 chaînons facultativement substitué par 1, 2 ou 3 substituants indépendamment choisis dans le groupe constitué par halogène, cyano, nitro, hydroxy, alkyle en C₁ à C₄, halogénoalkyle en C₁ à C₄, alcoxy en C₁ à C₄, cycloalkyle en C₃ à C₆, -NH₂, -NH(alkyle en C₁ à C₄), -N(alkyle en C₁ à C₄)₂, -NH(cycloalkyle en C₃ à C₆), -N(alkyle en C₁ à C₄)(cycloalkyle en C₃ à C₆), - NHSO₂(alkyle en C₁ à C₄), -S-alkyle en C₁ à C₄, -S(O)-alkyle en C₁ à C₄, -SO₂-alkyle en C₁ à C₄, -S(O)-halogénoalkyle en C₁ à C₄ et -SO₂-halogénoalkyle en C₁ à C₄, dans lequel l'aryle de 6 chaînons est condensé avec un hétérocycloalkyle de 4 à 7 chaînons ayant 1 ou 2 hétéroatomes choisi dans le groupe O, S et N et dans lequel les carbones de l'hétérocycloalkyle sont facultativement substitués par 1, 2 ou 3 substituants indépendamment choisis dans le groupe constitué par halogène, cyano, nitro, hydroxy, oxo, alkyle en C₁ à C₄, cycloalkyle en C₃ à C₆, halogénoalkyle en C₁ à C₄, alcoxy en C₁ à C₄, -NH₂, -NH(alkyle en C₁ à C₄) et -N(alkyle en C₁ à C₄)₂ et l'un quelconque N dans l'hétérocycloalkyle est substitué par un substituant choisi dans le groupe constitué par hydrogène, alkyle en C₁ à C₄, et cycloalkyle en C₃ à C₆ ;
ou un sel de celui-ci.

8. Composé selon l'une quelconque des revendications 1 à 7, dans lequel
Y₁ est CR₈R₉ et Y₂ est O ; ou un sel de celui-ci ;

9. Composé selon la revendication 1, dans lequel le composé est choisi dans le groupe constitué par
(4R)-N-[8-(3,5-dichlorophényl)-4-(diméthylamino)-3-quinolyl]chromane-4-carboxamide ;
(4S)-N-[8-(3,5-dichlorophényl)-4-(diméthylamino)-3-quinolyl]chromane-4-carboxamide ;
(1R)-N-[8-(3,5-dichlorophényl)-4-(diméthylamino)-3-quinolyl]tétraline-1-carboxamide
(1S)-N-[8-(3,5-dichlorophényl)-4-(diméthylamino)-3-quinolyl]tétraline-1-carboxamide ;
(4R)-N-[4-cyclopropyl-8-(3,5-dichlorophényl)-3-quinolyl]chromane-4-carboxamide ;
(4S)-N-[4-cyclopropyl-8-(3,5-dichlorophényl)-3-quinolyl]chromane-4-carboxamide ;
(4R)-N-[8-(3,5-dichlorophényl)-4-[méthoxy(méthyl)amino]-3-quinolyl]chromane-4-carboxamide ;
(4S)-N-[8-(3,5-dichlorophényl)-4-[méthoxy(méthyl)amino]-3-quinolyl]chromane-4-carboxamide ;
(4S)-N-[8-(2,3-dichlorophényl)-4-(diméthylamino)-3-quinolyl]chromane-4-carboxamide ;
(4R)-N-[8-[3-chloro-5-(trifluorométhyl)phényl]-4-(diméthylamino)-3-quinolyl]chromane-4-carboxamide ;
(4S)-N-[8-[3-chloro-5-(trifluorométhyl)phényl]-4-(diméthylamino)-3-quinolyl]chromane-4-carboxamide ;
(4S)-N-[8-(3,5-dichlorophényl)-4-morpholino-3-quinolyl]chromane-4-carboxamide ;
(4R)-N-[8-(3,5-dichlorophényl)-4-morpholino-3-quinolyl]chromane-4-carboxamide ;
(4S)-N-[4-morpholino-8-(2,3,5-trifluorophényl)-3-quinolyl]chromane-4-carboxamide ;
(4R)-N-[4-morpholino-8-(2,3,5-trifluorophényl)-3-quinolyl]chromane-4-carboxamide ;
(4S)-N-[8-(3,5-dichlorophényl)-4-(diméthylamino)-1,7-naphtyridin-3-yl]chromane-4-carboxamide ;
(4R)-N-[8-(3,5-dichlorophényl)-4-(diméthylamino)-1,7-naphtyridin-3-yl]chromane-4-carboxamide ;
(4S)-N-[4-morpholino-8-(2,3,5-trifluorophényl)-1,7-naphtyridin-3-yl]chromane-4-carboxamide ;
(4R)-N-[4-morpholino-8-(2,3,5-trifluorophényl)-1,7-naphtyridin-3-yl]chromane-4-carboxamide ;
(4S)-N-[4-(3,5-dichlorophényl)-8-(diméthylamino)pyrido[3,2-d]pyrimidin-7-yl]chromane-4-carboxamide ;
(4R)-N-[4-(3,5-dichlorophényl)-8-(diméthylamino)pyrido[3,2-d]pyrimidin-7-yl]chromane-4-carboxamide ;
(4R)-N-[8-(3,5-dichlorophényl)-4-(diméthylamino)-1,5-naphtyridin-3-yl]chromane-4-carboxamide ;
(4S)-N-[8-(3,5-dichlorophényl)-4-(diméthylamino)-1,5-naphtyridin-3-yl]chromane-4-carboxamide ;
(4S)-N-[8-(3,5-dichlorophényl)-4-morpholino-1,5-naphtyridin-3-yl]chromane-4-carboxamide ;
1-[(4S)-chroman-4-yl]-3-[8-(3,5-dichlorophényl)-4-(diméthylamino)-3-quinolyl]urée ;
(4R)-N-[5-(3,5-dichlorophényl)-1-(diméthylamino)-2-naphtyl]chromane-4-carboxamide ; et
(4S)-N-[5-(3,5-dichlorophényl)-1-(diméthylamino)-2-naphtyl]chromane-4-carboxamide ; ou un sel de chacun des composés précités.

10. Composition comprenant un composé selon l'une quelconque des revendications 1 à 9, ou un sel de celui-ci, et au moins un véhicule acceptable.

11. Composé selon l'une quelconque des revendications 1 à 9, ou sel de celui-ci, pour une utilisation en tant que médicament.

12. Composé selon l'une quelconque des revendications 1 à 9, ou sel de celui-ci, pour une utilisation dans le traitement et/ou la lutte contre des endoparasites.

13. Composé ou sel de celui-ci pour utilisation selon la revendication 12, dans lequel les endoparasites sont le ver du coeur.
